(19) Europäisches Patentamt
European Patent Office
Office européen des brevets

(11) **EP 2 407 100 A1**

(12) **EUROPEAN PATENT APPLICATION**

(43) Date of publication:
**18.01.2012 Bulletin 2012/03**

(51) Int Cl.:
*A61B 5/053* (2006.01)    *A61B 5/08* (2006.01)

(21) Application number: **11173341.6**

(22) Date of filing: **08.07.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.07.2010 JP 2010160950**
**26.07.2010 JP 2010167481**
**13.04.2011 JP 2011088770**
**13.04.2011 JP 2011088771**

(71) Applicant: **Tanita Corporation**
**Tokyo 174-8630 (JP)**

(72) Inventor: **Masuo, Yoshihisa**
**Itabashi-ku, Tokyo 174-8630 (JP)**

(74) Representative: **Haley, Stephen**
**Gill Jennings & Every LLP**
**The Broadgate Tower**
**20 Primrose Street**
**London EC2A 2ES (GB)**

(54) **Respiration characteristic analysis**

(57) A respiration characteristic analysis apparatus includes a bioelectrical impedance determiner adapted for determining a first bioelectrical impedance at the upper body trunk of a human subject including the upper lobes of the lungs of the human subject and excluding the abdomen of the human subject and a second bioelectrical impedance at the middle body trunk of the human subject including the median and lower lobes of the lungs of the human subject and the abdomen of the human subject; and an analyzer adapted for analyzing a respiration characteristic of the human subject on the basis of change over time in each of the first bioelectrical impedance and the second bioelectrical impedance determined by the bioelectrical impedance determiner.

Fig. 1

EP 2 407 100 A1

Printed by Jouve, 75001 PARIS (FR)

**Description**

BACKGROUND OF THE INVENTION

FIELD OF THE INVENTION

The present invention relates to apparatuses and systems for analyzing characteristics of respiration (breathing) in human subjects.

RELATED ART

There are known various apparatuses for measuring bioelectrical impedance and for estimating body conditions based on the measured impedance. For example, US 2007/043302 A1 discloses a technique for estimating the breathing capacity of the lungs of a human subject on the basis of the impedance of the body trunk.

Respiration (breathing) is distinctive among vital activity (e.g., blood pressure, body temperature, skin temperature, brain waves, and pulse waves) because it can be under voluntary control. Accordingly, many methods have been developed around the world for maintaining health, e.g., *yoga, Qigong,* chiropractic, or *zazen.* Respiration is classified into abdominal (diaphragmatic breathing) and costal (chest breathing). Abdominal respiration is linked with not only various methods of health maintenance, but also dieting and voice training.

For health maintenance of a human subject, it may sometimes be advantageous to determine respiration functions of the human subject.

Accordingly, the present invention provides apparatuses and systems for analyzing a characteristic of respiration of human subjects using bioelectrical impedances of the human subjects.

SUMMARY OF THE INVENTION

In accordance with the present invention, a respiration characteristic analysis apparatus includes: a bioelectrical impedance determiner adapted for determining a first bioelectrical impedance at the upper body trunk of a human subject including the upper lobes of the lungs of the human subject and excluding the abdomen of the human subject, and a second bioelectrical impedance at the middle body trunk of the human subject including the median and lower lobes of the lungs of the human subject and the abdomen of the human subject; and an analyzer adapted for analyzing a respiration characteristic of the human subject on the basis of change over time in each of the first bioelectrical impedance and the second bioelectrical impedance determined by the bioelectrical impedance determiner.

According to the present invention, on the basis of change over time in each of the first bioelectrical impedance and the second bioelectrical impedance, the analyzer analyzes a respiration characteristic of the human subject. For example, on the basis of change over time in each of the first bioelectrical impedance and the second bioelectrical impedance, the analyzer may calculate indicative information that is used for identifying whether respiration of the human subject is abdominal or costal. Alternatively, the analyzer may decide whether a function of the part of the human subject that contributes to respiration of the human subject is normal or abnormal, on the basis of change over time in each of the first bioelectrical impedance and the second bioelectrical impedance.

The respiration characteristic analysis apparatus may further include a centering value generator adapted for generating a first centering value that is an average of the first bioelectrical impedances within a past unit time on the basis of change over time in the first bioelectrical impedance, and for generating a second centering value that is an average of the second bioelectrical impedances within a past unit time on the basis of change over time in the second bioelectrical impedance, the first centering value being a standard level of change over time in the first bioelectrical impedance, the second centering value being a standard level of change over time in the second bioelectrical impedance; a first difference calculator adapted for calculating a first difference between the first bioelectrical impedance and the first centering value; and a second difference calculator adapted for calculating a second difference between the second bioelectrical impedance and the second centering value. The analyzer may be adapted for analyzing the respiration characteristic of a part of the human subject that contributes to respiration of the human subject on the basis of the first difference and the second difference.

In this aspect, on the basis of the first difference and the second difference, the respiration characteristic of a part of the human subject that contributes to respiration of the human subject is analyzed, so that the respiration characteristic of the human subject can be determined accurately.

The respiration characteristic analysis apparatus may further include a zero-cross time decider for deciding zero-cross times in which the first bioelectrical impedance is equal to the first centering value. The bioelectrical impedance determiner may be adapted for determining the first bioelectrical impedance and the second bioelectrical impedance at each sampling time occurring at a predetermined cycle. The centering value generator may be adapted for generating the first centering value on the basis of the first bioelectrical impedance at each of the sampling times, the number of the sampling times being predetermined. The centering value generator may be adapted for generating the second centering value on the basis of the second bioelectrical impedance at each of the zero-cross times decided by the zero-cross time decider, the number of the zero-cross times being predetermined.

When a human performs respiratory actions that consist of inhalation and exhalation, the first bioelectrical impedance at the upper body trunk and the second bioelectrical impedance at the middle body trunk change. In all of abdominal respiration, costal respiration, and a draw-in respiration (respiration in which inhalation and exhalation are repeated with the abdomen held in a constricted position), the lungs expand and contract, so that the bioelectrical impedance at the lungs increases at inhalations due to increase in the volume of air inside tissues in the lungs, and the bioelectrical impedance at the lungs decreases at exhalations due to decrease in the volume of air inside tissues in the lungs. Therefore, irrespective to the type of respiration of the human subject, the first bioelectrical impedance at the upper body trunk including the upper lobes of the lungs of the human subject and excluding the abdomen of the human subject increases at inhalations and decreases at exhalations.

In abdominal respiration, by the action of the abdominal skeletal muscle, the visceral tissue raises the diaphragm at exhalations, so that the abdominal bioelectrical impedance increases. In other words, increase in the bioelectrical impedance of the abdominal region beneath the diaphragm cancels decrease in the bioelectrical impedance at the chest region above the diaphragm when the human subject performs exhalation in abdominal respiration. The same is not true for costal respiration or draw-in respiration. Consequently, when respiration of the human subject is abdominal respiration, change in the second bioelectrical impedance at the middle body trunk including the median and lower lobes of lungs and the abdomen of the human subject is different from that in the first bioelectrical impedance.

Irrespective to the type of respiration of the human subject, the waveform of change in the first bioelectrical impedance in respiration is nearly sinusoidal. It is preferable to obtain a suitable first centering value (standard level of change over time in the first bioelectrical impedance used for extracting information on respiration of the human subject) even if one or more instantaneous values of the first bioelectrical impedance are disturbed by body motion or for other reasons. Accordingly, the centering value generator may be adapted for generating the first centering value on the basis of first bioelectrical impedance at each of sampling times, the number of the sampling times being predetermined. In this case, it is possible to obtain a suitable first centering value even if one or more instantaneous values of the first bioelectrical impedance are disturbed by body motion or for other reasons.

On the other hand, change in the second bioelectrical impedance in abdominal respiration is not sinusoidal and is different from that of the first bioelectrical impedance. Accordingly, in contrast to the calculation of the first centering value, if a moving average is calculated on the basis of measurement values of the second bioelectrical impedance at the sampling times of which the number is predetermined, the second centering value cannot be calculated accurately. Accordingly, the centering value generator may be adapted for generating the second centering value on the basis of the second bioelectrical impedance at each of the zero-cross times decided by the zero-cross time decider. In this case, the second centering value that is the standard level of the second bioelectrical impedance can be calculated accurately.

More specifically, the centering value generator may be adapted for calculating a moving average at each sampling time, the moving average being a moving average of the first bioelectrical impedances at multiple sampling times within a centering period starting from a time point that is a predetermined time length before a current sampling time and ending at the current sampling time. The centering value generator may be adapted for generating the first centering value at the current sampling time on the basis of the moving averages at multiple sampling times. In this case, it is possible to obtain a suitable first centering value even if one or more instantaneous values of the first bioelectrical impedance are disturbed by body motion or for other reasons.

The time length of the centering period may be variable and may be set depending on the respiration speed of the human subject at the current sampling time. The moving average may be calculated with or without the use of weighting factors. For example, the moving average may be calculated with the use of weighting factors depending on the frequency at each sampling time.

The centering value generator may be adapted for deciding whether or not each sampling time is a zero-cross time, and for generating the second centering value at the current sampling time on the basis of the second bioelectrical impedances including the second bioelectrical impedance at the current sampling time if the current sampling time is a zero-cross time. The centering value generator may be adapted for deciding the second centering value generated at a last sampling time as the second centering value at the current sampling time if the current sampling time is not a zero-cross time. In this case, the second centering value that is the standard level of the second bioelectrical impedance can be calculated accurately.

The analyzer may be adapted for analyzing whether or not a function of the part of the human subject that contributes to respiration of the human subject is normal, on the basis of change over time in each of the first bioelectrical impedance and the second bioelectrical impedance.

For example, if the human subject has a history of disease at the chest and the function at the chest part that contributes to respiration (e.g., internal and external intercostal muscles) is deteriorated, the motion at the chest skeletal muscle in respiration is very small and is less than that of a physically unimpaired person. In order to ensure a sufficient ventilation volume, such a human subject will move the diaphragm remarkably, so that the displacement of the diaphragm is large. The same is true for a human subject having deteriorated function at the chest due to aging.

Change in the first bioelectrical impedance at the upper body trunk including the upper lobes of the lungs of the human subject and excluding the abdomen of the human subject corresponding to a volume of air entering and leaving the lungs of the human subject. Change in the second bioelectrical impedance at the middle body trunk including the median and lower lobes of the lungs of the human subject and the abdomen of the human subject corresponds to movement of the diaphragm. The greater the movement of the diaphragm, the greater the change in the second bioelectrical impedance.

Even if the ventilation volume of air entering and leaving the lungs of the human subject with deteriorated function at the chest part that contributes to respiration in a single respiration were the same as that of a physically unimpaired person, change in the second bioelectrical impedance in a single respiration of the human subject is greater than that of the physically unimpaired person because of the greater movement of the diaphragm.

Accordingly, in this aspect of the present invention, it is decided whether or not a respiration characteristic at the chest part that contributes to respiration of the human subject is normal on the basis of change over time in each of the second bioelectrical impedance and the first bioelectrical impedance. Thus, the respiration characteristics of the human subject (e.g., deterioration of function at the chest part that contributes to respiration) can be determined.

In costal respiration, which expands and contracts the thoracic cage, the chest skeletal muscle that contributes to respiration (e.g., internal and external intercostal muscles) expands and contracts in a similar way to the expansion and contraction of the lungs. Therefore, in costal respiration, when the lungs expand so that the bioelectrical impedance at the lungs increases, the chest skeletal muscle also expands, and the bioelectrical impedance at the chest skeletal muscle also increases. Similarly, when the lungs contract so that the bioelectrical impedance at the lungs decreases, the chest skeletal muscle also contracts and the bioelectrical impedance at the chest skeletal muscle also decreases. However, in abdominal respiration in which the thoracic cage does not change in volume significantly, although the bioelectrical impedance at the lungs changes significantly due to respiration, the bioelectrical impedance at the chest skeletal muscle does not change significantly. In both of costal respiration and abdominal respiration, the first bioelectrical impedance at the upper body trunk including the upper lobes of the lungs and excluding the abdomen increases at inhalations and decreases at exhalations.

The characteristic of abdominal respiration that does not appear in costal respiration is that the visceral tissue expands and contracts in the perpendicular direction so as to move the diaphragm up and down due to ventrodorsal contraction and expansion of the abdominal skeletal muscle. More specifically, in abdominal respiration, during exhalations, the human subject contracts the abdominal muscle ventrodorsally so as to move up the diaphragm together. As a result, the visceral tissue and the abdominal skeletal muscle expand in the perpendicular direction, thereby increasing the impedance at the abdominal skeletal muscle and the impedance at the viscera. During exhalations, the bioelectrical impedance at the lungs decreases due to reduction in the volume of air inside tissues in the lungs. Therefore, in abdominal respiration, when the bioelectrical impedance at the chest region above the diaphragm decreases, the bioelectrical impedance at the abdominal region beneath the diaphragm increases. This is not true for costal respiration.

Change in the second bioelectrical impedance during exhalations of abdominal respiration is completely different from that of the first bioelectrical impedance. Irrespective whether respiration of the human subject is costal respiration or abdominal respiration, change in the second bioelectrical impedance during inhalations is similar to change in the first bioelectrical impedance. Accordingly, the waveform of change in second bioelectrical impedance during respiration includes distortion resulting from exhalations in abdominal respiration. Thus, it is preferable to determine whether or not the function at the chest part that contributes to respiration of the human subject is normal on the basis of change in each of the first bioelectrical impedance and the second bioelectrical impedance at inhalations.

More specifically, the analyzer may be adapted for deciding that a function of the part of the human subject that contributes to respiration of the human subject is abnormal if a ratio of the peak value of change in the second difference to the peak value of change in the first difference is equal to or greater than a predetermined threshold, and the analyzer may be adapted for deciding that a function of the part of the human subject that contributes to respiration of the human subject is normal if the ratio of the peak value of change in the second difference to the peak value of change in the first difference is less than the predetermined threshold. In this case, it is possible to accurately decide whether or not function at the chest part that contributes to respiration of the human subject is normal.

In another aspect, the analyzer may be adapted for calculating indicative information that is used for identifying whether respiration of the human subject is abdominal or costal, on the basis of change over time in each of the first bioelectrical impedance and the second bioelectrical impedance. In this case, the respiration characteristic analysis apparatus may be used as a respiration type determination apparatus.

The analyzer may not only calculate the indicative information, but also decide whether respiration of the human subject is abdominal respiration or costal respiration on the basis of the indicative information. The analyzer may report the decision result to the human subject or other person, or may output a signal indicating the decision result.

The indicative information may indicate a ratio between variation in the costal circumference and variation in the abdominal circumference in respiration, and the analyzer may be adapted for executing an arithmetic process in accordance with a formula expressing a relationship among indicative information, first differences, and second differences, thereby calculating the indicative information corresponding to the first difference calculated by the first difference calculator and the second difference calculated by the second difference calculator.

On the basis of the first difference and the second difference, the analyzer calculates the indicative information that is used for identifying whether respiration of the human subject is abdominal or costal, so that the type of respiration of the human subject can be determined in real time accurately. The present inventor found that there was a close correlative relationship among the ratio between variation in the costal circumference of the human subject and variation in the abdominal circumference, the first difference, and the second difference. The analyzer may execute an arithmetic process in accordance with a formula expressing the relationship among indicative information, first differences, and second differences, thereby calculating the indicative information corresponding to the first difference and the second difference. From the indicative information, the type of respiration of the human subject can be assumed.

The formula may be expressed as

$$\Delta R_{ib}/\Delta A_b = (a * \Delta Z_b - \Delta Z_a)/\Delta Z_a + b,$$

in which $\Delta R_{ib}$ is the variation in the costal circumference of the human subject, $\Delta A_b$ is the variation in the abdominal circumference of the human subject, $\Delta R_{ib}/\Delta A_b$ is the indicative information, $\Delta Z_a$ is the first difference, $\Delta Z_b$ is the second difference, and $a$ and b are constants.

The ratio $\Delta R_{ib}/\Delta A_b$ indicates that respiration of the human subject is costal respiration if $\Delta R_{ib}/\Delta A_b$ is greater than a predetermined threshold, whereas the ratio $\Delta R_{ib}/\Delta A_b$ indicates that respiration of the human subject is abdominal respiration if $\Delta R_{ib}/\Delta A_b$ is equal to or less than the predetermined threshold. From the ratio $\Delta R_{ib}/\Delta A_b$, it is possible to accurately determine whether respiration of the human subject is costal respiration or abdominal respiration.

The analyzer may be adapted for calculating indicative information that is used for identifying whether respiration of the human subject is abdominal respiration, costal respiration, or a respiration in which inhalation and exhalation are repeated with the abdomen held in a constricted position, on the basis of change over time in each of the first bioelectrical impedance and the second bioelectrical impedance.

In this case, in addition to determining whether respiration of the human subject is abdominal respiration or costal respiration, it is possible to decide whether or not respiration of the human subject is a respiration (draw-in respiration) in which inhalation and exhalation are repeated with the abdomen held in a constricted position.

The analyzer may not only calculate the indicative information, but also decide that respiration of the human subject is abdominal respiration, costal respiration, or a respiration in which inhalation and exhalation are repeated with the abdomen held in a constricted position, on the basis of the indicative information. The analyzer may report the decision result to the human subject or other person, or may output a signal indicating the decision result.

The analyzer may be adapted for calculating the ratio $\Delta R_n/\Delta A_b$ as the indicative information that is used for identifying whether respiration of the human subject is abdominal respiration, costal respiration, or a respiration in which inhalation and exhalation are repeated with the abdomen held in a constricted position, on the basis of change over time in each of the first bioelectrical impedance and the second bioelectrical impedance, in which the ratio $\Delta R_{ib}/\Delta A_b$ indicates that respiration of the human subject is abdominal respiration if $\Delta R_{ib}/\Delta A_b$ is equal to or less than a predetermined threshold. The ratio $\Delta R_{ib}/\Delta A_b$ indicates that respiration of the human subject is respiration in which inhalation and exhalation are repeated with the abdomen held in a constricted position if $\Delta R_{ib}/\Delta A_b$ is greater than a predetermined threshold and if the current second centering value generated by the centering value generator is equal to or greater than a sum of a standard second centering value in costal respiration of the human subject and a predetermined value. The ratio $\Delta R_{ib}/\Delta A_b$ indicates that respiration of the human subject is costal respiration if $\Delta R_{ib}/\Delta A_b$ is greater than a predetermined threshold and if the current second centering value generated by the centering value generator is less than a sum of a standard second centering value in costal respiration of the human subject and a predetermined value. Thus, from the ratio $\Delta R_{ib}/\Delta A_b$, it is possible to accurately decide that respiration of the human subject is costal respiration, abdominal respiration, or draw-in respiration.

The respiration characteristic analysis apparatus may further include: a respiration depth calculator adapted for calculating a respiration depth of the human subject at every respiration of the human subject; an abdominal respiration percentage level calculator adapted for calculating, at every respiration of the human subject, an abdominal respiration percentage level that is a ratio of the abdominal respiration in the single respiration on the basis of the indicative information calculated by the analyzer; and a reporter adapted for reporting, at every respiration of the human subject, a magnitude of each of abdominal respiration and costal respiration and a margin level beyond an essential respiration depth with respect to each of abdominal respiration and costal respiration in a single respiration, on the basis of the respiration depth and the abdominal respiration percentage level at a current single respiration.

More specifically, the respiration characteristic analysis apparatus may further include a normalizer adapted for normalizing the respiration depth calculated by the respiration depth calculator. The reporter may be adapted for executing an arithmetic process in accordance with a second formula expressing a relationship between respiration depths and one-time ventilation volumes each of which is a volume of air entering and leaving the lungs of a human being in a single respiratory action, thereby calculating a one-time ventilation volume corresponding to the respiration depth normalized by the normalizer. The reporter may be adapted for deciding the magnitude of each of abdominal respiration and costal respiration and the margin level beyond the essential respiration depth with respect to each of abdominal respiration and costal respiration, on the basis of the one-time ventilation volume and the abdominal respiration percentage level, and for reporting the magnitude of each of abdominal respiration and costal respiration and the margin level beyond the essential respiration depth with respect to each of abdominal respiration and costal respiration.

The reporter reports to the human subject or other person at every respiration of the human subject, the magnitude of each of abdominal respiration and costal respiration and a margin level beyond the essential respiration depth with respect to each of abdominal respiration and costal respiration in the single respiration, so that the human subject or other person can understand strengths and weaknesses of activity of the costal respiratory muscles and abdominal respiratory muscles of the human subject. Whereas the human subject is made aware of the strength of the human subject, the human subject may be motivated to train the respiratory muscles by, for example, voluntary abdominal respiration, in order to overcome a weakness. In accordance with this aspect, the margin level of the respiration capability of the human subject can be known even if the human subject does not breathe at the maximum respiration depth, in contrast to use of spirometers. Therefore, the use of this aspect is safer for human subjects than the use of spirometers.

The respiration characteristic analysis apparatus may further include a display data generator adapted for generating display data for displaying a Lissajous figure showing change over time in the first bioelectrical impedance and change over time in the second bioelectrical impedance in an orthogonal coordinate system having two orthogonal coordinate axes in which a first axis is the first bioelectrical impedance and a second axis is the second bioelectrical impedance.

Two orthogonal coordinate axes may be, for example, the X axis and the Y axis. However, two orthogonal coordinate axes may be two axes obtained by inclining the X axis and the Y axis by 45 degrees. The Lissajous figure may show the status of only a single respiration as shown in Fig. 32 or 33, or may show the status of multiple respirations continually as shown in Fig. 34 or Fig. 35. The respiration characteristic analysis apparatus may include a display device for displaying the Lissajous figure, or an output part for outputting display data for displaying the Lissajous figure to an external display device.

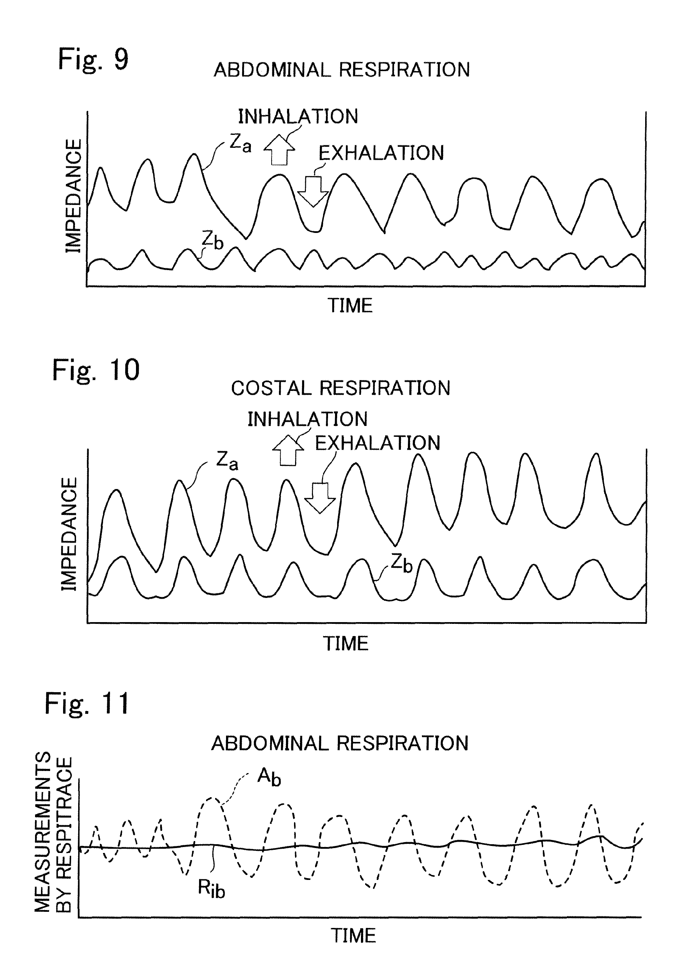

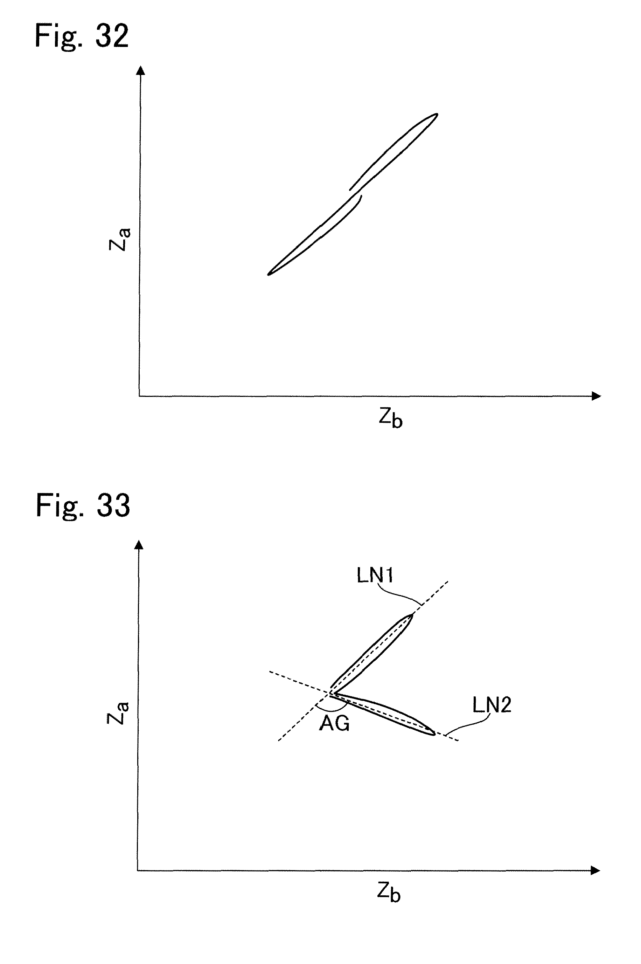

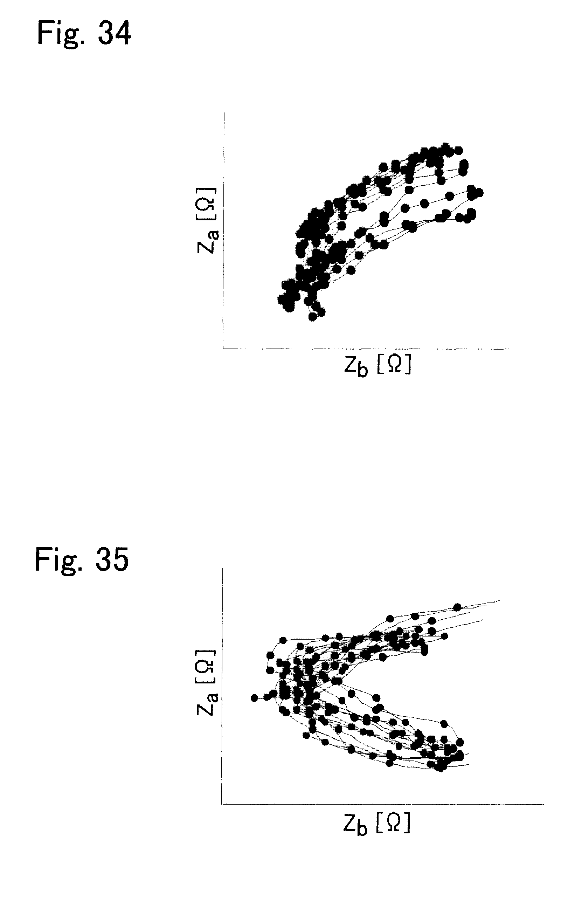

When respiration of the human subject is costal respiration, as shown in Fig. 10, both of the first bioelectrical impedance $Z_a$ and the second bioelectrical impedance $Z_b$ increase at inhalations whereas both of the first bioelectrical impedance $Z_a$ and the second bioelectrical impedance $Z_b$ decrease at exhalations. Therefore, when the ratio of costal respiration in respiration is extremely high, the track of the Lissajous figure is of an inclined straight shape as shown in Fig. 32 or Fig. 34. When costal respiration is shallow, the track of the Lissajous figure is small. When costal respiration is deep, the track of the Lissajous figure is large.

In contrast, in abdominal respiration, as shown in Fig. 9, both of the first bioelectrical impedance $Z_a$ and the second bioelectrical impedance $Z_b$ increase at inhalations, but the first bioelectrical impedance $Z_a$ decreases whereas the second bioelectrical impedance $Z_b$ increases at exhalations. Therefore, when respiration includes abdominal respiration, the track of the Lissajous figure is of a bent shape, as shown in Fig. 33 or Fig. 35.

The Lissajous figure in Fig. 33 shows a case in which 50% of a single respiration is costal and 50% of the single respiration is abdominal. In this case, the track of the Lissajous figure is of a boomerang shape (an L-shape) that is symmetric with respect to a horizontal line. However, if the percentage of abdominal respiration is less than that of costal respiration, the upper upward-sloping portion of the track of the Lissajous figure corresponding to costal respiration is larger than that in Fig. 33 whereas the lower downward-sloping portion of the track of the Lissajous figure corresponding to abdominal respiration is smaller than that in Fig. 33. If the percentage of abdominal respiration is greater than that of costal respiration, the upper upward-sloping portion of the track of the Lissajous figure corresponding to costal respiration is smaller than that in Fig. 33 whereas the lower downward-sloping portion of the track of the Lissajous figure corresponding to abdominal respiration is larger than that in Fig. 33. Thus, depending on the percentage of abdominal respiration, the track of the Lissajous figure describes various tracks.

Theoretically, when abdominal respiration occupies 100% of respiration, the track of the Lissajous figure is of an inclined straight shape in which the inclination is opposite to that in costal respiration. However, respiration of human beings must include costal respiration, except for those in which the diaphragms do not work at all due to a disorder (e.g., a disease). This can be confirmed by observing that even if a human stops breathing, when the human expands and contracts the abdomen, the diaphragm moves up and down so as to expand and contract the lungs. Accordingly, even if the human subject performs abdominal respiration as much as possible, the track of the Lissajous figure is of a bent shape having a straight portion corresponding to costal respiration.

The bend angle AG formed between the straight portion (approximate straight line LN1) corresponding to costal respiration and the straight portion (approximate straight line LN2) corresponding to abdominal respiration shown in Fig. 33 is small when abdominal respiration is shallow. When abdominal respiration is deep, the bend angle AG is large. In addition, the shallower the abdominal respiration, the smaller the track of the Lissajous figure.

Thus, the track of the Lissajous figure varies depending on whether or not respiration is costal or abdominal. The size and the shape of the track of the Lissajous figure vary depending on the magnitude (depth) of each of costal respiration and abdominal respiration. By observing the Lissajous figure, the human subject or another person can understand whether current respiration of the human subject is costal or abdominal, or can understand whether respiration of the human subject is mainly dependent on costal respiration or abdominal respiration. The human subject or another person can also understand the magnitude of each of costal respiration and abdominal respiration by the Lissajous figure. Accordingly, the respiration characteristic analysis apparatus can be used as a breathing training apparatus.

When the human subject trains for costal breathing, the human subject may pay attention to the Lissajous figure so that the track of the Lissajous figure becomes an inclined straight shape and the size of the track becomes large. When the human subject trains for abdominal breathing, the human subject may pay attention to the Lissajous figure so that the track of the Lissajous figure is of a bent shape, and the size and the bend angle AG become large. Thus, by observing the Lissajous figure and confirming the type and the magnitude of respiration at any time, the human subject can train for appropriate costal or abdominal breathing.

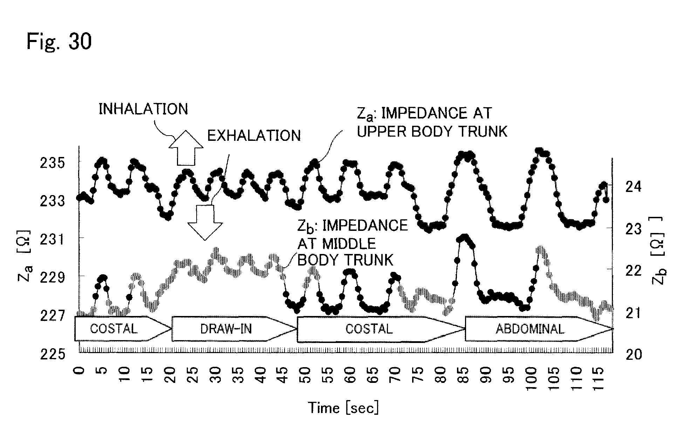

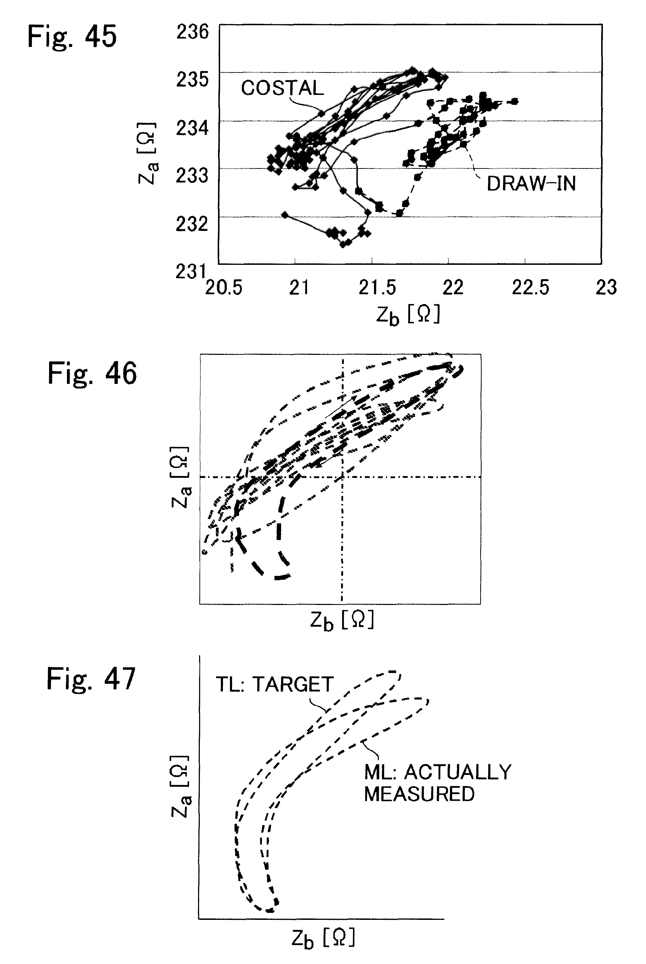

When respiration of the human subject is draw-in respiration, both of the first bioelectrical impedance $Z_a$ and the second bioelectrical impedance $Z_b$ increase at inhalations, whereas both of the first bioelectrical impedance $Z_a$ and the second bioelectrical impedance $Z_b$ decrease at exhalations. The manner of change is the same as that in costal respiration since human beings expand and contract the thoracic cage in both of costal respiration and draw-in respiration. However, in draw-in respiration, the abdomen is held in a constricted position continually so as to be stressed continually. Therefore, the standard level of the second bioelectrical impedance $Z_b$ at the middle body trunk in draw-in respiration is higher than that in costal respiration as shown in Fig. 30. For this reason, as in Fig. 45 which shows Lissajous figures in draw-in respiration and costal respiration, both tracks of the Lissajous figures are of a straight shape rising from bottom left to top right, but locations of both tracks are different in the axis indicating the second bioelectrical impedance $Z_b$ (X axis in Fig. 45).

Thus, by observing the Lissajous figure, the human subject or another person can understand whether or not respiration of the human subject is draw-in respiration. The shallower draw-in respiration, the smaller the track of the Lissajous figure, so that the magnitude of the draw-in respiration can be understood from the Lissajous figure. By observing the Lissajous figure and confirming the type and the magnitude of respiration at any time, the human subject can train for appropriate draw-in breathing.

As has been described above, by virtue of displaying the Lissajous figure, the human subject or another person can understand the type and magnitude of respiration of the human subject, and can understand whether or not the human subject is appropriately performing the target type of breathing. In addition, the human subject can train to breath effectively.

In respiratory inductance plethysmography, the variation ratio in the costal circumference $R_{rc}$ (%) and the variation ratio in the abdominal circumference $R_{abd}$ (%) in respiration may be determined on the basis of variation of inductance of each coil wound around a human body. The coils are incorporated into bands that can be wound around the chest (at the level of the ensiform cartilage) and the abdomen (at the level of the navel). In the field of respiratory inductance plethysmography, the Konno-Mead Diagram is known, which is a Lissajous figure in which, for example, the X axis is the abdominal displacement $R_{abd}$, whereas the Y axis is the rib cage displacement $R_{rc}$.

However, in respiratory inductance plethysmography, bands must be deployed around the chest and the abdomen of the human subject. In addition, if the human subject is conscious of measurements or feels nervous during measurements, the measurements of the rib cage displacement $R_{rc}$ and the abdominal displacement $R_{abd}$ are disturbed. When the human subject is asleep, the measurements in the respiratory inductance plethysmography may be of high reliability. However, when the human subject is awake, the measurements in the respiratory inductance plethysmography may be of lower reliability.

In contrast, in determination with the use of bioelectrical impedances, for example, when the limb-lead eight-electrode method is used, current electrodes and voltage electrodes are deployed at both palms and both soles. Then, it is unnecessary to adhere current electrodes and voltage electrodes to the body trunk of the human subject, or to restrict the human body. In addition, for example, the first bioelectrical impedance $Z_a$ at the upper body trunk is about 80 percent dependent on the air entering and leaving the lungs, and only 20 percent dependent on the respiratory muscle. Accordingly, even if the human subject is conscious of measurements or feels nervous during measurements, reliability of measurements may be enhanced in comparison with respiratory inductance plethysmography. In addition, determination with the use of bioelectrical impedances is more reliable since it is more sensitive to actions related to respiration, e.g., the flow of air into and out of the lungs, and the vertical movement of the diaphragm.

Therefore, the Lissajous figure obtained from the determination of bioelectrical impedances better corresponds to actions related to respiration, e.g., the flow of air into and out of the lungs, and the vertical movement of the diaphragm in comparison with the Lissajous figure obtained by the respiratory inductance plethysmography. In addition, as described above, in the Lissajous figure obtained by the respiratory inductance plethysmography, for example, the X axis is the abdominal displacement $R_{abd}$, whereas the Y axis is the rib cage displacement $R_{rc}$. In this case, the track of the Lissajous figure for a single costal respiration and the track of the Lissajous figure for a single abdominal respiration are of a straight shape rising from bottom left to top right. The inclination angle of the upward-sloping track of the Lissajous figure with respect to the X axis is greater (nearer to 90 degrees) when the ratio of costal respiration in respiration is higher. Consequently, the shapes of the tracks of the Lissajous figures for costal respiration and abdominal respiration obtained by the respiratory inductance plethysmography are similar to each other, although the inclination angles are different from each other, so that the type of respiration cannot be easily understood from the shape of the Lissajous figure.

The same is true for a Lissajous figure, which is similar to the Konno-Mead Diagram, using the costal circumference $R_{ib}$ and the abdominal circumference $A_b$ measured by Respitrace (Trademark, AMI Inc, Ardsley, New York, U.S.A.).

The respiration characteristic analysis apparatus may further include: a display data generator adapted for generating display data for displaying a Lissajous figure showing change over time in the first bioelectrical impedance and change over time in the second bioelectrical impedance in an orthogonal coordinate system having two orthogonal coordinate axes in which a first axis is the first bioelectrical impedance and a second axis is the second bioelectrical impedance; and a centering value generator adapted for generating a first centering value that is an average of the first bioelectrical impedances within a past unit time on the basis of change over time in the first bioelectrical impedance, and for generating a second centering value that is an average of the second bioelectrical impedances within a past unit time on the basis of change over time in the second bioelectrical impedance, the first centering value being a standard level of change over time in the first bioelectrical impedance, the second centering value being a standard level of change over time in the second bioelectrical impedance. The display data generator may be adapted for generating the display data for displaying the Lissajous figure so that a position on the Lissajous figure defined by the first centering value and the second centering value is located at a center of a screen in which the Lissajous figure is displayed. In this case, since the location of the Lissajous figure is centered with respect to the screen, visualization of the Lissajous figure can be facilitated.

The respiration characteristic analysis apparatus may further include: a display data generator adapted for generating display data for displaying a Lissajous figure showing change over time in the first bioelectrical impedance and change over time in the second bioelectrical impedance in an orthogonal coordinate system having two orthogonal coordinate axes in which a first axis is the first bioelectrical impedance and a second axis is the second bioelectrical impedance; and a centering value generator adapted for generating a first centering value that is an average of the first bioelectrical impedances within a past unit time on the basis of change over time in the first bioelectrical impedance, and for generating a second centering value that is an average of the second bioelectrical impedances within a past unit time on the basis of change over time in the second bioelectrical impedance, the first centering value being a standard level of change over time in the first bioelectrical impedance, the second centering value being a standard level of change over time in the second bioelectrical impedance. When the display data generator generates the display data for displaying the Lissajous figure, the display data generator may be adapted for executing a first location centering process in which the Lissajous figure is centered in the first axis with respect to a screen in which the Lissajous figure is displayed on the basis of the first centering value, and may be adapted for executing a second location centering process in which the Lissajous figure is centered in the second axis with respect to the screen on the basis of the second centering value. The display data generator may be adapted for executing the second location centering process less frequently than that for the first location centering process.

As in Fig. 45, which shows Lissajous figures in draw-in respiration and costal respiration, both tracks of the Lissajous figures are of a straight shape rising from bottom left to top right, but locations of both tracks are different in the axis indicating the second bioelectrical impedance $Z_b$ (X axis in Fig. 45). If centering of the displayed location of the Lissajous figure is repeated at small intervals, the Lissajous figure will always be displayed at the center of the screen, and it will be difficult to understand whether respiration of the human subject is draw-in respiration or costal respiration from looking at the Lissajous figure. In this aspect, the second location centering process is executed less frequently than that for the first location centering process. For example, the first location centering process may be executed at every respiration whereas the second location centering process may be executed only once (for example, at an initial stage of the process). It will be easy to understand whether respiration of the human subject is draw-in respiration or costal respiration from observation of the Lissajous figure. This is because the locations of tracks of the Lissajous figures for draw-in respiration and costal respiration will become different in the second axis for a certain period, even though the shapes of the tracks are similar. In addition, it is possible to reduce power consumption at the respiration determination apparatus by reducing the frequency of the second location centering process. Although it is performed less frequently, by executing the second location centering process, the Lissajous figure can be displayed at the center of the screen.

The respiration characteristic analysis apparatus may further include a local-maximum-and-minimum decider adapted for deciding a first local maximum that is a local maximum of change in the first bioelectrical impedance, for deciding a first local minimum that is a local minimum of change in the first bioelectrical impedance, for deciding a second local maximum that is a local maximum of change in the second bioelectrical impedance, and for deciding a second local minimum that is a local minimum of change in the second bioelectrical impedance. The display data generator may be adapted for generating the display data for displaying the Lissajous figure so that a range of the Lissajous figure on a screen in which the Lissajous figure is displayed in the first and second axes is adjusted on the basis of the first local maximum, the first local minimum, the second local maximum, and the second local minimum.

In this case, the Lissajous figure can be displayed at a suitable size with respect to the screen by adjusting the range in the first and second axes, and can be centered with respect to the screen, so that visualization of the Lissajous figure can be facilitated.

The respiration characteristic analysis apparatus may further include a local-maximum-and-minimum decider adapted for deciding a first local maximum that is a local maximum of change in the first bioelectrical impedance, for deciding a first local minimum that is a local minimum of change in the first bioelectrical impedance, for deciding a second local maximum that is a local maximum of change in the second bioelectrical impedance, and for deciding a second local minimum that is a local minimum of change in the second bioelectrical impedance. When the display data generator generates the display data for displaying the Lissajous figure, the display data generator may be adapted for executing a first range adjustment process in which a range of the Lissajous figure on a screen in which the Lissajous figure is displayed in the first axis is adjusted on the basis of the first local maximum and the first local minimum, and may be adapted for executing a second range adjustment process in which a range of the Lissajous figure on the screen in the second axis is adjusted on the basis of the second local maximum and the second local minimum. The display data generator may be adapted for executing the second range adjustment process less frequently than that for the first range adjustment process.

In this case, it will be easy to understand whether respiration of the human subject is draw-in respiration or costal respiration from looking at the Lissajous figure. In addition, it is possible to reduce power consumption at the respiration determination apparatus by reducing the frequency of the second range adjustment process. Although the frequency is less, by executing the second range adjustment process, the Lissajous figure can be displayed at a suitable size with respect to the screen.

The display data generator may be adapted for generating the display data for displaying the Lissajous figure so that a displaying manner for a track of the Lissajous figure for a latest single respiration is different from a displaying manner for a track of the Lissajous figure for past respirations.

In a Lissajous figure that continually shows status of multiple respirations, for example, as shown in Fig. 34 or 35, if the manner for displaying the track within a single respiration is the same as that for the track within another single respiration, it is difficult to identify the track for the latest single respiration. However, in this aspect, it is possible to easily understand the track for the latest single respiration in view of the variation of the displaying manner (e.g., color or line type).

The display data generator may be adapted for generating the display data for displaying the Lissajous figure so that a displaying manner for tracks of the Lissajous figure is changed depending on an elapsed time.

For example, the display data generator may lighten the color as the elapsed time increases. In this case, the newer the track, the fainter the color of the track. It is easy to identify the tracks for newer respirations (e.g., the track for the latest respiration).

The display data generator may be adapted for further generating target display data for displaying a target Lissajous figure showing a target model of breathing having a type and a magnitude of respiration to be performed by the human subject for guiding the human subject to perform breathing.

In this case, in addition to the measured Lissajous figure showing the status of breathing of the human subject, a target Lissajous figure showing a target model of breathing to be performed by the human subject is displayed. Thus, the human subject can train for breathing comparing the two Lissajous figures. The human subject may focus on making the track of the Lissajous figure showing the status of breathing of the human subject coincide with the track of the target Lissajous figure, so as to learn the target breathing. Thus, the human subject is effectively guided to perform appropriate breathing by the use of the Lissajous figure for breathing guidance.

The display data generator may be adapted for generating the display data for the measured Lissajous figure and the target display data so that the measured Lissajous figure showing the status of breathing of the human subject and the target Lissajous figure are overlaid on a screen. In this case, it is possible to easily understand the difference between the target respiration and the actual respiration. The display data generator may be adapted for generating the display data for the measured Lissajous figure and the target display data so that a displaying manner for a track of the measured Lissajous figure is different from a displaying manner for a track of the target Lissajous figure. In this case, it is possible to easily distinguish the Lissajous figures in view of the variation of the displaying manner (e.g., color or line type), even when the measured Lissajous figure showing the status of breathing of the human subject and the target Lissajous figure are overlaid on the screen.

The respiration characteristic analysis apparatus may further include: an inclination angle calculator adapted for calculating an inclination angle of a track of the Lissajous figure; and a ventilation capability determiner adapted for comparing the inclination angle calculated by the inclination angle calculator with a predetermined reference inclination angle, so as to decide whether or not a lung ventilation capability of the human subject is good or bad.

In this case, it is possible to easily determine whether the lung ventilation capability is good or bad on the basis of the inclination angle of the track of the Lissajous figure. Depending on the posture of the human subject (standing, sitting, or supine), the inclination angle may be varied. Accordingly, multiple reference inclination angles may be defined depending on the posture.

The respiration characteristic analysis apparatus may further include: a respiration depth calculator adapted for calculating a respiration depth of the human subject at every respiration of the human subject; and a graph generator adapted for generating display data for indicating a graph showing change over time of respiration depth calculated by the respiration depth calculator, in such a manner that the graph is nonlinearly compressed in a direction of time axis and time intervals are more compressed than later time intervals, so that a time resolution for later time intervals is higher than that for earlier time intervals.

The time for breathing training may frequently be long, e.g., ten minutes or more. In order to display the entire graph from the start of measurement to the current time, it is preferable that the graph be compressed in the direction of the time axis. If the entire graph is uniformly compressed, the time resolution will be reduced uniformly in the graph. This results in it being difficult to recognize details of the magnitude of the latest respirations. In this aspect, the graph is nonlinearly compressed in the direction of the time axis and earlier time intervals are more compressed than later time intervals, so that the time resolution for later time intervals is higher than that for earlier time intervals.

The respiration characteristic analysis apparatus may further include: a memory adapted for storing training menus that are used for training the human subject for breathing, the training menus being classified into rankings of respiration capability, the memory storing requirements for advancing through the rankings; a respiration capability determiner adapted for determining a respiration capability of the human subject on the basis of change over time in each of the first bioelectrical impedance and the second bioelectrical impedance; and a training manager adapted for referring to the memory for identifying a ranking corresponding to the respiration capability determined by the respiration capability determiner, and for executing a process for training the human subject for breathing using the training menus corresponding to the ranking. The training manager may be adapted for advancing the ranking to a next ranking if the requirement for advancing through the ranking is satisfied.

In this case, the human subject can effectively train for breathing in accordance with the training menus that match the respiration capability of the human subject. The training menus are prepared at each ranking of respiration capability, and if the requirement defined at each ranking is satisfied, the human subject can advance to the next ranking. Accordingly, the training process has a game element by which the human subject is amused, and the human subject is encouraged to train for breathing.

The bioelectrical impedance determiner may be adapted for determining a right first bioelectrical impedance at the right upper body trunk of the human subject including the upper lobe of the right lung of the human subject and excluding the abdomen of the human subject, for determining a left first bioelectrical impedance at the left upper body trunk of the human subject including the upper lobe of the left lung of the human subject and excluding the abdomen of the human subject, and for determining the second bioelectrical impedance at the middle body trunk. The analyzer may be adapted for calculating indicative information that is used for identifying whether respiration of the human subject is abdominal or costal, on the basis of change over time in each of the right first bioelectrical impedance, the left first bioelectrical impedance, and the second bioelectrical impedance. In this case, the respiration characteristic analysis apparatus may be used as a respiration type determination apparatus. In this case, the type of respiration of the right lung or the left lung can be decided.

The analyzer may not only calculate the indicative information, but it may also decide whether respiration of the human subject is abdominal respiration or costal respiration, on the basis of the indicative information. The analyzer may report the decision result to the human subject or another person, or may output a signal indicating the decision result.

The respiration characteristic analysis apparatus may further include a display data generator adapted for generating first display data for displaying a first Lissajous figure showing change over time in the right first bioelectrical impedance and change over time in the second bioelectrical impedance in an orthogonal coordinate system having two orthogonal coordinate axes in which a first axis is the right first bioelectrical impedance and a second axis is the second bioelectrical impedance, and for generating second display data for displaying a second Lissajous figure showing change over time in the left first bioelectrical impedance and change over time in the second bioelectrical impedance in an orthogonal coordinate system having two orthogonal coordinate axes in which a first axis is the left first bioelectrical impedance and a second axis is the second bioelectrical impedance.

In this case, since two Lissajous figures for the right lung and the left lung are displayed, the type and the magnitude of respiration with respect to the right lung and the left lung can be understood. By comparing two Lissajous figures, it is possible to easily understand the difference between the respiration capabilities of the right lung and the left lung. In addition, it is possible to train for breathing in the right lung and the left lung, respectively. There is no significant difference between the respiration capabilities of the right lung and the left lung of a physically unimpaired person. However, if one of the right lung and the left lung is diseased, there is a significant difference between the respiration capabilities of the right lung and the left lung. If one of the right lung and the left lung was diseased, there may be a difference between the respiration capabilities of the right lung and the left lung. A method for improving the respiration capability of only the left lung is one in which the human subject repeats respiration while a load is applied to the left lung by positioning the left arm behind the right shoulder and pushing the left elbow backward with the right hand. This method is suitable for, for example, a person whose respiration capability of the left lung is lower than the respiration capability of the right lung.

The display data generator may be adapted for generating the first display data for displaying the first Lissajous figure and the second display data for displaying the second Lissajous figure so that the first Lissajous figure and the second Lissajous figure are overlaid on a screen. In this case, since the first Lissajous figure for the right lung and the second Lissajous figure for the left lung are overlaid on a screen, it is possible to easily understand the difference between the respiration capabilities of the right lung and the left lung.

The display data generator may be adapted for generating the first display data for displaying the first Lissajous figure and the second display data for displaying the second Lissajous figure so that a displaying manner for the first Lissajous figure is different from a displaying manner for the second Lissajous figure. In this case, it is possible to easily distinguish the first and second Lissajous figures in view of the variation of the displaying manner (e.g., color or line type) although the first and second Lissajous figures are overlaid on the screen.

The respiration characteristic analysis apparatus may further include a track analyzer adapted for detecting differences between a track of the first Lissajous figure and a track of the second Lissajous figure. The display data generator may be adapted for generating the first display data for displaying the first Lissajous figure and the second display data for displaying the second Lissajous figure so that the differences are highlighted on a screen. In this case, it is possible to easily understand the difference between the respiration capabilities of the right lung and the left lung.

In another aspect of the present invention, a respiration characteristic analysis apparatus includes: an input part for inputting to the respiration characteristic analysis apparatus a first bioelectrical impedance at the upper body trunk of a human subject including the upper lobes of the lungs of the human subject and excluding the abdomen of the human subject and a second bioelectrical impedance at the middle body trunk of the human subject including the median and lower lobes of the lungs of the human subject and the abdomen of the human subject, the first bioelectrical impedance and the second bioelectrical impedance being determined at a bioelectrical impedance determination apparatus; and an analyzer adapted for analyzing respiration characteristics of the human subject on the basis of change over time in each of the first bioelectrical impedance and the second bioelectrical impedance.

This respiration characteristic analysis apparatus also analyzes respiration characteristics of the human subject. For example, it is possible to decide the type of respiration of the human subject (abdominal respiration or costal respiration, or draw-in respiration). This respiration characteristic analysis apparatus may be, for example, a game machine, a personal computer, or a portable electrical device (e.g., a cell phone).

The analyzer may not only calculate the indicative information, but also decide that respiration of the human subject is abdominal respiration, costal respiration, or draw-in respiration, on the basis of the indicative information. The analyzer may report the decision result to the human subject or another person, or may output a signal indicating the decision result.

According to the present invention, there is provided a respiration characteristic analysis system including: a bioelectrical impedance determiner adapted for determining a first bioelectrical impedance at the upper body trunk of a human subject including the upper lobes of the lungs of the human subject and excluding the abdomen of the human subject and a second bioelectrical impedance at the middle body trunk of the human subject including the median and lower lobes of the lungs of the human subject and the abdomen of the human subject; and an analyzer adapted for analyzing a respiration characteristic of the human subject on the basis of change over time in each of the first bioelectrical impedance and the second bioelectrical impedance determined by the bioelectrical impedance determiner.

This respiration characteristic analysis system also analyzes respiration characteristics of the human subject. For example, it is possible to decide the type of respiration of the human subject (abdominal respiration or costal respiration, or draw-in respiration). This respiration characteristic analysis system may include, for example, a game machine, a personal computer, or a portable electrical device (e.g., a cell phone).

The analyzer may not only calculate the indicative information, but also decide that respiration of the human subject is abdominal respiration, costal respiration, or draw-in respiration, on the basis of the indicative information. The analyzer may report the decision result to the human subject or another person, or may output a signal indicating the decision result.

The analyzer may be adapted for calculating indicative information that is used for identifying whether or not respiration of the human subject is draw-in respiration (respiration in which inhalation and exhalation are repeated with the abdomen held in a constricted position), on the basis of change over time in each of the first bioelectrical impedance and the second bioelectrical impedance. It is possible to calculate indicative information that is used for identifying whether or not respiration of the human subject is draw-in respiration as similar to the manner for calculating the indicative information that is used for identifying whether respiration of the human subject is abdominal or costal. Respiration of the human subject may be assumed as draw-in respiration if the ratio $\Delta R_{ib}/\Delta A_b$ is greater than a predetermined threshold and if the current second centering value generated by the centering value generator is equal to or greater than the sum of a standard second centering value in costal respiration of the human subject and a predetermined value.

The bioelectrical impedance determiner may be adapted for determining a right first bioelectrical impedance at the right upper body trunk of the human subject including the upper lobe of the right lung of the human subject and excluding the abdomen of the human subject, a left first bioelectrical impedance at the left upper body trunk of the human subject including the upper lobe of the left lung of the human subject and excluding the abdomen of the human subject, and the second bioelectrical impedance at the middle body trunk of the human subject including the median and lower lobes of the lungs of the human subject and the abdomen of the human subject. The respiration characteristic analysis apparatus may further include a display data generator adapted for generating first display data for displaying a first Lissajous figure showing change over time in the right first bioelectrical impedance and change over time in the second bioelectrical impedance in an orthogonal coordinate system having two orthogonal coordinate axes in which a first axis is the right first bioelectrical impedance and a second axis is the second bioelectrical impedance, and for generating second display data for displaying a second Lissajous figure showing change over time in the left first bioelectrical impedance and change over time in the second bioelectrical impedance in an orthogonal coordinate system having two orthogonal coordinate axes in which a first axis is the left first bioelectrical impedance and a second axis is the second bioelectrical impedance.

This respiration characteristic analysis apparatus can be used as a breathing training apparatus. Since two Lissajous figures for the right lung and the left lung are displayed, the type and the magnitude of respiration with respect to the right lung and the left lung can be understood. By comparing two Lissajous figures, it is possible to easily understand the difference between the respiration capabilities of the right lung and the left lung. In addition, it is possible to train for breathing of the right lung and the left lung, respectively. There is no significant difference between the respiration capabilities of the right lung and the left lung of a physically unimpaired person. However, if one of the right lung and the left lung is diseased, there is a significant difference between the respiration capabilities of the right lung and the left lung. If one of the right lung and the left lung was previously diseased, there may be a difference between the respiration capabilities of the right lung and the left lung. A method for improving the respiration capability of only the left lung is one in which the human subject repeats breathing while a load is applied to the left lung by positioning the left arm behind the right shoulder and pushing the left elbow backward with the right hand. This method is suitable for, for example, a person whose respiration capability of the left lung is lower than the respiration capability of the right lung.

The display data generator may be adapted for generating the first display data for displaying the first Lissajous figure and the second display data for displaying the second Lissajous figure so that the first Lissajous figure and the second Lissajous figure are overlaid on a screen. In this case, since the first Lissajous figure for the right lung and the second Lissajous figure for the left lung are overlaid on a screen, it is possible to easily understand the difference between the respiration capabilities of the right lung and the left lung.

The display data generator may be adapted for generating the first display data for displaying the first Lissajous figure and the second display data for displaying the second Lissajous figure so that a displaying manner for the first Lissajous figure is different from a displaying manner for the second Lissajous figure. In this case, it is possible to easily distinguish the first and second Lissajous figures in view of the variation of the displaying manner (e.g., color or line type) although the first and second Lissajous figures are overlaid on the screen.

The respiration characteristic analysis apparatus may further include a track analyzer adapted for detecting differences between a track of the first Lissajous figure and a track of the second Lissajous figure. The display data generator may be adapted for generating the first display data for displaying the first Lissajous figure and the second display data for displaying the second Lissajous figure so that the differences are highlighted on a screen. In this case, it is possible to easily understand the difference between the respiration capabilities of the right lung and the left lung.

The display data generator may be adapted for generating the display data for displaying the Lissajous figure so that a position on the Lissajous figure defined by the first centering value and the second centering value is located at a center of a screen in which the Lissajous figure is displayed. In this case, since the location of the Lissajous figure is centered with respect to the screen, visualization of the Lissajous figure can be facilitated.

When the display data generator generates the display data for displaying the Lissajous figure, the display data generator may be adapted for executing a first location centering process in which the Lissajous figure is centered in the first axis with respect to a screen in which the Lissajous figure is displayed on the basis of the first centering value, and may be adapted for executing a second location centering process in which the Lissajous figure is centered in the second axis with respect to the screen on the basis of the second centering value. The display data generator is adapted for executing the second location centering process less frequently than that for the first location centering process.

In another aspect of the present invention, a respiration characteristic analysis apparatus includes: an input part for inputting to the respiration characteristic analysis apparatus a first bioelectrical impedance at the upper body trunk of a human subject including the upper lobes of the lungs of the human subject and excluding the abdomen of the human subject and a second bioelectrical impedance at the middle body trunk of the human subject including the median and lower lobes of the lungs of the human subject and the abdomen of the human subject, the first bioelectrical impedance and the second bioelectrical impedance being determined at a bioelectrical impedance determination apparatus; and a display data generator adapted for generating display data for displaying a Lissajous figure showing change over time in the first bioelectrical impedance and change over time in the second bioelectrical impedance in an orthogonal coordinate system having two orthogonal coordinate axes in which a first axis is the first bioelectrical impedance and a second axis is the second bioelectrical impedance.

This respiration characteristic analysis apparatus can be used as a breathing training apparatus. Since the Lissajous figure is used as biofeedback information for training for appropriate breathing, the human subject can train for breathing effectively. This breathing training apparatus may be, for example, a game machine, a personal computer, or a portable electrical device (e.g., a cell phone).

In another aspect of the present invention, a respiration characteristic analysis system may include: an input part for inputting to a respiration characteristic analysis apparatus a first bioelectrical impedance at the upper body trunk of a human subject including the upper lobes of the lungs of the human subject and excluding the abdomen of the human subject and a second bioelectrical impedance at the middle body trunk of the human subject including the median and lower lobes of the lungs of the human subject and the abdomen of the human subject to the respiration characteristic analysis apparatus, the first bioelectrical impedance and the second bioelectrical impedance being determined at a bioelectrical impedance determination apparatus; a display data generator adapted for generating display data for displaying a Lissajous figure showing change over time in the first bioelectrical impedance and change over time in the second bioelectrical impedance in an orthogonal coordinate system having two orthogonal coordinate axes in which a first axis is the first bioelectrical impedance and a second axis is the second bioelectrical impedance; and a display device adapted for displaying the Lissajous figure on the basis of the display data generated by the display data generator.

This respiration characteristic analysis system can be used as a breathing training system. Since the Lissajous figure is used as biofeedback information for training for appropriate breathing, the human subject can train breathing effectively. This breathing training system may include, for example, a game machine, a personal computer, or a portable electrical device (e.g., a cell phone).

BRIEF DESCRIPTION OF THE DRAWINGS

With reference to the accompanying drawings, various embodiments of the present invention will be described hereinafter. In the drawings:

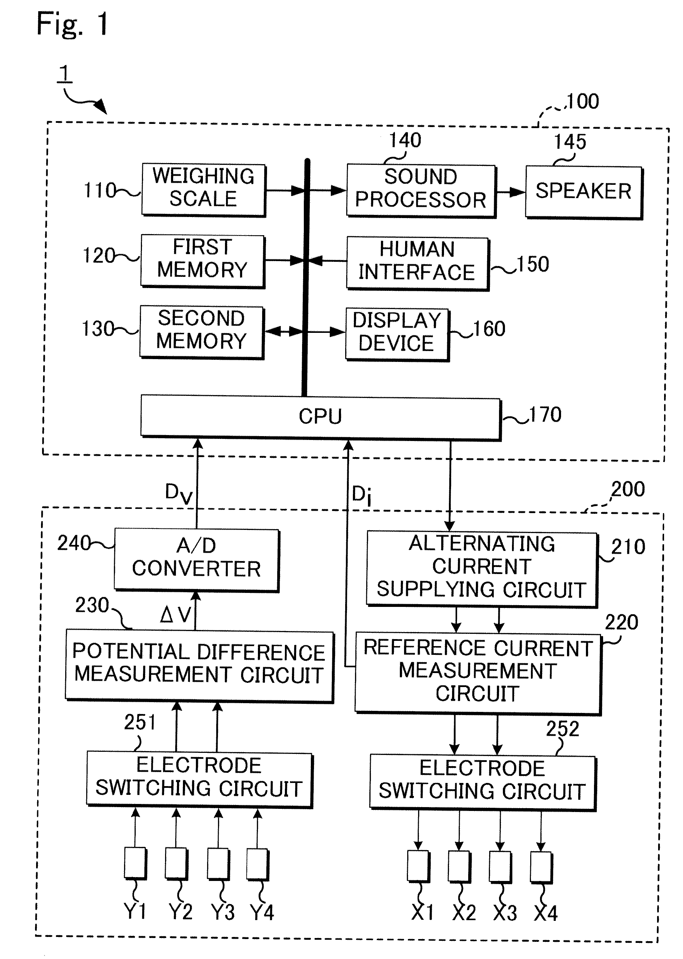

Fig. 1 is a block diagram showing an electrical structure of a body condition determination apparatus of an embodiment according to the present invention;

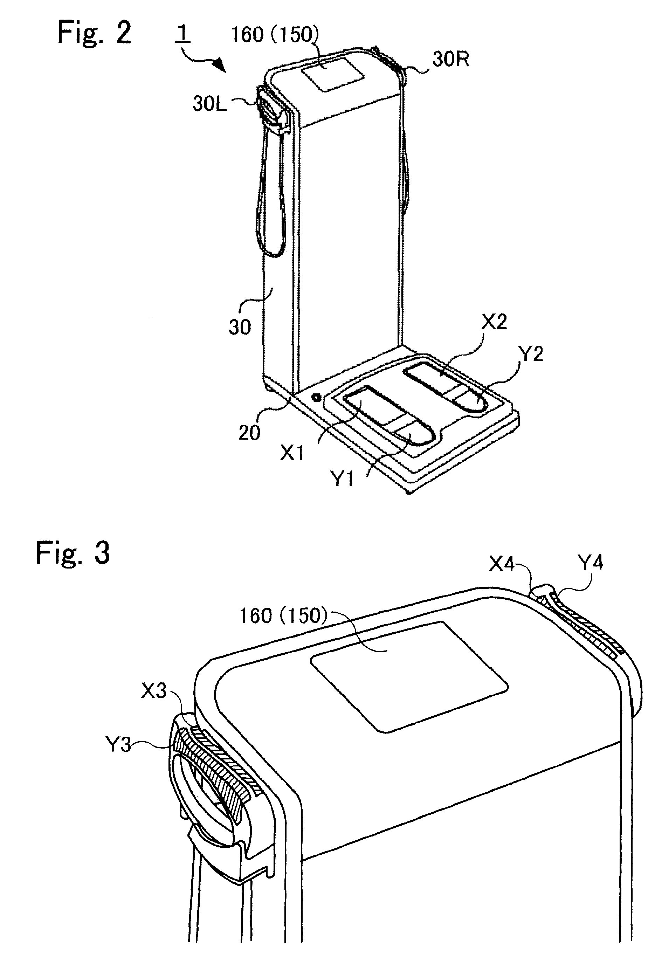

Fig. 2 is a perspective view showing the body condition determination apparatus;
Fig. 3 is an enlarged view of a part of Fig. 2;

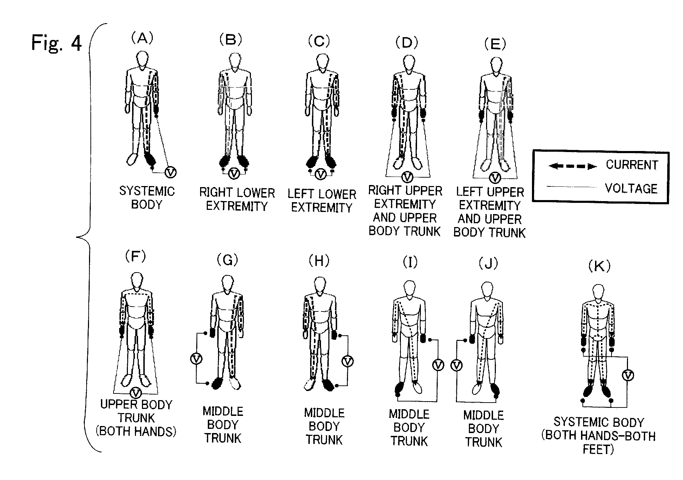

Fig. 4 is a diagram for explaining body regions determined by using selected current electrodes and selected voltage electrodes;

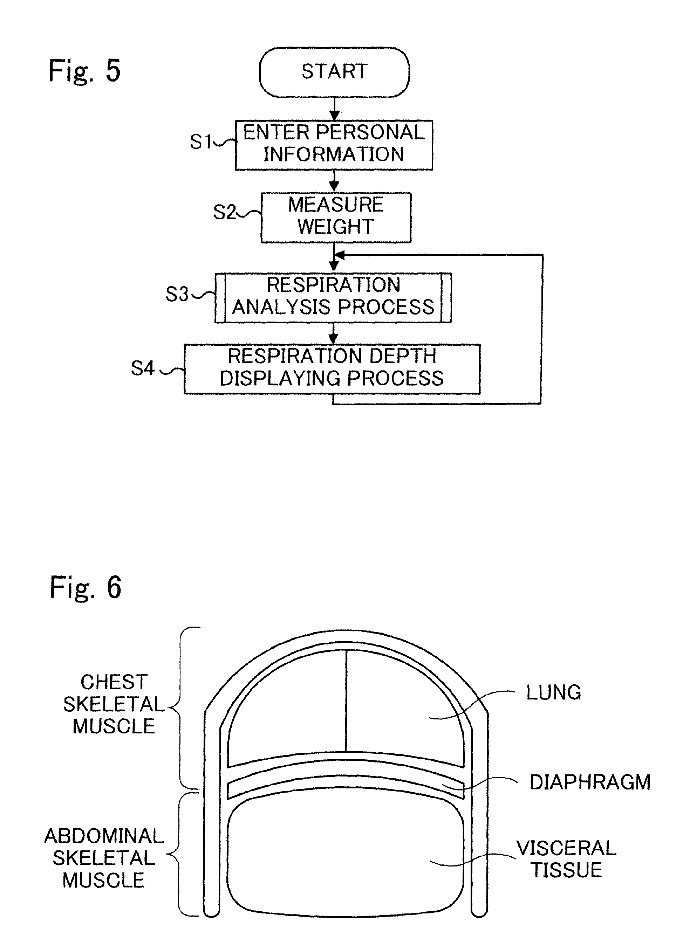

Fig. 5 is a flow chart showing an operation of the body condition determination apparatus;
Fig. 6 is a schematic view showing tissues in the body trunk of humans;

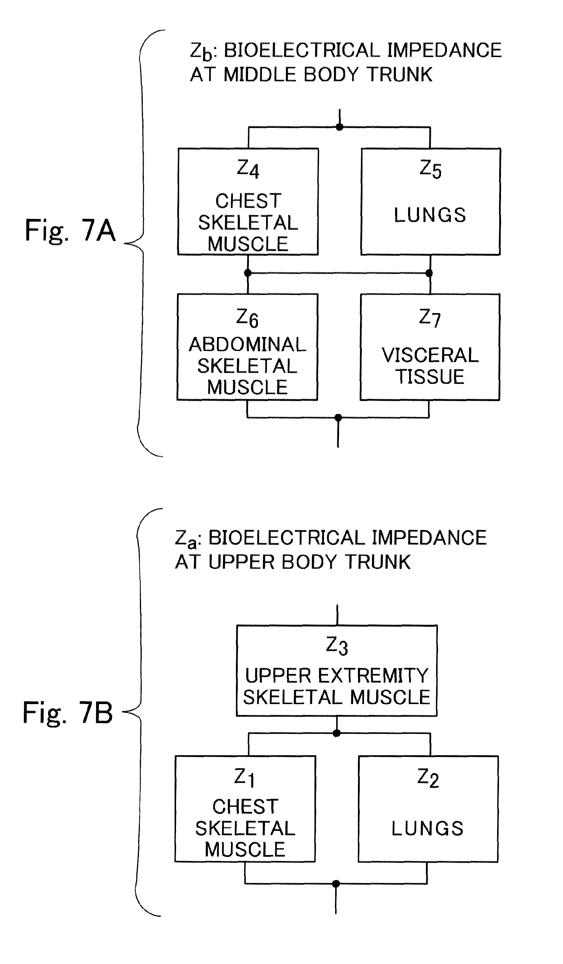

Fig. 7A shows an equivalent circuit model of the middle body trunk of humans;
Fig. 7B shows an equivalent circuit model of the upper body trunk of humans;

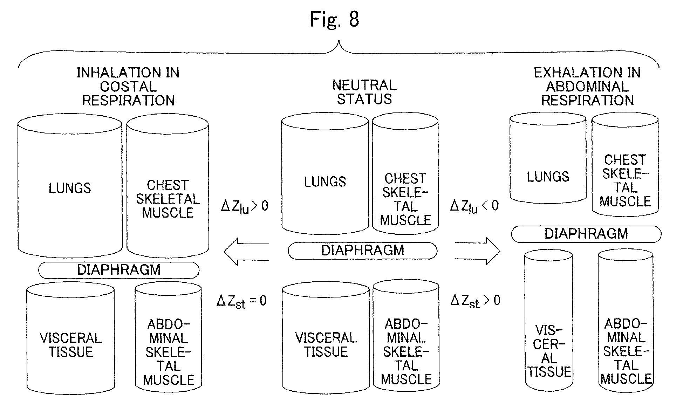

Fig. 8 is a diagram showing relationships between respiration and change in bioelectrical impedance;
Fig. 9 is a graph showing change over time of each of bioelectrical impedances at the middle body trunk and the upper body trunk in abdominal respiration;
Fig. 10 is a graph showing change over time of each of bioelectrical impedances at the middle body trunk and the upper body trunk in costal respiration;
Fig. 11 is a graph showing change over time of each of costal and abdominal circumferences in abdominal respiration;

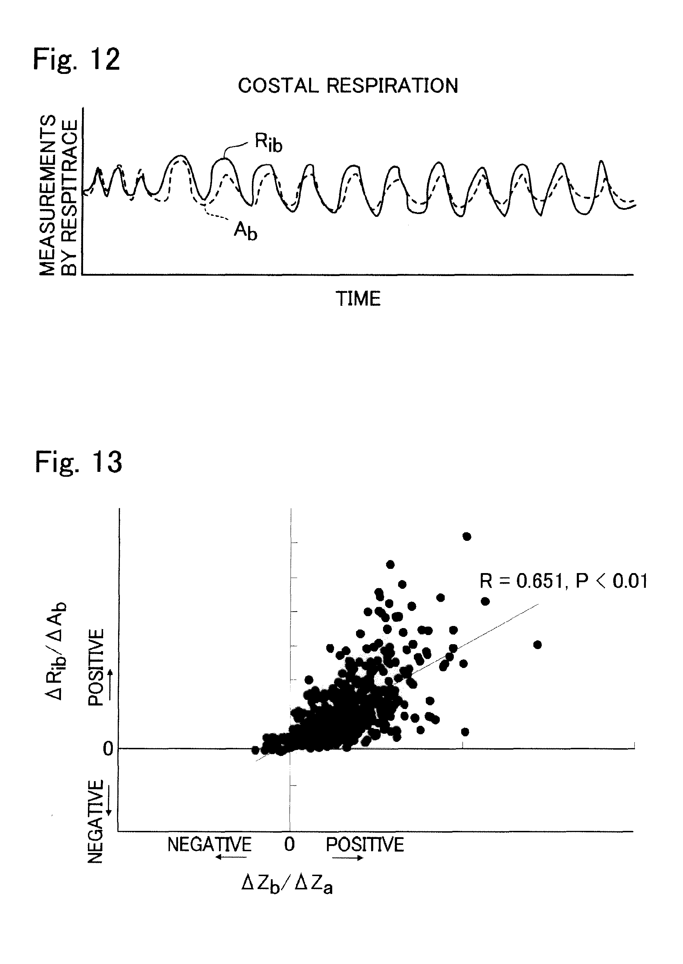

Fig. 12 is a graph showing change over time of each of costal and abdominal circumferences in costal respiration;
Fig. 13 is a graph showing relationships between a ratio of change in costal circumference to change in abdominal circumference and a ratio of change in bioelectrical impedance at the middle body trunk to change in bioelectrical impedance at the upper body trunk;

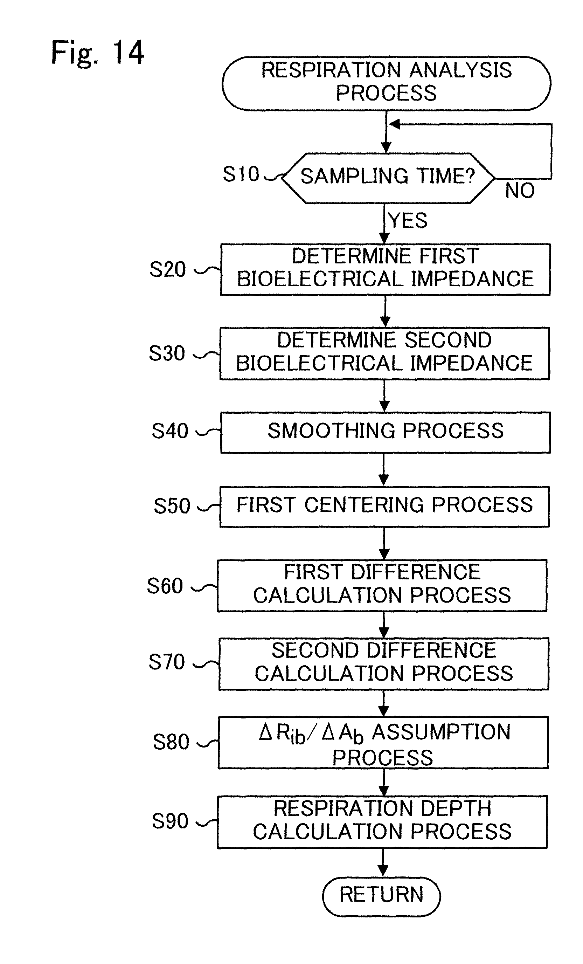

Fig. 14 is a flow chart showing an example of a respiration analysis process executed in the body condition determination apparatus;

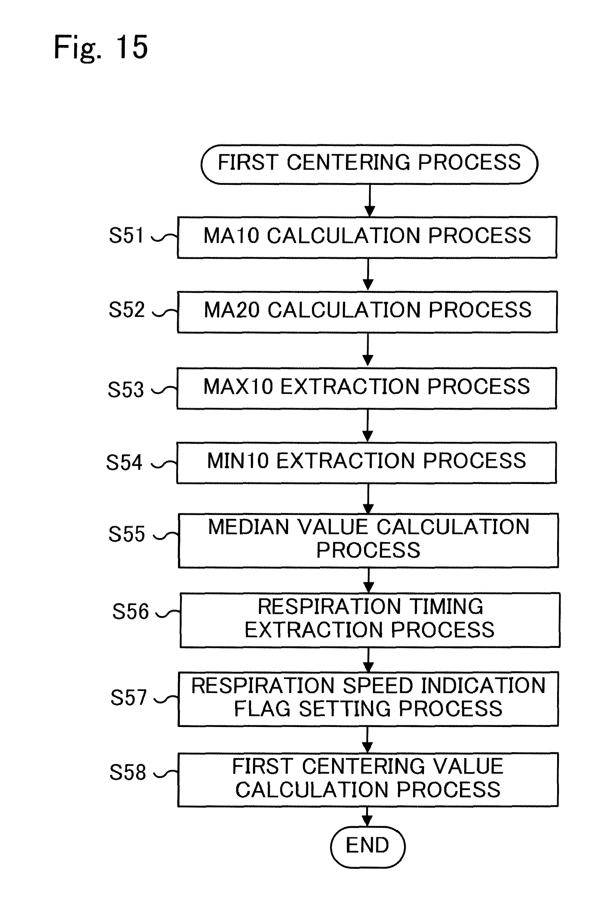

Fig. 15 is a flow chart showing an example of a first centering process executed in the body condition determination apparatus;

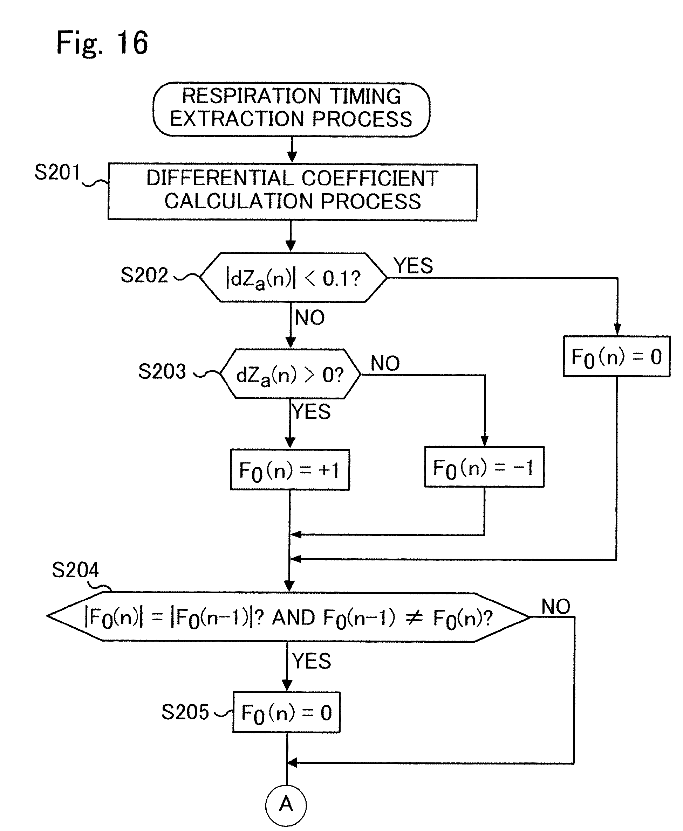

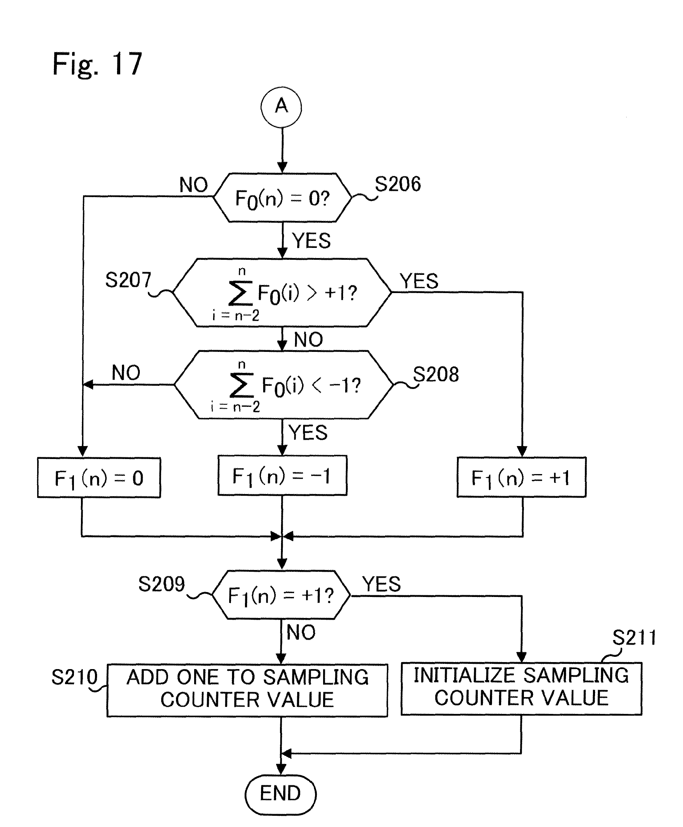

Figs. 16 and 17 form a flow chart showing an example of a respiration timing extraction process executed in the body condition determination apparatus;

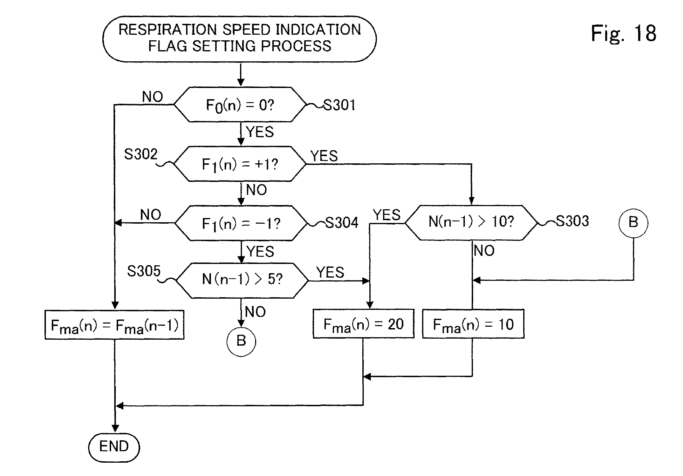

Fig. 18 is a flow chart showing an example of a respiration speed indication flag setting process executed in the body condition determination apparatus;

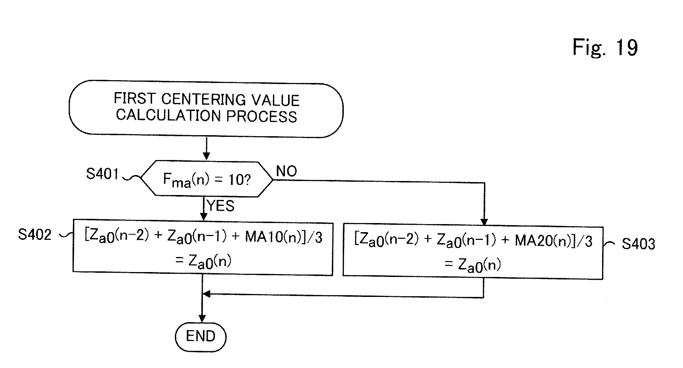

Fig. 19 is a flow chart showing an example of a first centering value calculating process executed in the body condition determination apparatus;

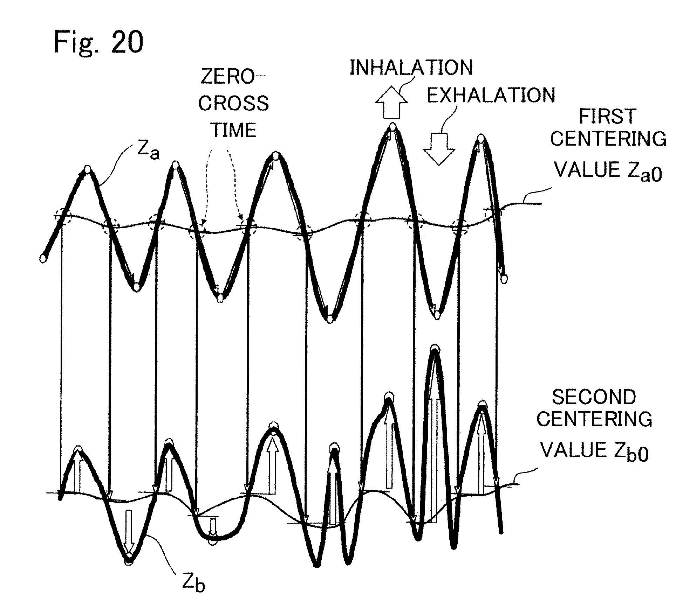

Fig. 20 is a graph for explaining a scheme for generating a second centering value;

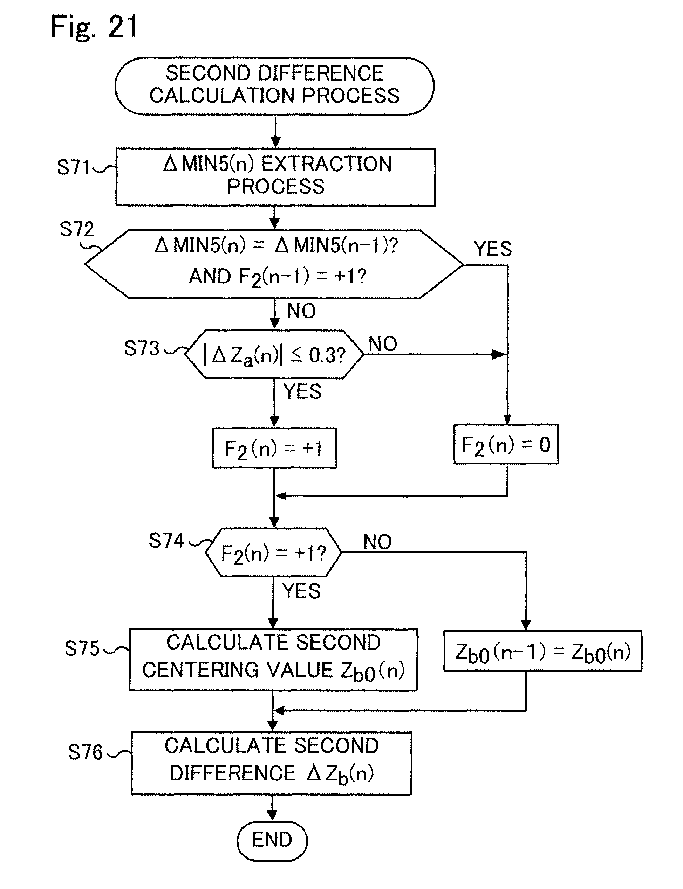

Fig. 21 is a flow chart showing an example of a second difference calculating process executed in the body condition determination apparatus;

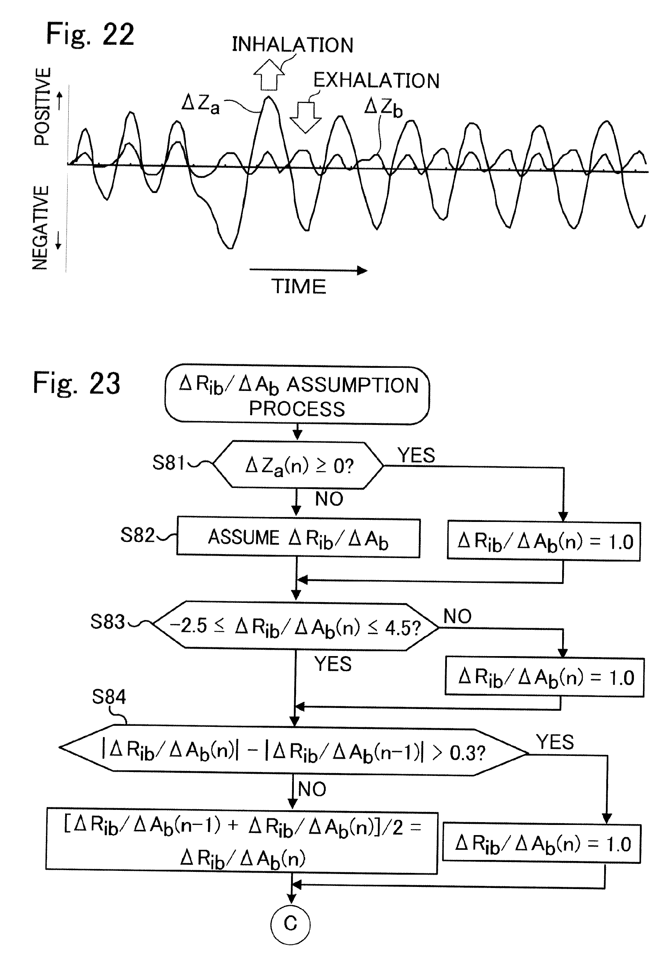

Fig. 22 is a graph showing change over time of each of a first difference and a second difference in abdominal respiration;

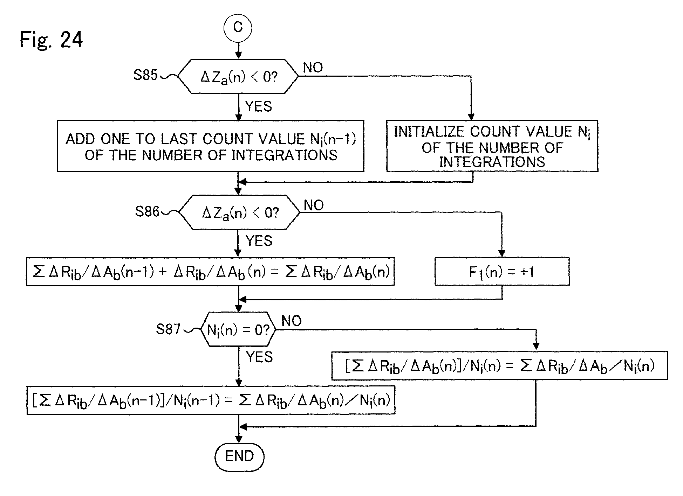

Figs. 23 and 24 form a flow chart showing an example of a $\Delta R_{ib}/\Delta A_b$ assumption process executed in the body condition determination apparatus;

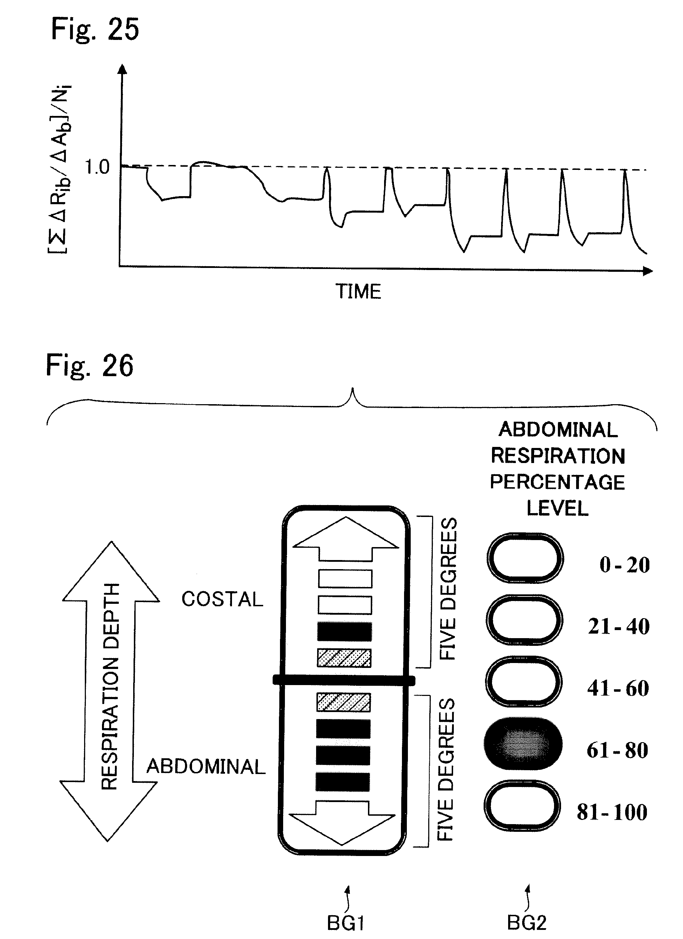

Fig. 25 is a graph showing the algorithmic results of the $\Delta R_{ib}/\Delta A_b$ assumption process;

Fig. 26 is a view showing an image shown on a display device of the body condition determination apparatus;

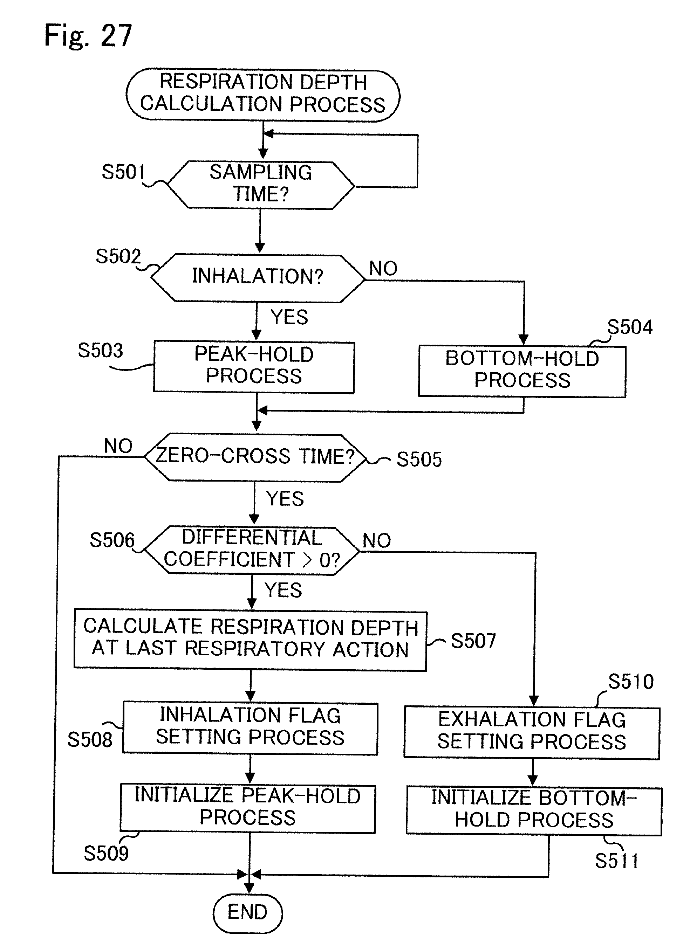

Fig. 27 is a flow chart showing an example of a respiration depth calculating process executed in the body condition determination apparatus;

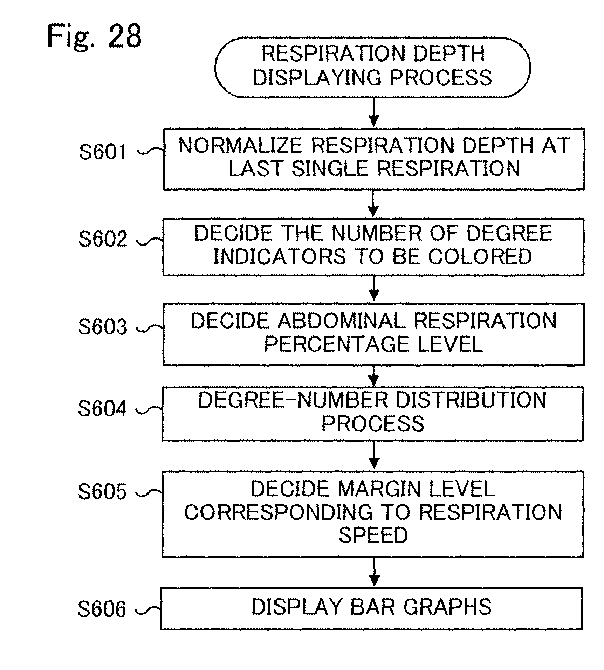

Fig. 28 is a flow chart showing an example of a respiration depth displaying process executed in the body condition determination apparatus;

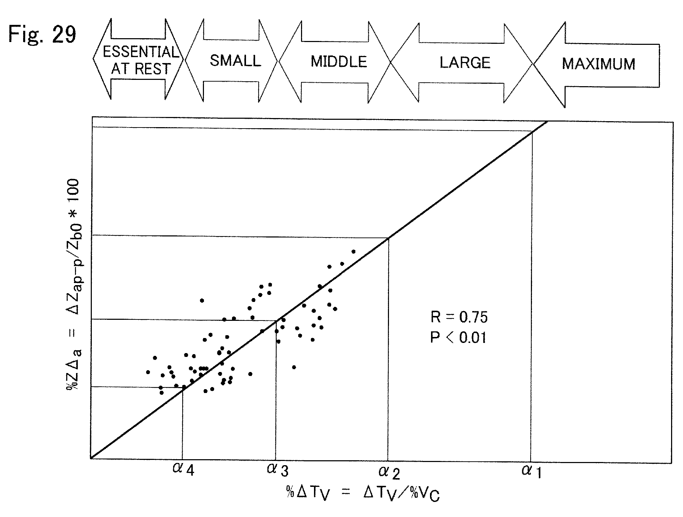

Fig. 29 is a diagram showing relationships between a respiration depth and a one-time ventilation volume;

Fig. 30 is graph showing change over time of each of bioelectrical impedances at the upper body trunk and the middle body trunk;

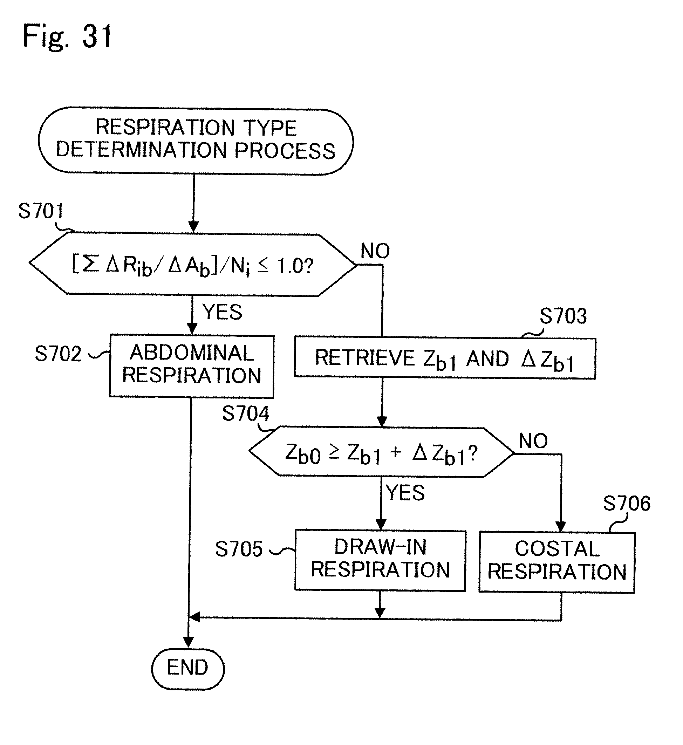

Fig. 31 is a flow chart showing an example of a respiration type determination process executed in the body condition determination apparatus;

Fig. 32 is a diagram showing a Lissajous figure obtained through a single costal respiratory action;

Fig. 33 is a diagram showing a Lissajous figure obtained through a single abdominal respiratory action;

Fig. 34 is a diagram showing a Lissajous figure obtained through multiple costal respiratory actions;

Fig. 35 is a diagram showing a Lissajous figure obtained through multiple abdominal respiratory actions;

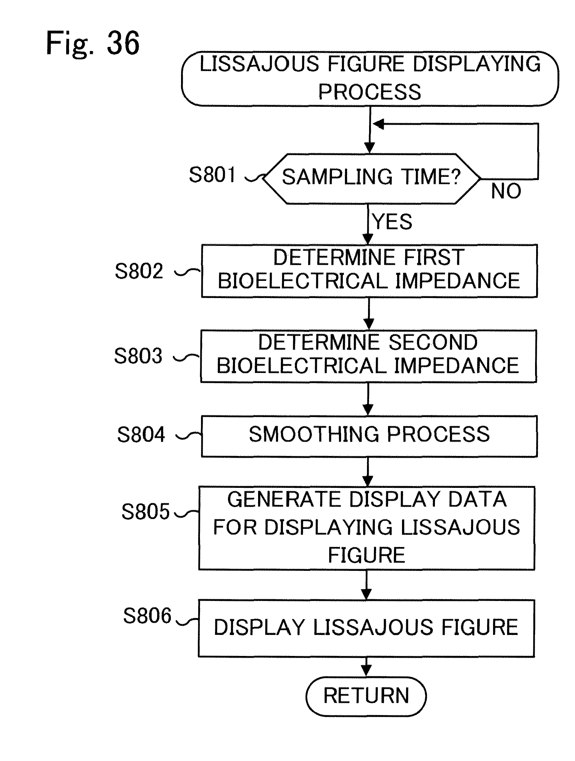

Fig. 36 is a flow chart showing an example of a Lissajous figure displaying process executed in the body condition determination apparatus;

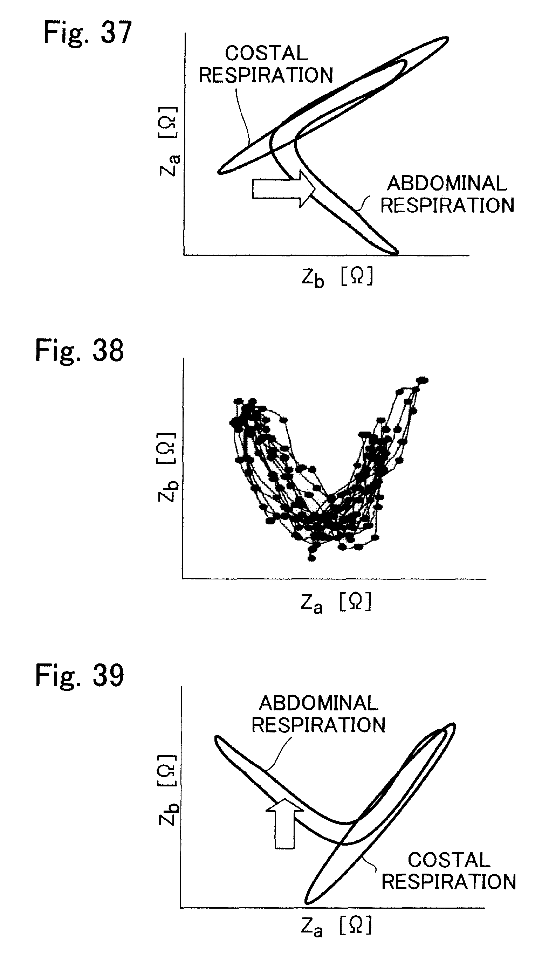

Fig. 37 is a diagram showing change in the Lissajous figure when a human subject switches from costal respiration to abdominal respiration;

Fig. 38 is a diagram showing a Lissajous figure in which the X axis and the Y axis are replaced;

Fig. 39 is a diagram showing change in the Lissajous figure when a human subject switches from costal respiration to abdominal respiration, in which the X axis and the Y axis are replaced;

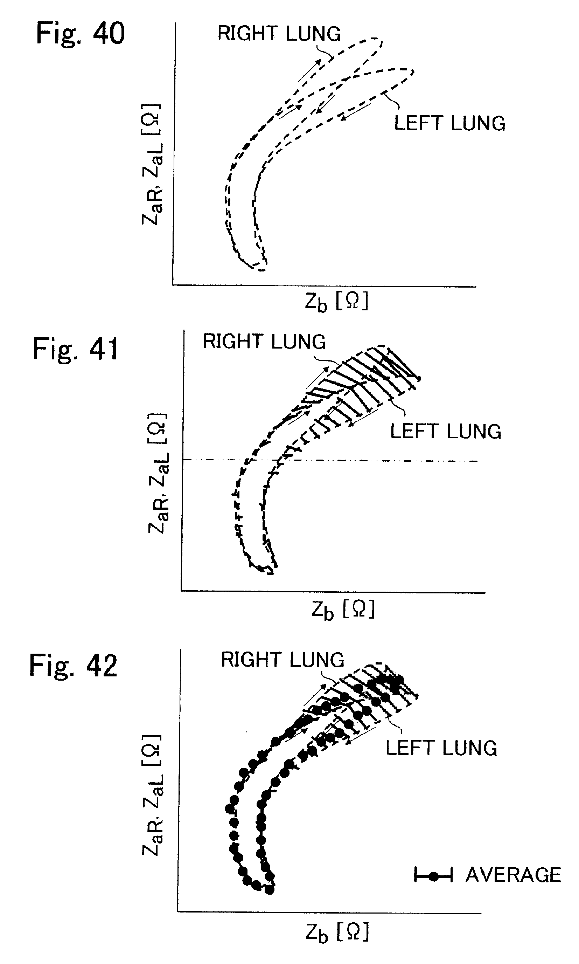

Fig. 40 is a diagram in which two Lissajous figures for the right lung and the left lung obtained through a single respiratory action are overlapped one on the other;

Fig. 41 is a diagram in which differences between two Lissajous figures for the right lung and the left lung are highlighted;

Fig. 42 is a diagram showing a Lissajous figure in which averages of samples for the right lung and the left lung are plotted;

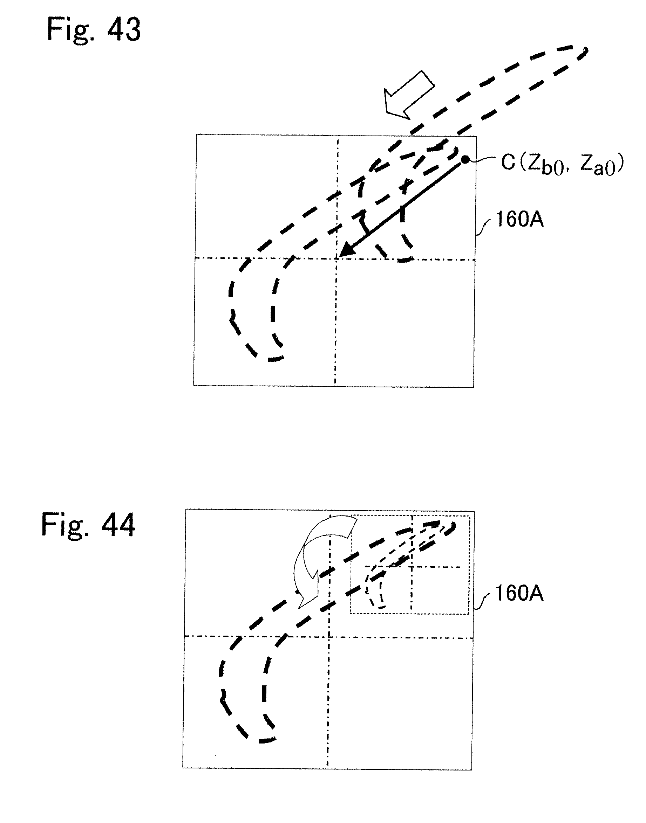

Fig. 43 is a diagram for explaining a scheme for centering the displayed location of a Lissajous figure;

Fig. 44 is a diagram for explaining another scheme for centering the displayed location of a Lissajous figure;

Fig. 45 is a diagram in which two Lissajous figures for draw-in respiration and costal respiration are shown;

Fig. 46 is a diagram showing a Lissajous figure in which the track for the latest respiration and the track for earlier respirations are shown in different depth of color;

Fig. 47 is a diagram for explaining an assistance display using Lissajous figures;

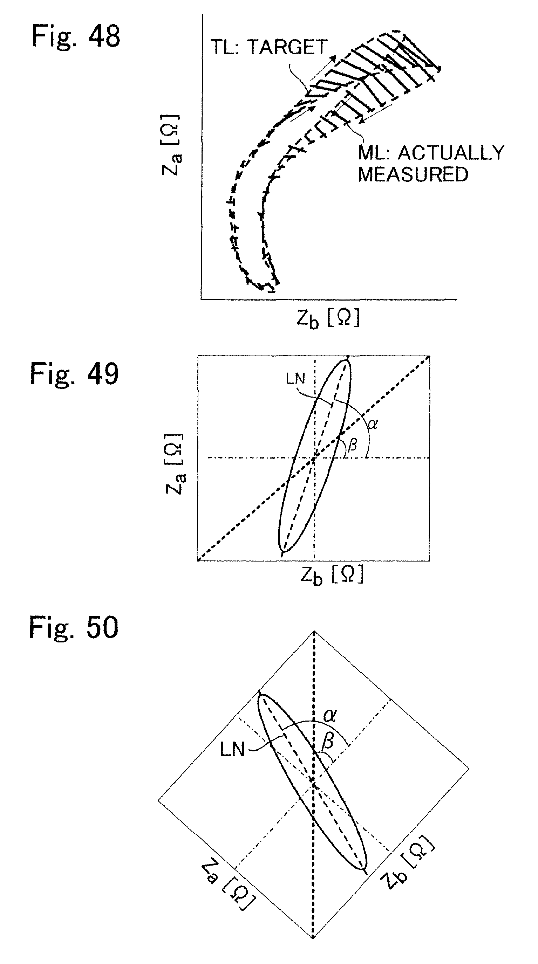

Fig. 48 is a diagram for explaining another assistance display using Lissajous figures;

Fig. 49 is a diagram for explaining a scheme for determining whether the respiration capability is good or bad;

Fig. 50 is a diagram for explaining another scheme for determining whether the respiration capability is good or bad;

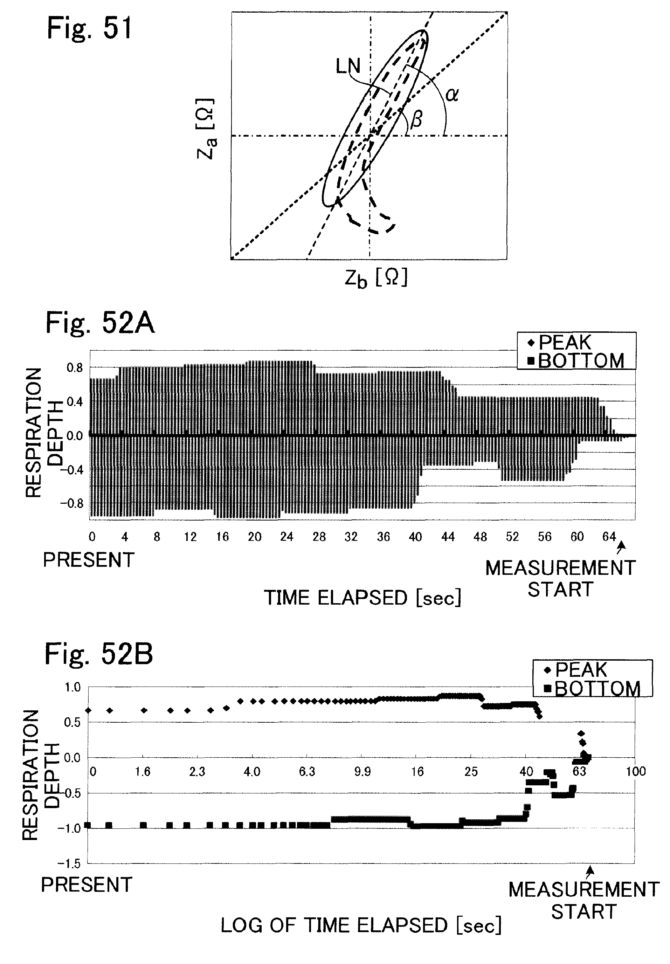

Fig. 51 is a diagram for explaining another scheme for determining whether the respiration capability is good or bad;

Fig. 52A is a graph showing an example of change over time of respiration depth;

Fig. 52B is a graph showing an example of change over time of respiration depth;

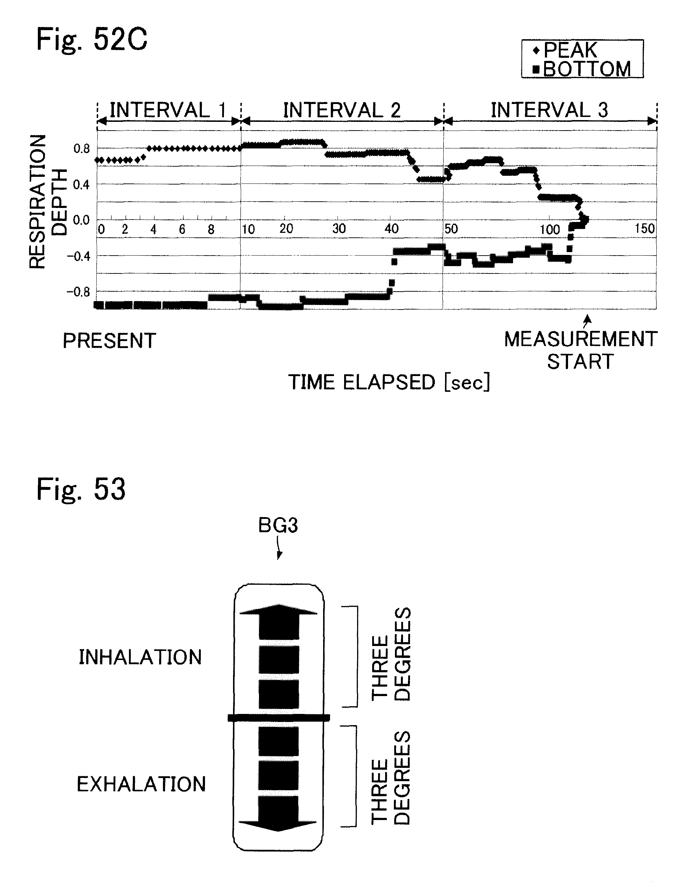

Fig. 52C is a graph showing an example of change over time of respiration depth;

Fig. 53 is a view showing an image of assistance information shown on a display device of the body condition determination apparatus;

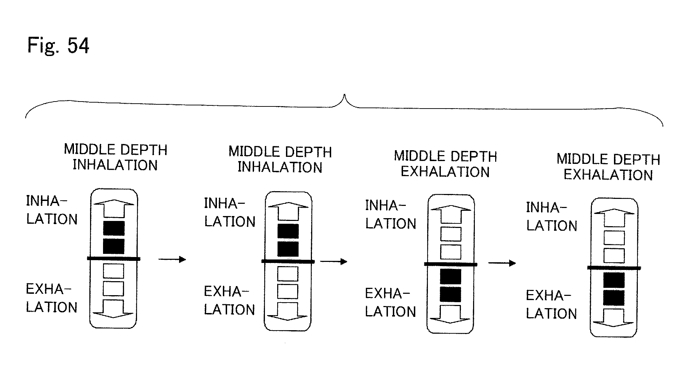

Fig. 54 is a view showing change over time of the assistance information shown on the display device;

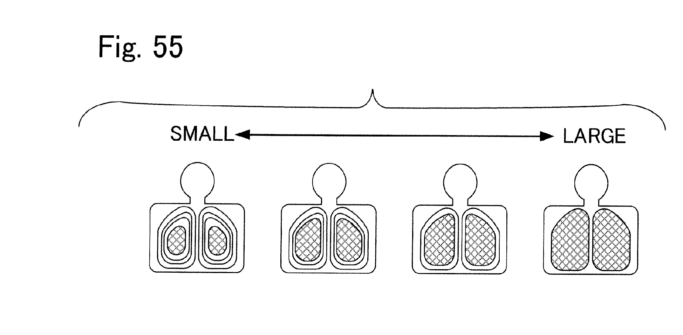

Fig. 55 is a view showing a displaying scheme for reporting a respiration magnitude;

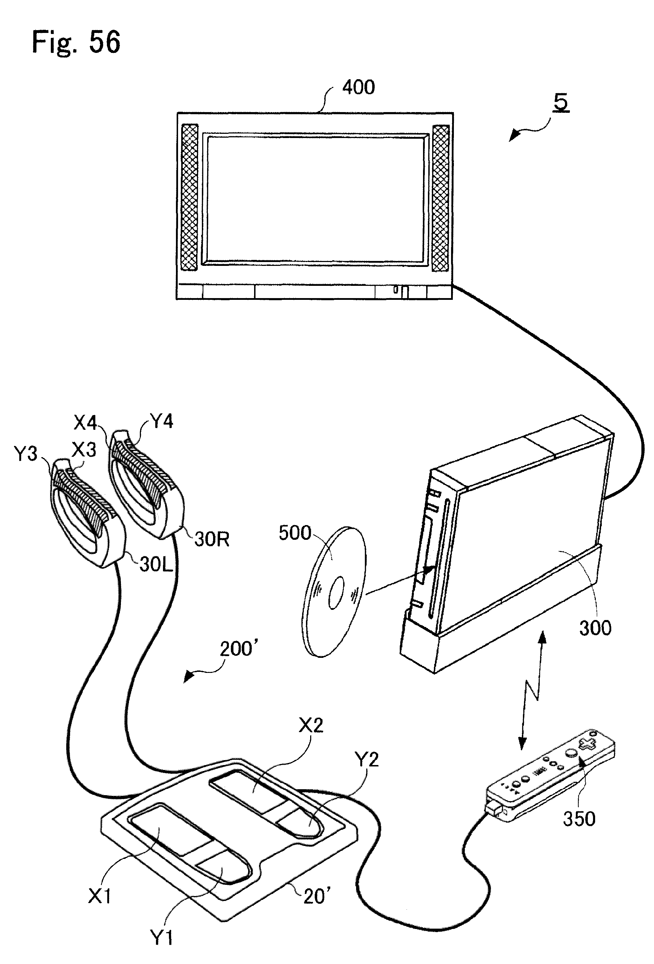

Fig. 56 is an overall view showing a body condition determination system of an embodiment according to the present invention having a household game machine;

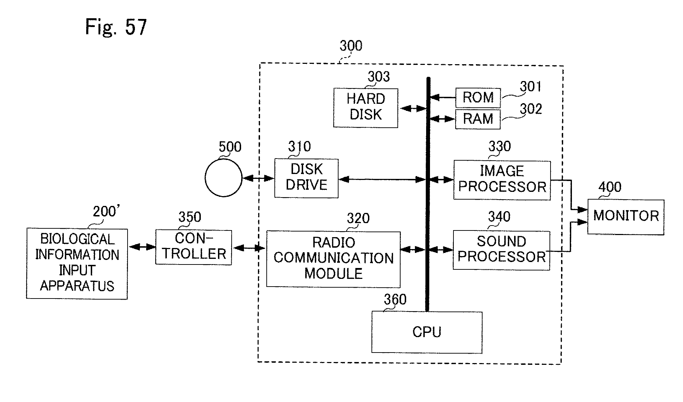

Fig. 57 is a block diagram showing a structure of the game machine;

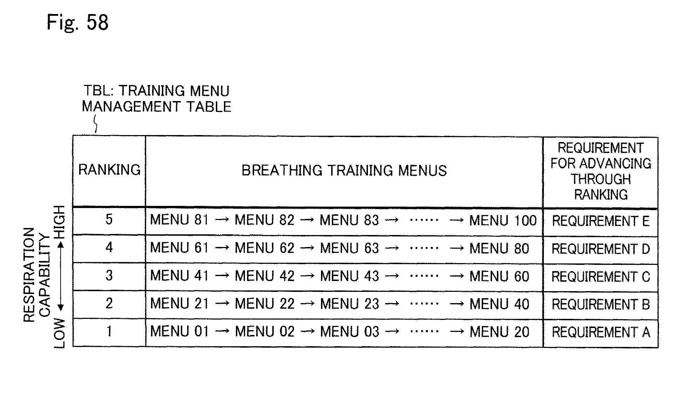

Fig. 58 is a diagram showing a data format of a training menu management table;

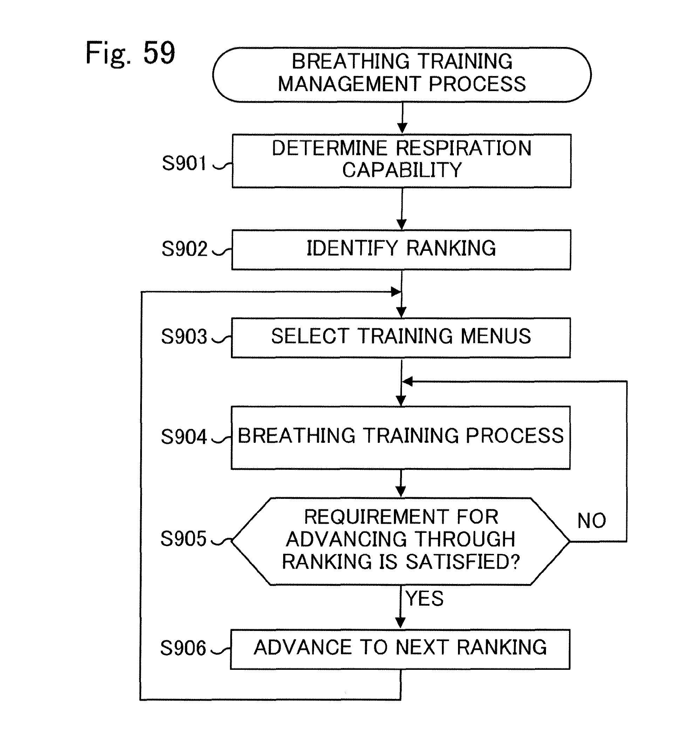

Fig. 59 is a flow chart showing an example of a breathing training management process executed in the body condition determination apparatus;

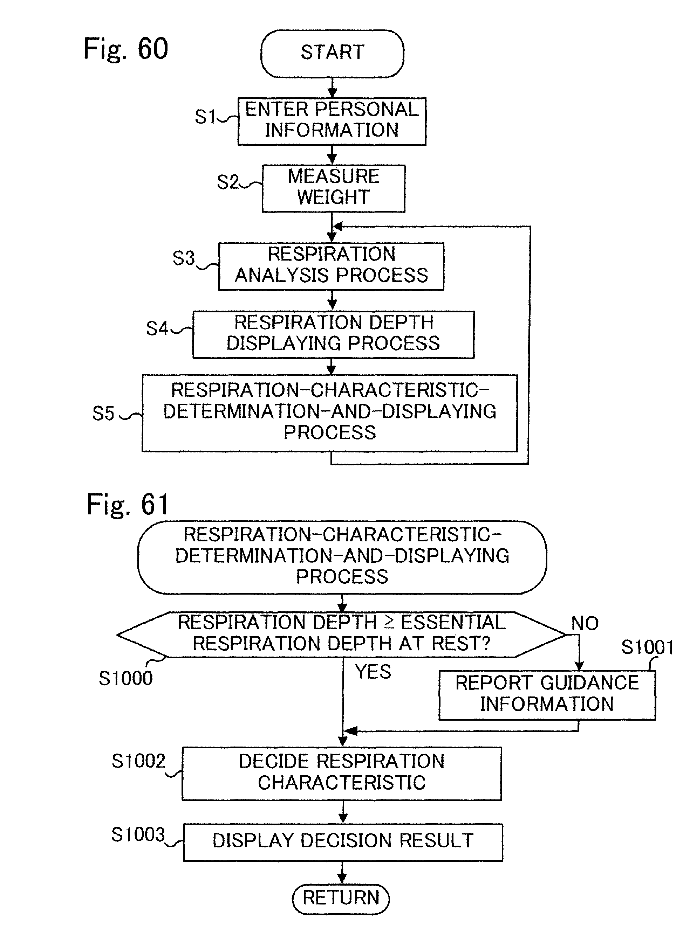

Fig. 60 is a flow chart showing an operation of a body condition determination apparatus of a fourth embodiment according to the present invention;

Fig. 61 is a flow chart showing a process for determining and displaying respiration characteristics;

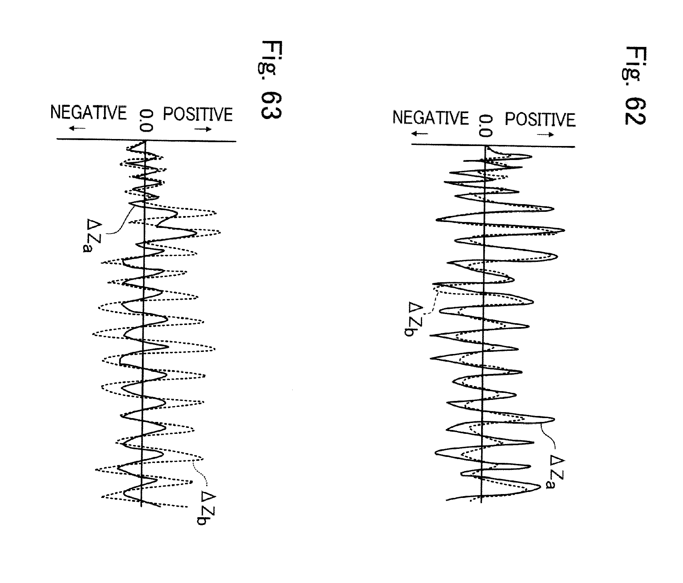

Fig. 62 is a graph showing change over time of each of a first difference and a second difference in respiration of a human subject who does not have a history of chest disease;

Fig. 63 is a graph showing change over time of each of a first difference and a second difference in respiration of a human subject who has a history of chest disease;

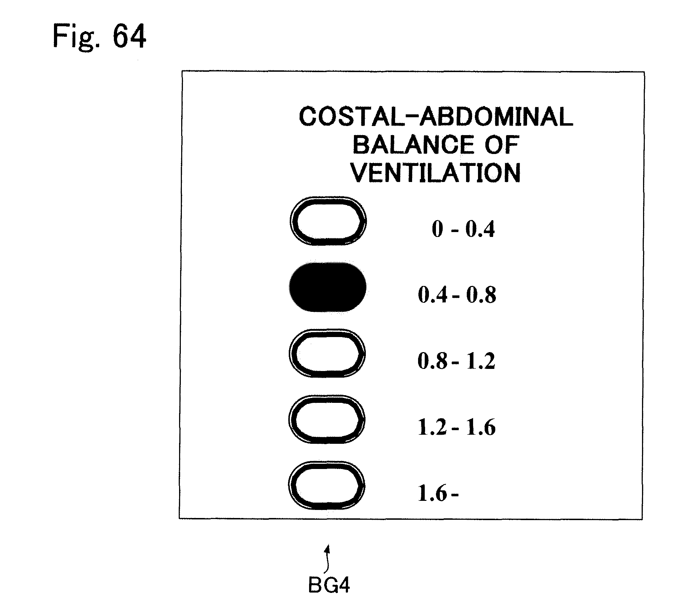

Fig. 64 is a view showing an image of balance of ventilation in the right lung and the left lung shown on a display device of the body condition determination apparatus;

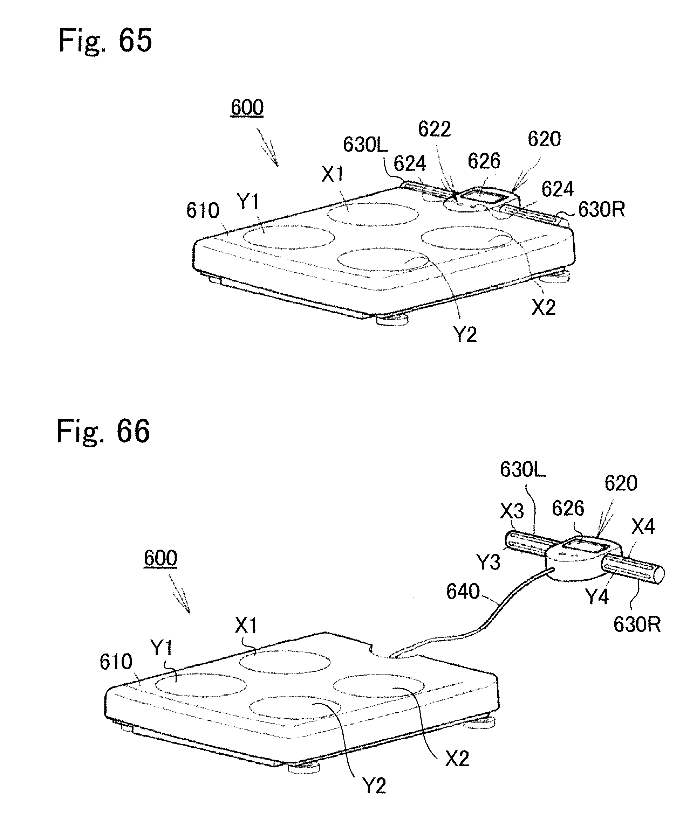

Fig. 65 is a perspective view showing a body condition determination apparatus of a variation; and

Fig. 66 is a perspective view showing the body condition determination apparatus of Fig. 65 in a different position.


DETAILED DESCRIPTION OF PREFERRED EMBODIMENTS

1. FIRST EMBODIMENT

1.1. STRUCTURE

Fig. 1 is a block diagram showing an electrical structure of a respiration characteristic analysis apparatus (body condition determination apparatus) 1 of an embodiment according to the present invention. The body condition determination apparatus 1 determines conditions of human subjects, and functions as a respiration characteristic determination apparatus that determines respiration characteristics of human subjects. More specifically, the body condition determination apparatus 1 functions as a respiration determination apparatus that determines the type of respiration of the human subject, and determines the degree of costal respiration and the degree of abdominal respiration.

The body condition determination apparatus 1 includes a management part 100 for measuring the body weights of human subjects and for managing overall operations of the body condition determination apparatus 1, and a bioelectrical impedance determination part 200 for determining bioelectrical impedances at various body regions of human subjects.

The management part 100 includes a weighing scale 110, a first memory 120, a second memory 130, a sound processor 140, a speaker 145, a human interface 150, and a display device 160. These elements are connected with a processor that is typically a CPU (Central Processing Unit) 170 via a bus. The CPU 170 serves as a main controller for controlling the entire apparatus. During operation of the CPU 170, the CPU 170 receives clock signals from a clock signal generation circuit (not shown). When a power switch (not shown) is turned on, a power source circuit supplies these elements with power.

The weighing scale 110 measures weights of human subjects and supplies weight data to the CPU 170 via the bus.

The first memory 120 is a nonvolatile memory, for example, a ROM (Read Only Memory). The first memory 120 stores a control program for controlling the entire apparatus. In accordance with the control program, the CPU 170 executes a predetermined arithmetic process.

The second memory 130 is a volatile memory, for example, a DRAM (Dynamic Random Access Memory). The second memory 130 serves as a work area for the CPU 170. During the execution of the predetermined arithmetic process by the CPU 170, the second memory 130 stores various data.

Under control by the CPU 170, the sound processor 140 conducts digital-to-analog conversion on sound data, amplifies the resulting sound signals to the speaker 145, and supplies the amplified sound signals to the speaker 145. The speaker 145 converts the amplified sound signals into sound vibrations and emits sound. Accordingly, the speaker 145 can provide human subjects with advisory or informative sounds, for example, guidance in manners of breathing.

The human interface 150 includes input devices. The human subject or another person may manipulate the input devices in order to input personal information on the human subject, for example, the height, age, and sex into the body condition determination apparatus 1.

The display device 160 shows the measurement results, such as the weight or the type of respiration. The display device 160 also shows instructions (of rhythm and pattern of exhalation and inhalation) to exhale and inhale in order to lead the human subject to perform abdominal breathing. The display device 160 shows messages for leading the human subject to input various information into the human interface 150. The display device 160 (reporter) may be, for example, a liquid crystal display device.

The bioelectrical impedance determination part 200 is used for determining bioelectrical impedances of the human subject (human body). The bioelectrical impedance determination part 200 includes an alternating current supplying circuit 210, a reference current measurement circuit 220, a potential difference measurement circuit 230, an A/D converter 240, and electrode switching circuits 251 and 252.

The alternating current supplying circuit 210 generates a reference current $I_{ref}$ having a frequency determined in the control program. The reference current measurement circuit 220 supplies the reference current $I_{ref}$ to the human subject, measures the actual value of the reference current $I_{ref}$ flowing through the human subject, and supplies electric current data $D_i$ indicative of the actual value of the reference current $I_{ref}$. The electrode switching circuit 252 selects, among four alternating current electrodes X 1 through X4, two or four electrodes through which the current flows. The current electrodes should be brought into contact with the human subject.

The potential difference measurement circuit 230 measures the potential difference between two voltage electrodes selected from among four voltage electrodes Y1 through Y4 by the other electrode switching circuit 251, and generates a potential difference signal $\Delta V$ indicative of the potential difference. The A/D converter 240 converts the analog potential difference signal $\Delta V$ into a digital signal, that is, voltage data $D_v$, and supplies the voltage data $D_v$ to the CPU 170. The CPU 170 calculates the bioelectrical impedance Z on the basis of the voltage data $D_v$ and the current data $D_i$ That is, the bioelectrical impedance Z is the potential difference divided by the current. Thus, the CPU 170 and the bioelectrical impedance determination part 200 serve as a bioelectrical impedance determiner for determining bioelectrical impedance at at least a body region of the human subject.

The first memory 120 may store various data in advance. For example, the first memory 120 may store correlation equations or tables for deriving the body fat percentage and the amount of muscle mass on the basis of bioelectrical impedances at various body regions.

The CPU 170 calculates the weight and bioelectrical impedances at various body regions (e.g., bioelectrical impedances at the upper limbs, bioelectrical impedances at the lower limbs, and the bioelectrical impedance at the body trunk), and controls various operations including signal inputting, signal outputting, measurements, and calculations. The CPU 170 can calculate the ratio of visceral fat to subcutaneous fat, the visceral fat amount, the subcutaneous fat ratio, the subcutaneous fat amount, the systemic body fat ratio, and body regional fat ratios (e.g., body fat ratios at the upper limbs, the lower limbs, and the body trunk).

Fig. 2 shows the appearance of the body condition determination apparatus 1. The body condition determination apparatus 1 has an L-shape including a platform 20 on which the human subject stands and a rectangular-columnar housing 30 extending upwardly from the platform 20. The platform 20 is provided with a left-foot current electrode X1 and a left-foot voltage electrode Y1 on which the left foot of the human subject will be placed, and a right-foot current electrode X2 and a right-foot voltage electrode Y2 on which the right foot of the human subject will be placed.

At the top of the housing 30, the aforementioned display device 160 is located. The display device 160 is a touch panel and serves as the human interface 150.

A left electrode handle 30L is located at the left side surface of the housing 30, whereas a right electrode handle 30R is located at the right side surface of the housing 30.

Fig. 3 is an enlarged view showing the upper part of the housing 30. As shown in Fig. 3, the left electrode handle 30L includes a left-hand current electrode X3 and a left-hand voltage electrode Y3, whereas the right electrode handle 30R includes a right-hand current electrode X4 and a right-hand voltage electrode Y4. For measurements of bioelectrical impedances, the human subject stands on the platform 20 and grips the electrode handles 30L and 30R with the left and right hands, respectively, with the arms extending downward.

Under control by the CPU 170, the electrode switching circuit 251 selects two voltage electrodes from among the four voltage electrodes Y1 through Y4, whereas the electrode switching circuit 252 selects two current electrodes from among the four current electrodes X through X4, so that impedances Z at various body regions can be determined.

More specifically, as shown in part (A) of Fig. 4, when the reference current $I_{ref}$ is supplied to flow between the left-foot current electrode X1 and the left-hand current electrode X3 and when the potential difference between the left-foot voltage electrode Y1 and the left-hand voltage electrode Y3 is measured, the systemic body bioelectrical impedance can be determined. Although not illustrated, when the reference current $I_{ref}$ is is supplied to flow between the right-foot current electrode X2 and the right-hand current electrode X4 and when the potential difference between the right-foot voltage electrode Y2 and the right-hand voltage electrode Y4 is measured, the systemic body bioelectrical impedance can also be determined.

As shown in part (K) of Fig. 4, when the current is supplied to all of the four current electrodes so that a current flows between both hands and a current flows between both feet, and when the potential difference between a hand and a foot is measured, the systemic body bioelectrical impedance can also be determined.

As shown in part (B) of Fig. 4, when the reference current $I_{ref}$ is supplied to flow between the right-foot current electrode X2 and the right-hand current electrode X4 and when the potential difference between the right-foot voltage electrode Y2 and the left-foot voltage electrode Y1 is measured, the bioelectrical impedance at the right lower extremity can be determined.

As shown in part (C) of Fig. 4, when the reference current $I_{ref}$ is supplied to flow between the left-foot current electrode X1 and the left-hand current electrode X3 and when the potential difference between the left-foot voltage electrode Y1and the right-foot voltage electrode Y2 is measured, the bioelectrical impedance at the left lower extremity can be determined.

As shown in part (D) of Fig. 4, when the reference current $I_{ref}$ is supplied to flow between the right-hand current electrode X4 and the right-foot current electrode X2 and when the potential difference between the right-hand voltage electrode Y4 and the left-hand voltage electrode Y3 is measured, the bioelectrical impedance at the right upper extremity and the upper body trunk can be determined. Although not illustrated, when the reference current $I_{ref}$ is supplied to flow between the left-hand current electrode X3 and the right-hand current electrode X4 and when the potential difference between the right-hand voltage electrode Y4 and the right-foot voltage electrode Y2 is measured, the bioelectrical impedance at the right upper extremity and the upper body trunk can also be determined.

As shown in part (E) of Fig. 4, when the reference current $I_{ref}$ is supplied to flow between the left-hand current electrode X3 and the left-foot current electrode X1 and when the potential difference between the right-hand voltage electrode Y4 and the left-hand voltage electrode Y3 is measured, the bioelectrical impedance at the left upper extremity and the upper body trunk can be determined. Although not illustrated, when the reference current $I_{ref}$ is supplied to flow between the left-hand current electrode X3 and the right-hand current electrode X4 and when the potential difference between the left-hand voltage electrode Y3 and the left-foot voltage electrodes Y1 is measured, the bioelectrical impedance at the left upper extremity and the upper body trunk can also be determined.

As shown in part (F) of Fig. 4, when the reference current $I_{ref}$ is supplied to flow between the right-hand current electrode X4 and the left-hand current electrode X3 and when the potential difference between the right-hand voltage electrode Y4 and the left-hand voltage electrode Y3 is measured, the bioelectrical impedance at both upper extremities and the upper body trunk can be determined.

As will be understood from each of parts (D), (E), and (F), the determined bioelectrical impedance is influenced by the bioelectrical impedance at the upper body trunk. If right and left upper extremities do not move during the measurement of bioelectrical impedance, the bioelectrical impedances thereat do not change. Accordingly, change over time of the bioelectrical impedance at the right upper extremity and the upper body trunk can be considered as change over time of the bioelectrical impedance at the upper body trunk. Similarly, change over time of the bioelectrical impedance at the left upper extremity and the upper body trunk can be considered as change over time of the bioelectrical impedance at the upper body trunk. Change over time of the bioelectrical impedance at both upper extremities and the upper body trunk can be considered as change over time of the bioelectrical impedance at the upper body trunk.

As shown in part (G) of Fig. 4, when the reference current $I_{ref}$ is supplied to flow between the left-hand current electrode X3 and the left-foot current electrode X1 and when the potential difference between the right-hand voltage electrode Y4 and the right-foot voltage electrode Y2 is measured, the bioelectrical impedance at the middle body trunk can be determined.

As shown in part (H) of Fig. 4, when the reference current $I_{ref}$ is supplied to flow between the right-hand current electrode X4 and the right-foot current electrode X2 and when the potential difference between the left-hand voltage electrode Y3 and the left-foot voltage electrode Y1 is measured, the bioelectrical impedance at the middle body trunk can be determined.

As shown in part (I) of Fig. 4, when the reference current $I_{ref}$ is supplied to flow between the right-hand current electrode X4 and the left-foot current electrode X1 and when the potential difference between the left-hand voltage electrode Y3 and the right-foot voltage electrode Y2 is measured, the bioelectrical impedance at the middle body trunk can be determined.

As shown in part (J) of Fig. 4, when the reference current $I_{ref}$ is supplied to flow between the right-foot current electrode X2 and the left-hand current electrode X3 and when the potential difference between the right-hand voltage electrode Y4 and the left-foot voltage electrode Y1 is measured, the bioelectrical impedance at the middle body trunk can be determined.

The manner for determining the bioelectrical impedance at the upper or middle body trunk is not limited to the above-described manner. For example, bioelectrical impedances at various regions, e.g., upper and lower extremities, and the systemic body are determined by suitably selecting the current electrodes through which the reference current $I_{ref}$ is supplied and by suitably selecting the voltage electrodes for measuring the potential difference. Then, the bioelectrical impedance at the upper or middle body trunk can be obtained by addition or subtraction of the bioelectrical impedances.

In addition, if electrodes are brought into contact with the ear-lobes instead of extremities, the bioelectrical impedance at the body trunk can be obtained. Alternatively, if electrodes are brought into contact with the body trunk directly, the bioelectrical impedance at the upper or middle body trunk can be obtained.

## 1.2. OPERATION

Fig. 5 is a flow chart showing an operation of the body condition determination apparatus 1. Once the power switch (not shown) on the human interface 150 is turned on, the power source circuit (not shown) provides power to electrical elements. Then, the CPU 170 causes the display device 160 to show a screen for facilitating the user to enter personal or individual body information (including the height, age, and sex) at step S1.

After the human interface 150 inputs the personal information into the CPU 170, the CPU 170 causes the weighing scale 110 to measure the weight, and obtains the weight from the weighing scale 110 at step S2.

The CPU 170 causes the bioelectrical impedance determination part 200 to measure the voltages and the currents, each of which is influenced by bioelectrical impedances at desired regions (for example, the extremities and the body trunk), and determines the impedances at the desired body region. Thus, the CPU 170 and the bioelectrical impedance determination part 200 serve as a bioelectrical impedance determiner for determining bioelectrical impedance at the desired body regions. The CPU 170 executes a respiration analysis process at step S3. As will be described later in detail, in the respiration analysis process, the CPU 170 serves as an analyzer that obtains indicative information that is used for identifying whether respiration of the human subject is abdominal or costal.

After step S3, the CPU 170 executes a respiration depth displaying process (step S4). As will be described later in detail, in the respiration depth displaying process, the CPU 170 causes the display device 160 to show the magnitude of each of abdominal respiration and costal respiration at every respiration and to show the margin level beyond the essential respiration depth for each of abdominal respiration and costal respiration at every respiration. Although not shown, the operation may be ended by the user's instruction.

## 1.3. PRINCIPLES OF RESPIRATION ANALYSIS

The principles of respiration analysis will be described. Fig. 6 is a schematic view showing tissues in the body trunk of humans. As shown in Fig. 6, tissues in the body trunk are separated by the diaphragm into the upper part and the lower part. The upper part includes the lungs and the chest skeletal muscle including the internal and external intercostal muscles. On the other hand, the lower part includes the visceral tissue and the abdominal skeletal muscle including the internal and external abdominal oblique muscles, the transverse abdominal muscle, and the abdominal rectus muscle.

In both abdominal respiration and costal respiration, the diaphragm moves up to compress the lungs at exhalation and moves down to expand the lungs at inhalation. The characteristic of abdominal respiration that does not appear in costal respiration is that the visceral tissue expands and contracts in the perpendicular direction so as to move the diaphragm up and down due to ventrodorsal contraction and expansion of the abdominal respiratory muscles including the abdominal rectus muscle, the internal and external abdominal oblique muscles, and the transverse abdominal muscle.

With reference to the equivalent circuit models in Figs. 7A and 7B, factors influencing the first bioelectrical impedance $Z_a$ at the upper body trunk and the second bioelectrical impedance $Z_b$ at the middle body trunk will be described. Let us assume that the bioelectrical impedance at the chest skeletal muscle is $Z_1$ and $Z_4$, that at the lungs is $Z_2$ and $Z_5$, that at the upper extremity skeletal muscle is $Z_3$, that at the abdominal skeletal muscle is $Z_6$, and that at the visceral tissue is $Z_7$. As will be understood from Fig. 7B, the first bioelectrical impedance $Z_a$ at the upper body trunk is influenced by impedances $Z_1$ and $Z_2$ in parallel with each other and by impedance $Z_3$. The synthetic impedance of $Z_1$ and $Z_2$ corresponds to the bioelectrical impedance at the upper lobes of the lungs. Accordingly, the first bioelectrical impedance $Z_a$ is the bioelectrical impedance at the upper body trunk including the upper lobes of the lungs of the human subject and excluding the abdomen of the human subject.

As will be understood from Fig. 7A, the second bioelectrical impedance $Z_b$ at the middle body trunk is influenced by impedances $Z_4$ and $Z_5$ in parallel with each other and by impedances $Z_6$ and $Z_7$ in parallel with each other. The synthetic impedance of $Z_4$ and $Z_5$ corresponds to the bioelectrical impedance at the median and lower lobes of the lungs. The bioelectrical impedance $Z_7$ at the visceral tissue includes the bioelectrical impedance of the diaphragm. Accordingly, the second bioelectrical impedance $Z_b$ is the bioelectrical impedance at the middle body trunk including the median and lower lobes of lungs and the abdomen of the human subject.

With reference to Fig. 8, the relationship between respiration and change of bioelectrical impedance will be described.

Change in the first bioelectrical impedance $Z_a$ at the upper body trunk in respiration is mainly caused by air entering and leaving the lungs, which has a high electrical insulation property and causes change in the electrical characteristics (i.e., the electrical conductivity or the inverse of the volume resistivity) at the upper body trunk. During exhalations (expirations), the bioelectrical impedance $Z_2$ at the lungs decreases due to reduction in the volume of air inside tissues in the lungs ($\Delta Z_{lu} < 0$). During inhalations (inspirations), the bioelectrical impedance $Z_2$ at the lungs increases due to increase in the volume of air inside tissues in the lungs ($\Delta Z_{lu} > 0$).

In costal respiration, which expands and contracts the thoracic cage, the chest skeletal muscle that contributes to respiration (e.g., internal and external intercostal muscles) expands and contracts in a similar way to the expansion and contraction of the lungs. Therefore, in costal respiration, when the lungs expand so that the bioelectrical impedance $Z_2$ at the lungs increases, the chest skeletal muscle also expands, and the bioelectrical impedance $Z_1$ at the chest skeletal muscle also increases. Similarly, when the lungs contract so that the bioelectrical impedance $Z_2$ at the lungs decreases, the chest skeletal muscle also contracts and the bioelectrical impedance $Z_1$ at the chest skeletal muscle also decreases.

However, in abdominal respiration in which the thoracic cage does not change in volume significantly, although the bioelectrical impedance $Z_2$ at the lungs changes significantly due to respiration, the bioelectrical impedance $Z_1$ at the chest skeletal muscle does not change significantly. The first bioelectrical impedance $Z_a$ includes the bioelectrical impedance $Z_3$ at the upper extremity skeletal muscle, but the upper extremity skeletal muscle does not directly contribute to respiration. In this embodiment, the human subject stands on the platform 20 shown in Fig. 2 and grips the electrode handles 30L and 30R with the left and right hands, respectively, with the arms extending downward, so that the upper extremity skeletal muscle does not move during the measurement of bioelectrical impedance, and the bioelectrical impedance $Z_3$ thereat does not change significantly. As shown in Figs. 9 and 10, in both of costal respiration and abdominal respiration, the first bioelectrical impedance $Z_a$ increases at inhalations and decreases at exhalations.

Change in the second bioelectrical impedance $Z_b$ at the middle body trunk in respiration relates to movement of the diaphragm. As described above, in both abdominal respiration and costal respiration, the diaphragm moves up to compress the lungs at exhalation and moves down to expand the lungs at inhalation. The characteristic of abdominal respiration is that the visceral tissue expands and contracts in the perpendicular direction for moving the diaphragm up and down due to ventrodorsal contraction and expansion of the abdominal respiratory muscles (abdominal skeletal muscles).

More specifically, in abdominal respiration, during exhalations, the human subject contracts the abdominal muscle ventrodorsally so as to move up with the diaphragm together. As a result, the visceral tissue and the abdominal skeletal muscle expand in the perpendicular direction, thereby increasing the impedance $Z_6$ at the abdominal skeletal muscle and the impedance $Z_7$ at the visceral tissue ($\Delta Z_{st} > 0$). During exhalations, the bioelectrical impedance at the lungs decreases due to reduction in the volume of air inside tissues in the lungs as described above ($\Delta Z_{lu} < 0$). Therefore, in abdominal respiration, when the bioelectrical impedance at the chest region above the diaphragm decreases, the bioelectrical impedance at the abdominal region beneath the diaphragm increases. In other words, increase in the bioelectrical impedance of the abdominal region beneath the diaphragm cancels decrease in the bioelectrical impedance at the chest region above the diaphragm when the human subject performs exhalation in abdominal respiration. As will be understood from the above description, the movement of the abdominal region (including abdominal skeletal muscle and the visceral tissue) beneath the diaphragm varies depending on the type of respiration (costal and abdominal respirations).

As shown in Fig. 9, when respiration of the human subject is abdominal respiration, the second bioelectrical impedance $Z_b$ increases at inhalations, and also increases at exhalations since the bioelectrical impedance of the abdominal region beneath the diaphragm cancels decrease in the bioelectrical impedance at the chest region above the diaphragm. As shown in Fig. 10, when respiration of the human subject is costal respiration, the second bioelectrical impedance $Z_b$ increases at inhalations and decreases at exhalation, in a manner similar to the above-described change of the first bioelectrical impedance $Z_a$.

Next, with reference to Figs. 11 and 12, relationships among respiration of the human subject, the costal circumference $R_{ib}$ of human subjects, and abdominal circumference $A_b$ of human subjects will be described. Let us assume that respiration of the human subject is abdominal respiration. Fig. 11 is a graph showing change over time of each of costal and abdominal circumferences in abdominal respiration of a human subject measured by Respitrace (Trademark, AMI Inc, Ardsley, New York, U.S.A.). As will be understood from Fig. 11, when respiration of the human subject is abdominal respiration, the abdominal circumference $A_b$ significantly changes due to respirations, whereas the costal circumference $R_{ib}$ does not significantly change. Let us define that variation in the costal circumference $R_{ib}$ of a human subject is $\Delta R_{ib}$. More specifically, the difference between the measurement value of $R_{ib}$ and the standard level, i.e., standard value of measurement values of $R_{ib}$ is $\Delta R_{ib}$. Let us define that variation in the abdominal circumference $A_b$ of the human subject is $\Delta A_b$. More specifically, the difference between the measurement value of $A_b$ and the standard level, i.e., standard value of measurement values of $A_b$ is $\Delta A_b$. In abdominal respiration, the ratio $AR_{ib}/\Delta A_b$ is equal to or less than one. Respitrace outputs the absolute value (zero-to-peak value) of the peak value or the bottom value of differences between the measurement values and the standard value, or outputs the sum (peak-to-peak value) of the absolute values of the peak value and the bottom value.

Let us assume that respiration of the human subject is costal respiration.

Fig. 12 is a graph showing change over time of each of costal and abdominal circumferences in costal respiration of the human subject measured by Respitrace. When respiration of the human subject is costal respiration, variation in the costal circumference $R_{ib}$ due to respirations is significantly large and greater than variation in the abdominal circumference $A_b$. Therefore, the above-described ratio $\Delta R_{ib}/\Delta A_b$ is greater than one. Thus, the ratio $\Delta R_{ib}/\Delta A_b$ can be considered as indicative information that is used for identifying whether respiration of the human subject is abdominal or costal.

The present inventor found that there was a close correlative relationship among the ratio $AR_{ib}/\Delta A_b$ of the variation in the costal circumference $\Delta R_{ib}$ and the variation in the abdominal circumference $\Delta A_b$, a first difference $\Delta Z_a$, and a second difference $\Delta Z_b$. The first difference $\Delta Z_a$ is a difference between an instantaneous measurement value of the first bioelectrical impedance $Z_a$ and a first centering value $Z_{a0}$ of measurement values of the first bioelectrical impedance $Z_a$, whereas the second difference $\Delta Z_b$ is a difference between an instantaneous measurement value of the second bioelectrical impedance $Z_b$ and a second centering value $Z_{b0}$ of measurement values of the second bioelectrical impedance $Z_b$. Using a formula expressing the correlative relationship, the ratio $\Delta R_{ib}/\Delta A_b$ can be calculated from the first difference $\Delta Z_a$ and the second difference $\Delta Z_b$. In addition, from the calculated ratio $\Delta R_{ib}/\Delta A_b$, it is possible to determine the type of respiration of the human subject (costal respiration or abdominal respiration). As will be described later in detail, the first centering value $Z_{a0}$ is a standard level of change over time in the first bioelectrical impedance $Z_a$ used for extracting information on respiration of the human subject, and is the average of the first bioelectrical impedances within a unit time. As will be described later in detail, the second centering value $Z_{b0}$ is a standard level of change over time in the second bioelectrical impedance $Z_b$ used for extracting information on respiration of the human subject, and is the average of the second bioelectrical impedances within a unit time.

Fig. 13 is a graph showing relationships between $\Delta R_{ib}/\Delta A_b$ and $\Delta Z_b/\Delta Z_a$ obtained by measurement data on a plurality of human subjects. As will be understood from Fig. 13, there is a close correlative relationship between the ratio $\Delta R_{ib}/\Delta A_b$ and the ratio $\Delta Z_b/\Delta Z_a$, in which the coefficient of correlation R = 0.651, and the probability P < 0.01. Regression formula (1) below is derived from the correlative relationship.

$$\Delta R_{ib}/\Delta A_b = a_0 * \Delta Z_b/\Delta Z_a + b_0 \quad ... (1)$$

where $a_0$ is a coefficient of regression, and $b_0$ is a constant. According to the inventor's analysis, $a_o$ is 2.7882, whereas $b_0$ is 0.2015.

Regression formula (1) can be rewritten as follows:

$$\Delta R_{ib}/\Delta A_b = (a_0 * \Delta Z_b - \Delta Z_a)/\Delta Z_a + b_1 \quad ... (2)$$

where $b_1$ is a constant and is equal to $a_0$ plus $b_0$.

Change in the first bioelectrical impedance $Z_a$ at the upper body trunk in respiration can be considered as change in the bioelectrical impedance at the upper lobes of lungs (the synthetic impedance of $Z_1$ and $Z_2$ in parallel with each other). Change in the second bioelectrical impedance $Z_b$ at the middle body trunk in respiration can be considered as the sum of change in the bioelectrical impedance at the median and lower lobes of the lungs (the synthetic impedance of $Z_4$ and $Z_5$ in parallel with each other) and change in the abdominal bioelectrical impedance (the synthetic impedance of $Z_6$ and $Z_7$ in parallel with each other).

Change in the bioelectrical impedance at the upper lobes of the lungs can be equivalent to change in the bioelectrical impedance at the median and lower lobes of the lungs since the locations are near at the chest. Thus, the difference between change in the second bioelectrical impedance $Z_b$ and change in the first bioelectrical impedance $Z_a$ is equivalent to change in the abdominal bioelectrical impedance. Accordingly, formula (2) can be considered to be a formula expressing a relationship between the ratio of the abdominal bioelectrical impedance to change in the costal bioelectrical impedance and the ratio $\Delta R_{ib}/\Delta A_b$. Constant $a_0$ in formula (2) can be a compensation coefficient for compensating the difference between sensitivities for measurements on the upper lobes of the lungs and the median and lower lobes of the lungs.

## 1.4. RESPIRATION ANALYSIS PROCESS

Next, a respiration analysis process executed by the CPU 170 will be described. Fig. 14 is a flow chart showing an example of the respiration analysis process. In this embodiment, the CPU 170 executes the respiration analysis process ten times within a single respiration, i.e., single respiratory action (consisting of a single inhalation and a single exhalation) at a normal speed. Since a normal single respiration takes four seconds, the CPU 170 executes the respiration analysis process every 0.4 seconds. In the following, the start time for each respiration analysis process that occurs every 0.4 seconds will be referred to as a sampling time. However, this is only an example, and the cycle of the respiration analysis process may be decided in a different manner.

As shown in Fig. 14, the CPU 170 first decides whether or not the current time reaches a sampling time (step S 10), and the process proceeds to step S20 if the decision at step S10 is affirmative. Description of subsequent steps will be continued on the assumption that the current time reaches the n-th sampling time where n is a natural number that is equal to or greater than one. Prior to detailed description of subsequent steps, brief description will be made for each of the subsequent steps.

At step S20, the CPU 170 (bioelectrical impedance determiner) determines the first bioelectrical impedance $Z_a$ at the upper body trunk. Next, at step S30, the CPU 170 (bioelectrical impedance determiner) determines the second bioelectrical impedance $Z_b$ at the middle body trunk. Next, at step S40, the CPU 170 executes a smoothing process for each of the first bioelectrical impedance $Z_a$ determined at step S20 and the second bioelectrical impedance $Z_b$ determined at step S30. Next, at step S50, the CPU 170 (centering value generator) generates the first centering value $Z_{a0}$ that is a standard level of change over time in the first bioelectrical impedance $Z_a$. Next, at step S60, the CPU 170 (first difference calculator) calculates the first difference $\Delta Z_a$ that is the difference between the instantaneous measurement value of the first bioelectrical impedance $Z_a$ and the first centering value $Z_{a0}$. Next, at step S70, the CPU 170 (centering value generator) generates the second centering value $Z_{b0}$ that is a standard level of change over time in the second bioelectrical impedance $Z_b$, and the CPU 170 (second difference calculator) calculates the second difference $\Delta Z_b$ that is the difference between the instantaneous measurement value of the second bioelectrical impedance $Z_b$ and the second centering value $Z_{b0}$. Next, at step S80, the CPU 170 (analyzer) executes an arithmetic process in accordance with regression formula (2) described above so as to calculate the ratio $\Delta R_{ib}/\Delta A_b$ corresponding to the first difference $\Delta Z_a$ and the second difference $\Delta Z_b$. Next, at step S90, the CPU 170 executes a respiration depth calculating process for calculating the depth of respiration of the human subject. In the following, those steps will be described in detail.

As shown in Fig. 14, at step S20, the CPU 170 determines the first bioelectrical impedance $Z_a$ at the upper body trunk. For example, the CPU 170 controls the electrode switching circuit 252 to select the left-hand current electrode X3 and the right-hand current electrode X4, and controls the electrode switching circuit 251 to select the right-foot voltage electrode Y2 and the right-hand voltage electrode Y4. Then, the CPU 170 determines the first bioelectrical impedance $Z_a$ at the right upper extremity and the upper body trunk on the basis of the current data $D_i$ indicating the reference current $I_{ref}$ flowing between the right and left hands and the voltage data $D_v$ indicating the potential difference between the right-foot voltage electrode Y2 and the right-hand voltage electrode Y4. Hereinafter, the actual measured value of the first bioelectrical impedance at the n-th sampling time (n is a natural number that is equal to or greater than one) will be referred to as $Z_a(n)'$.

Next, the CPU 170 determines the second bioelectrical impedance $Z_b$ at the middle body trunk (step S30). For example, the CPU 170 controls the electrode switching circuit 252 to select the left-foot current electrode X1 and the right-hand current electrode X4, and controls the electrode switching circuit 251 to select the left-hand voltage electrode Y3 and the right-foot voltage electrode Y2. Then, the CPU 170 determines the second bioelectrical impedance $Z_b$ at the middle body trunk on the basis of the current data $D_i$ indicating the reference current $I_{ref}$ flowing between the left foot and the right hand and the voltage data $D_v$ indicating the potential difference between the left-hand voltage electrode Y3 and the right-foot voltage electrode Y2. Hereinafter, the actual measured value of the second bioelectrical impedance at the n-th sampling time (n is a natural number that is equal to or greater than one) will be referred to as $Z_b(n)'$.

Next, at step S40, the CPU 170 executes a smoothing process for each of the first bioelectrical impedance $Z_a(n)'$ determined at step S20 and the second bioelectrical impedance $Z_b(n)'$ determined at step S30. First, the smoothing process for the first bioelectrical impedance $Z_a(n)'$ will be described in detail. The CPU 170 calculates the moving average of the actual measured value $Z_a(n-2)'$ of the first bioelectrical impedance at the n-2th sampling time, the actual measured value $Z_a(n-1)'$ of the first bioelectrical impedance at the n-1th sampling time, and the actual measured value $Z_a(n)'$ of the first bioelectrical impedance at the n-th sampling time. Then, the CPU 170 decides the calculated moving average as the measurement value of the first bioelectrical impedance at the n-th sampling time (this is called the smoothing process). The measurement value of the first bioelectrical impedance resulting from the smoothing process will be referred to as $Z_a(n)$.

Next, the smoothing process for the second bioelectrical impedance $Z_b(n)'$ will be described in detail. The CPU 170 calculates the moving average of the actual measured value $Z_b(n-2)'$ of the second bioelectrical impedance at the n-2th sampling time, the actual measured value $Z_b(n-1)'$ of the second bioelectrical impedance at the n-1th sampling time, and the actual measured value $Z_b(n)'$ of the second bioelectrical impedance at the n-th sampling time. Then, the CPU 170 decides the calculated moving average as the measurement value of the second bioelectrical impedance at the n-th sampling time (this is called the smoothing process). The measurement value of the second bioelectrical impedance resulting from the smoothing process will be referred to as $Z_b(n)$.

Next, the CPU 170 executes a first centering process for generating a first centering value $Z_{a0}$ that is a standard level of change over time in the first bioelectrical impedance $Z_a$ (step S50). Hereinafter, the first centering value at the n-th sampling time will be referred to as $Z_{a0}(n)$. In this embodiment, the CPU 170 generates or calculates the first centering value $Z_{a0}(n)$ on the basis of the measurement values of the first bioelectrical impedance $Z_a$ at multiple sampling times within a centering period starting from a time point that is a predetermined time length before the n-th sampling time and ending at the n-th sampling time. The time length of the centering period is variable and is set depending on the respiration speed of the human subject at the n-th sampling time. The first centering process will be described in detail.

Fig. 15 is a flow chart showing an example of the first centering process. As shown in Fig. 15, the CPU 170 first executes a MA10 calculating process for calculating the moving average (that is, MA10) of the measurement values of the first bioelectrical impedance $Z_a$ at the last ten sampling times (step S51). More specifically, the CPU 170 calculates the moving average of the measurement values ($Z_a(n-9)$ through $Z_a(n)$) of the first bioelectrical impedance $Z_a$ at the n-9th through n-th (ten) sampling times. The resulting moving average is referred to as the moving average MA10(n) at the n-th sampling time. The calculation is expressed as:

$$[Z_a(n-9) + Z_a(n-8) + ... + Z_a(n)]/10 = MA10(n).$$

Next, the CPU 170 executes a MA20 calculating process for calculating the moving average (that is, MA20) of the measurement values of the first bioelectrical impedance $Z_a$ at the last 20 sampling times (step S52). More specifically, the CPU 170 calculates the moving average of the measurement values ($Z_a(n-19)$ through $Z_a(n)$) of the first bioelectrical impedance $Z_a$ at the n-19th through n-th (twenty) sampling times. The resulting moving average is referred to as the moving average MA20(n) at the n-th sampling time. The calculation is expressed as:

$$[Z_a(n-19) + Z_a(n-18) + ... + Z_a(n)]/20 = MA20(n).$$

Next, the CPU 170 executes a MAX10 extraction process for extracting the maximum (referred to as MAX10) among the measurement values of the first bioelectrical impedance $Z_a$ at the last ten sampling times (step S53). More specifically, the CPU 170 selects the maximum among the measurement values of the first bioelectrical impedance $Z_a$ at the n-9th through n-th (ten) sampling times, and determines it to be the maximum MAX10(n) at the n-th sampling time.

Next, the CPU 170 executes a MIN10 extraction process for extracting the minimum (referred to as MIN10) among the measurement values of the first bioelectrical impedance $Z_a$ at the last ten sampling times (step S53). More specifically, the CPU 170 selects the minimum among the measurement values of the first bioelectrical impedance $Z_a$ at the n-9th through n-th (ten) sampling times, and determines it to be the minimum MIN10(n) at the n-th sampling time.

Next, at step S55, the CPU 170 executes a median value calculating process for calculating the moving average of mean values AV 10(n) at the last 20 sampling times, in which AV10(n) is the mean value of the maximum MAX10 and the minimum MIN10 at a sampling time. For example, AV10(n) is the mean value of the maximum MAX10(n) and the minimum MINI10(n) at the n-th sampling time. More specifically, the CPU 170 calculates the moving average of the mean values AV10(n-19) through AV10(n) at the n-19th through n-th (twenty) sampling times, and decides the resulting moving average as a median value CNT20(n) at the n-th sampling time. The calculation is expressed as:

$$[AV10(n-19) + AV10(n-18) + ... + AV10(n)]/20 = CNT20(n).$$

Although not described in detail, the median value CNT20(n) is calculated in order to exclude artifacts (measurement errors that are unsuitable for processing) caused by body motion or other reasons.

Next, the CPU 170 executes a respiration timing extraction process for deciding respiration timing of the human subject at the n-th sampling time (step S56). In the following, with reference to Figs. 16 and 17, respiration timing extraction process will be described in detail. Figs. 16 and 17 form a flow chart showing an example of the respiration timing extraction process. As shown in Fig. 16, the CPU 170 first executes a differential coefficient calculating process for calculating the differential coefficient $dZ_a(n)$ of the first bioelectrical impedance $Z_a$ at the n-th sampling time (step S201). More specifically, the CPU 170 executes an arithmetic process in accordance with formula (3) below for calculating the differential coefficient $dZ_a(n)$.

$$[Z_a(n) - Z_a(n-2)]/1.2 = dZ_a(n) \quad ... \quad (3)$$

Next, the CPU 170 decides whether or not the absolute value of the differential coefficient $dZ_a(n)$ calculated at step S201 is less than 0.1 (step S202). If the decision at step S202 is affirmative, the CPU 170 sets the tendency

indication flag $F_0(n)$ to zero, and the process proceeds to step S204. The tendency indication flag $F_0(n)$ indicates tendency (increase, decrease or no trend) of the differential coefficient $dZ_a(n)$. The fact that the tendency indication flag $F_0(n)$ is zero indicates that the measurement value $Z_a(n)$ of the first bioelectrical impedance $Z_a$ at the n-th sampling time has no trend, that is to say, it is at a local maximum (peak value) or at a local minimum (bottom value).

If the decision at step S202 is negative, the CPU 170 decides whether or not the differential coefficient $dZ_a(n)$ is greater than zero (step S203). If the decision at step S203 is affirmative, the CPU 170 sets the tendency indication flag $F_0(n)$ to plus one, and the process proceeds to step S204. The fact that the tendency indication flag $F_0(n)$ is plus one indicates that the measurement value $Z_a(n)$ of the first bioelectrical impedance $Z_a$ at the n-th sampling time has a positive (increasing) trend. If the decision at step S203 is negative, the CPU 170 sets the tendency indication flag $F_0(n)$ to minus one, and the process proceeds to step S204. The fact that the tendency indication flag $F_0(n)$ is minus one indicates that the measurement value $Z_a(n)$ of the first bioelectrical impedance $Z_a$ at the n-th sampling time has a negative (decreasing) trend.

At step S204, the CPU 170 decides whether or not the absolute value of the tendency indication flag $F_0(n)$ at the n-th sampling time is equal to the absolute value of the tendency indication flag $F_0(n-1)$ at the n-1th sampling time. In addition, the CPU 170 decides whether or not the value of $F_0(n-1)$ is unequal to $F_0(n)$. If the composite decision at step S204 is affirmative, the CPU 170 sets the tendency indication $F_0(n)$ to zero, and the process proceeds to step S206 (see Fig. 17). If the decision at step S204 is negative, the CPU 170 holds $F_0(n)$ set before step S204, and the process proceeds to step S206.

With reference to Fig. 17, description of the respiration timing extraction process will be continued. The CPU 170 decides whether or not the tendency indication flag $F_0(n)$ at the n-th sampling time is zero (step S206). If the decision at step S206 is negative, the CPU 170 sets a peak-or-bottom indication flag $F_1(n)$ to zero, and the process proceeds to step S209. The fact that the peak-or-bottom indication flag $F_1(n)$ is zero indicates that the measurement values $Z_a(n)$ of the first bioelectrical impedance at the n-th sampling time is not the peak value or the bottom value.

If the decision at step S206 is affirmative, the CPU 170 decides whether or not the sum of the tendency indication flags $F_0$ at the last three sampling times is greater than plus one (step S207). More specifically, the CPU 170 calculates the sum of the tendency indication flags $F_0(n-2)$ through $F_0(n)$ at the n-2th through the n-th sampling times, and makes the decision.

If the decision at step S207 is affirmative, the CPU 170 sets the peak-or-bottom indication flag $F_1(n)$ to plus one, and the process proceeds to step S209. The fact that the peak-or-bottom indication flag $F_1(n)$ is plus one indicates that the measurement value $Z_a(n)$ of the first bioelectrical impedance at the n-th sampling time is at a local maximum (peak value).

If the decision at step S207 is negative, the CPU 170 decides whether or not the sum of the tendency indication flags $F_0$ at the last three sampling times is less than minus one (step S208). In other words, the CPU 170 decides whether or not the sum of the tendency indication flags $F_0(n-2)$ through $F_0(n)$ at the n-2th through the n-th sampling times is less than minus one.

If the decision at step S208 is affirmative, the CPU 170 sets the peak-or-bottom indication flag $F_1(n)$ to minus one, and the process proceeds to step S209. The fact that the peak-or-bottom indication flag $F_1(n)$ is minus one indicates that the measurement value $Z_a(n)$ of the first bioelectrical impedance at the n-th sampling time is at a local minimum (bottom value). If the decision at step S208 is negative, the CPU 170 sets the peak-or-bottom indication flag $F_1(n)$ to zero, and the process proceeds to step S209.

At step S209, the CPU 170 decides whether or not the peak-or-bottom indication flag $F_1(n)$ is plus one. If the decision at step S209 is negative, the CPU 170 adds one to a sampling counter value $N(n-1)$ at the n-1th sampling time (step S210). If the decision at step S209 is affirmative, the CPU 170 initializes the sampling counter value N (step S211). As will be understood from Figs. 9 and 10, irrespective of whether respiration of the human subject is costal respiration or abdominal respiration, the waveform of change in the first bioelectrical impedance $Z_a$ in respiration is nearly sinusoidal. The sampling counter value N is initialized to be zero whenever the measurement value of the first bioelectrical impedance $Z_a$ reaches a peak value. The sampling counter value N is incremented at each new sampling time, so that the number of sampling times is counted by the sampling counter until the next peak value of the measurement value of the first bioelectrical impedance $Z_a$. Thus, the respiration timing extraction process at step S56 in Fig. 15 is completed.

Returning to Fig. 15, description will be continued. After the above-described respiration timing extraction process is completed, the CPU 170 sets a respiration speed indication flag that indicates whether respiration of the human subject is fast or slow (step S57). In the following, with reference to Fig. 18, the respiration speed indication flag setting process executed by the CPU 170 at step S57 will be described in detail. Fig. 18 is a flow chart showing an example of the respiration speed indication flag setting process. As shown in Fig. 18, the CPU 170 first decides whether or not the tendency indication flag $F_0(n)$ is zero (step S301). If the decision at step S301 is negative, the CPU 170 considers that the respiration speed indication flag $F_{ma}(n)$ at the n-th sampling time as being equal to the respiration speed indication flag $F_{ma}(n-1)$ at the n-1th sampling time, and the process ends.

If the decision at step S301 is affirmative, the CPU 170 decides whether or not the peak-or-bottom indication

flag $F_1(n)$ is plus one (step S302). If the decision at step S302 is affirmative, the CPU 170 decides whether or not the sampling counter value N(n-1) at the n-1th sampling time is greater than ten (step S303). If the respiration speed of the human subject is slower, the time length between the time point at which the measurement value of the first bioelectrical impedance $Z_a$ is at a peak value and the time point at which the measurement value of the first bioelectrical impedance $Z_a$ arrives at the next peak value is longer, so that the sampling counter value N directly before the next peak value is larger. In this embodiment, when the CPU 170 decides that the measurement value of the first bioelectrical impedance $Z_a$ at n-th sampling time has reached a peak value, the CPU 170 decides whether or not the sampling counter value at the n-1th sampling time is greater than ten. If the CPU 170 has decided that the sampling counter value is greater than ten, the CPU 170 determines that the respiration of the human subject is slow. Specifically, if the decision at step S303 is affirmative, the CPU 170 sets the respiration speed indication flag $F_{ma}(n)$ to 20, and the process ends. The fact that the respiration speed indication flag $F_{ma}(n)$ is 20 indicates that the respiration of the human subject is slow. If the decision at step S303 is negative, the CPU 170 sets the respiration speed indication flag $F_{ma}(n)$ to ten, and the process ends. The fact that the respiration speed indication flag $F_{ma}(n)$ is ten indicates that respiration of the human subject is fast.

If the decision at step S302 is negative, the CPU 170 decides whether or not the peak-or-bottom indication flag $F_1(n)$ is minus one (step S304). If the decision at step S304 is negative, the CPU 170 determines that the respiration speed indication flag $F_{ma}(n)$ at the n-th sampling time is equal to respiration speed indication flag $F_{ma}(n-1)$ at the n-1th sampling time, and the process ends. If the decision at step S304 is affirmative, the CPU 170 decides whether or not the sampling counter value N(n-1) at the n-1th sampling time is greater than five (step S305). In this embodiment, when the CPU 170 decides that the sampling counter value N(n-1) directly before its arrival at the bottom value is greater than five, the CPU 170 determines that respiration of the human subject is slow. When the CPU 170 decides that the sampling counter value N(n-1) directly before its arrival at the bottom value is less than five, the CPU 170 determines that respiration of the human subject is fast. Specifically, if the decision at step S305 is affirmative, the CPU 170 sets the respiration speed indication flag $F_{ma}(n)$ to 20, and the process ends. If the decision at step S305 is negative, the CPU 170 sets the respiration speed indication flag $F_{ma}(n)$ to ten, and the process ends. The respiration speed indication flag setting process at step S57 in Fig. 15 is thus completed.

Returning to Fig. 15, description will be continued. Once the above-described respiration speed indication flag setting process is completed, the CPU 170 calculates the first centering value $Z_{a0}(n)$ at the n-th sampling time (step S58). In the following, with reference to Fig. 19, the first centering value calculating process executed by the CPU 170 at step S58 will be described in detail. Fig. 19 is a flow chart showing an example of the first centering value calculating process. As shown in Fig. 19, the CPU 170 first decides whether or not the respiration speed indication flag $F_{ma}(n)$ is ten (step S401). In other words, the CPU 170 decides whether or not respiration of the human subject at the n-th sampling time is fast.

In this embodiment, on the basis of the measurement values of the first bioelectrical impedance $Z_a$ at multiple sampling times within a centering period that is variable and is set depending on the respiration speed of the human subject, the first centering value $Z_{a0}(n)$ at the n-th sampling time is calculated or generated. If the decision at step S401 is affirmative (i.e., the respiration speed of the human subject at the n-th sampling time is fast), a time length (e.g., about four seconds) taken for a faster single respiration (single respiratory action) is set as the centering period. Specifically, if respiration of the human subject is fast, a period starting from the n-9th sampling time and ending at the n-th sampling time is set as the centering period, and the first centering value $Z_{a0}(n)$ is generated or calculated on the basis of the moving average MA10(n) of the measurement values of the first bioelectrical impedance at the n-9 th through n-th sampling times. In other words, if the decision at step S401 is affirmative, the CPU 170 generates or calculates the first centering value $Z_{a0}(n)$ at the n-th sampling time on the basis of the moving average MA10(n) calculated at step S51 in Fig. 15 (step S402). More specifically, the CPU 170 calculates the moving average of the first centering value $Z_{a0}(n-2)$ at the n-2th sampling time, the first centering value $Z_{a0}(n-1)$ at the n-1th sampling time, and the moving average MA10(n), and decides the resulting moving average as the first centering value $Z_{a0}(n)$ at the n-th sampling time. The calculation is expressed as:

$$[Z_{a0}(n\text{-}2) + Z_{a0}(n\text{-}1) + MA10(n)]/3 = Z_{a0}(n).$$

If the decision at step S401 is negative (i.e., the respiration speed of the human subject at the n-th sampling time is slow), a time length (e.g., about eight seconds) taken for a slower single respiration (single respiratory action) is set as the centering period. Specifically, if respiration of the human subject is slow, a period starting from the n-19th sampling time and ending at the n-th sampling time is set as the centering period, and the first centering value $Z_{a0}(n)$ is generated or calculated on the basis of the moving average MA20(n) of the measurement values of the first bioelectrical impedance at the n-19th through n-th sampling times. In other words, if the decision at step S401 is negative, the CPU 170 generates or calculates the first centering value $Z_{a0}(n)$ at the n-th sampling time on the basis of the moving average MA20(n) calculated at step S52 in Fig. 15 (step S403). More specifically, the CPU 170 calculates the moving average of the first centering value $Z_{a0}(n-2)$ at the n-2th sampling time, the first centering value $Z_{ao}(n-1)$ at the n-1th sampling time, and the moving average MA20(n), and decides the resulting moving average as the first centering value $Z_{a0}(n)$ at the n-th sampling time. The calculation is expressed as:

$[Z_{a0}(n-2) + Z_{a0}(n-1) + MA20(n)]/3 = Z_{a0}(n)$. The first centering process at step S50 in Fig. 14 is thus completed.

As described above, irrespective of whether respiration of the human subject is costal respiration or abdominal respiration, the waveform of change in the first bioelectrical impedance $Z_a$ in respiration is nearly sinusoidal. The CPU 170 (centering value generator) generates or calculates the first centering value $Z_{a0}$ on the basis of measurement values of the first bioelectrical impedance $Z_a$ at the sampling times of which the number is predetermined in order to obtain a suitable first centering value $Z_{a0}$ even if one or more instantaneous values of the first bioelectrical impedance $Z_a$ are disturbed by body motion or for other reasons. More specifically, at each sampling time, the CPU 170 generates or calculates a moving average MA10(n) or MA20(n) of measurement values of the first bioelectrical impedance at multiple sampling times within a centering period starting from a time point that is a predetermined time length before the n-th sampling time and ending at the n-th sampling time, and calculates the first centering value $Z_{a0}$ at the sampling time. Accordingly, even if one or more instantaneous values of the first bioelectrical impedance $Z_a$ are disturbed, a suitable first centering value $Z_{a0}$ can be generated. In addition, the time length of the centering period is variable and is set depending on the respiration speed of the human subject at the sampling time.

Returning to Fig. 14, description will be continued. As shown in Fig. 14, after the first centering process of step S50 is completed, the CPU 170 executes a first difference calculating process for calculating a first difference $\Delta Z_a(n)$ that is the difference between the instantaneous measurement value $Z_a(n)$ of the first bioelectrical impedance and the first centering value $Z_{a0}(n)$ (step S60). More specifically, the CPU 170 calculates the difference between the measurement value $Z_a(n)$ of the first bioelectrical impedance calculated at step S40 and the first centering value $Z_{a0}(n)$ calculated at step S50, and decides the difference as the first difference $\Delta Z_a(n)$ at the n-th sampling time.

After step S60, the CPU 170 generates or calculates a second centering value $Z_{b0}$ that is a standard level of change over time in the second bioelectrical impedance $Z_b$, and calculates a second difference $\Delta Z_b$ that is the difference between the instantaneous measurement value of the second bioelectrical impedance $Z_b$ and the second centering value $Z_{b0}$ (step S70).

As described above, change in the second bioelectrical impedance $Z_b$ during exhalations of abdominal respiration is completely different from that of the first bioelectrical impedance $Z_a$. Accordingly, in contrast to the calculation of the first centering value $Z_{a0}$, if a moving average is calculated on the basis of measurement values of the second bioelectrical impedance $Z_b$ at the sampling times of which the number is predetermined, the standard level (second centering value $Z_{b0}$) of change over time in the second bioelectrical impedance $Z_b$ cannot be calculated accurately.

Accordingly, in this embodiment, as shown in Fig. 20, zero-cross times are decided in which the instantaneous measurement value of the first bioelectrical impedance $Z_a$ is equal to the first centering value $Z_{a0}$. In addition, the second centering value $Z_{b0}$ is generated or calculated on the basis of measurement values of the second bioelectrical impedance $Z_b$ at the zero-cross times, so that the standard level (second centering value $Z_{b0}$) of change over time in the second bioelectrical impedance $Z_b$ can be calculated accurately. Fig. 20 is a graph for explaining a scheme for generating the second centering value $Z_{b0}$. In the following, with reference to Fig. 21, the second difference calculating process executed at step S70 by the CPU 170 will be described in detail.

Fig. 21 is a flow chart showing an example of the second difference calculating process. As shown in Fig. 21, the CPU 170 extracts the minimum among the first difference $\Delta Z_a$ at the last five sampling times (step S71). More exactly, the CPU 170 selects the minimum among the absolute value $|\Delta Z_a|$ ($|\Delta Z_a(n-4)|$ through $|\Delta Z_a(n)|$) of the first difference $\Delta Z_a$ at the n-4th through the n-th sampling times, and decides the minimum as a zero-cross-time reference value $\Delta MIN5(n)$ at the n-th sampling time.

Next, at step S72, the CPU 170 (zero-cross time decider) decides whether or not the zero-cross-time reference value at the n-1th sampling time is equal to the current zero-cross-time reference value decided at step S71. In addition, the CPU 170 decides whether or not the n-1th sampling time is a zero-cross time. More specifically, the CPU 170 decides whether or not the zero-cross-time reference value $\Delta MIN5(n-1)$ at the n-1th sampling time is equal to the current $\Delta MIN5(n)$. In addition, the CPU 170 decides whether or not a zero-cross-time reference flag $F_2(n-1)$ at the n-1th sampling time is set to plus one. When the zero-cross-time reference flag $F_2(n-1)$ is set to plus one, the n-1th sampling time is a zero-cross time. The initial value (default value) of the zero-cross-time reference flag $F_2$, i.e., the zero-cross-time reference flag $F_2(1)$ at the first sampling time, is zero.

If the decision at step S72 is affirmative, the CPU 170 sets the zero-cross-time reference flag $F_2(n)$ to zero, which means that the n-th sampling time is not a zero-cross time, and the process proceeds to step S74. If the decision at step S72 is negative, the CPU 170 decides whether or not the absolute value of the first difference $\Delta Z_a(n)$ at the n-th sampling time is equal to or less than 0.3 (step S73). If the decision at step S73 is negative, the CPU 170 sets the zero-cross-time reference flag $F_2(n)$ to zero, which means that the n-th sampling time is not a zero-cross time, and the process proceeds to step S74. If the decision at step S73 is affirmative, the CPU 170 sets the zero-cross-time reference

flag $F_2(n)$ to plus one, which means that the n-th sampling time is a zero-cross time, and the process proceeds to step S74.

Next, the CPU 170 decides whether or not the zero-cross-time reference flag $F_2(n)$ at the n-th sampling time is plus one (step S74). If the decision at step S74 is affirmative, the CPU 170 (centering value generator) calculates the second centering value $Z_{b0}$ (step S75). More specifically, the CPU 170 calculates the average of the second centering value $Z_{b0}(n-2)$ at the n-2th sampling time, the second centering value $Z_{b0}(n-1)$ at the n-1th sampling time, and the measurement value $Z_b(n)$ of the second bioelectrical impedance at the n-th sampling time, and decides the resulting average as the second centering value $Z_{b0}(n)$ at the n-th sampling time. The calculation is expressed as:

$$[Z_{b0}(\text{n-2}) + Z_{b0}(\text{n-1}) + Z_b(\text{n})]/3 = Z_{b0}(\text{n}).$$

If the decision at step S74 is negative, the CPU 170 decides the second centering value $Z_{b0}(n-1)$ at the n-1th sampling time as the second centering value $Z_{b0}(n)$ at the n-th sampling time. This is expressed as:

$$Z_{b0}(\text{n-1}) = Z_{b0}(\text{n}).$$

Then, the CPU 170 calculates the second difference $\Delta Z_b(n)$ at the n-th sampling time (step S76). More specifically, the CPU 170 calculates the difference between the instantaneous measurement values $Z_b(n)$ of the second bioelectrical impedance and the second centering value $Z_{b0}(n)$, and decides the difference as the second difference $\Delta Z_b(n)$ at the n-th sampling time. The second difference calculating process at step S70 in Fig. 14 is thus completed.

When respiration of the human subject is abdominal respiration and the first bioelectrical impedance $Z_a$ and the second bioelectrical impedance $Z_b$ vary as shown in Fig. 9, the waveforms of the first difference $\Delta Z_a$ and the second difference $\Delta Z_b$ are as shown in Fig. 22. In Fig. 22, the waveform of change over time in the first difference $\Delta Z_a$ is described in such a manner that the first centering value $Z_{a0}$ that is the standard level of measurement values of the first bioelectrical impedance $Z_a$ is placed at zero on the Y axis, whereas the waveform of change over time in the second difference $\Delta Z_b$ is described in such a manner that the second centering value $Z_{b0}$ that is the standard level of measurement values of the second bioelectrical impedance $Z_b$ is placed at zero on the Y axis. When both waveforms are overlapped as in Fig. 22, the first difference $\Delta Z_a$ and the second difference $\Delta Z_b$ at inhalations can be distinguished from each other whereas the first difference $\Delta Z_a$ and the second difference $AZ_b$ at exhalations can be distinguished from each other. In this embodiment, the CPU 170 decides whether the respiratory action of the human subject is inhalation or exhalation on the basis of the first difference $AZ_a$. More specifically, if the first difference $\Delta Z_a$ is positive, the CPU 170 decides that the respiratory action is inhalation. If the first difference $\Delta Z_a$ is negative, the CPU 170 decides that the respiratory action is exhalation. The coefficient in the above-mentioned regression formula (2) may be amended on the basis of the difference between the amplitudes of the first difference $\Delta Z_a$ and the second difference $\Delta Z_b$ and the difference between integral values of the first difference $\Delta Z_a$ and the second difference $\Delta Z_b$, so as to improve the accuracy of determination.

Returning to Fig. 14, description will be continued. As shown in Fig. 14, after the second difference calculating process at step S70 is completed, the CPU 170 executes a $\Delta R_{ib}/\Delta A_b$ assumption process for calculating the ratio $\Delta R_{ib}/\Delta A_b$ corresponding to the first difference $\Delta Z_a(n)$ and the second difference $\Delta Z_b(n)$ (step S80). In the following, with reference to Figs. 23 and 24, the $\Delta R_{ib}/\Delta A_b$ assumption process executed at step S80 by the CPU 170 will be described in detail. Figs. 23 and 24 form a flow chart showing an example of the $\Delta R_{ib}/\Delta A_b$ assumption process. As shown in Fig. 23, the CPU 170 first decides whether or not the first difference $\Delta Z_a(n)$ at the n-th sampling time is equal to or greater than zero (step S81).

If the decision at step S81 is negative, the CPU 170 decides that respiration of the human subject is exhalation, and assumes or calculates the $\Delta R_{ib}/\Delta A_b(n)$ at the n-th sampling time (step S82). More specifically, the CPU 170 executes an arithmetic process in accordance with the above-described regression formula (2) to calculate the ratio $\Delta R_{ib}/\Delta A_b(n)$ corresponding to the first difference $\Delta Z_a(n)$ and the second difference $\Delta Z_b(n)$.

If the decision at step S81 is affirmative, the CPU 170 decides that respiration of the human subject is inhalation, and sets the ratio $\Delta R_{ib}/\Delta A_b(n)$ at the n-th sampling time to an initial value. In this embodiment, if the decision at step S81 is affirmative, the CPU 170 sets the ratio $\Delta R_{ib}/\Delta A_b(n)$ at the n-th sampling time to the initial value, 1.0.

Next, the CPU 170 decides whether or not the ratio $\Delta R_{ib}/\Delta A_b(n)$ is equal to or greater than -2.5 and is equal to or less than 4.5 (step S83). If the decision at step S83 is negative, the CPU 170 sets the value of $\Delta R_{ib}/\Delta A_b$ to the initial value 1.0, and the process proceeds to step S84. If the decision at step S83 is affirmative, the CPU 170 proceeds the process directly to step S84.

At step S84, the CPU 170 decides whether or not the difference $(|\Delta R_{ib}/\Delta A_b(n)| - |\Delta R_{ib}/\Delta A_b(n-1)|)$ between the absolute value of the ratio $\Delta R_{ib}/\Delta A_b(n)$ and the absolute value of the ratio $\Delta R_{ib}/\Delta A_b(n-1)$ at the n-1th sampling time is greater than 0.3. If the decision at step S84 is negative, the CPU 170 calculates the average of the ratio $\Delta R_{ib}/\Delta A_b(n-1)$ at the n-1th sampling time and the ratio $\Delta R_{ib}/\Delta A_b(n)$, and decides the resulting average as the $\Delta R_{ib}/\Delta A_b(n)$ at the n-th sampling time, and the process proceeds to step S85 (see Fig. 24). The calculation is expressed as: $[\Delta R_{ib}/\Delta A_b(n-1) + \Delta R_{ib}/\Delta A_b(n)]/2 = \Delta R_{ib}/\Delta_b(n)$. If the decision at step S84 is affirmative, the CPU 170 sets the $\Delta R_{ib}/\Delta A_b$ (n) at the n-th sampling time to the initial value 1.0, and the process proceeds to step S85.

With reference to Fig. 24, description of the $\Delta R_{ib}/\Delta A_b$ assumption process will be continued. As shown in Fig. 24, at step S85, the CPU 170 decides whether or not the first difference $\Delta Z_a(n)$ at the n-th sampling time is less than zero. If the decision at step S85 is affirmative, the CPU 170 decides that respiration of the human subject is exhalation, and it adds one to the last count value $N_i$ of the number of integrations. More specifically, the CPU 170 adds one to the last count value $N_i(n-1)$ of the number of integrations at the n-1th sampling time, and decides the resulting sum as the current count value $N_i(n)$ of the number of integrations at the n-th sampling time.

If the decision at step S85 is negative, the CPU 170 decides that respiration of the human subject is inhalation, and sets the count value $N_i$ of the number of integrations to an initial value of zero. That is to say, the count value $N_i(n)$ of the number of integrations at the n-th sampling time is set to zero.

Then, as shown in Fig. 24, the CPU 170 decides whether or not the first difference $\Delta Z_a(n)$ is less than zero, again (step S86). If the decision at step S86 is affirmative, the CPU 170 calculates the sum of the integral value ($\Sigma\Delta R_{ib}/\Delta A_b(n-1)$) of the ratio $\Delta R_{ib}/\Delta A_b$ at the n-1th sampling time and the ratio $\Delta R_{ib}/\Delta Ab(n)$, and decides the resulting sum as the resulting sum as the integral value ($\Sigma\Delta R_{ib}/\Delta A_b(n)$) of the ratio $\Delta R_{ib}/\Delta A_b$ at the n-th sampling time. Then, the CPU 170 proceeds the process to step S87. If the decision at step S86 is negative (i.e., respiration of the human subject is inhalation), the CPU 170 sets the peak-or-bottom indication flag $F_1(n)$ to plus one, and the process proceeds to step S87.

Next, the CPU 170 decides whether or not the count value $N_i(n)$ of the number of integrations at the n-th sampling time is zero (step S87). If the decision at step S87 is negative, the CPU 170 divides the integral value $[\Sigma\Delta R_{ib}/\Delta A_b(n)]$ at the n-th sampling time by the count value $N_i(n)$ of the number of integrations at the n-th sampling time, so as to calculate the current average of the ratio $\Delta R_{ib}/\Delta A_b$. If the decision at step S87 is affirmative, the CPU 170 decides the last average ($[\Sigma\Delta R_{ib}/\Delta A_b(n-1)]/N_i(n-1)$) of the ratio $\Delta R_{ib}/\Delta A_b$ at the n-1th sampling time as the current average of the ratio $\Delta R_{ib}/\Delta A_b$ at the n-th sampling time. The $\Delta R_{ib}/\Delta A_b$ assumption process at step S80 in Fig. 14 is thus completed, whereby the respiration analysis process at the n-th sampling time is thus completed.

When respiration of the human subject is abdominal respiration and the first bioelectrical impedance $Z_a$ and the second bioelectrical impedance $Z_b$ vary as shown in Fig. 22, the above-described average ($[\Sigma\Delta R_{ib}/\Delta A_b]/N_i$) of the ratio $\Delta R_{ib}/\Delta A_b$ varies as shown in Fig. 25. In Fig. 25, if the average ($[\Sigma\Delta R_{ib}/\Delta A_b]/N_i$) of the ratio $\Delta R_{ib}/\Delta A_b$ at exhalations is equal to or less than 1.0, it is assumed that respiration of the human subject is abdominal respiration. If the average is greater than 1.0, it is assumed that respiration of the human subject is costal respiration.

Returning to Fig. 14, description will be continued. As shown in Fig. 14, after the $\Delta R_{ib}/\Delta A_b$ assumption process (step S80) is completed, the CPU 170 executes a respiration depth calculating process for calculating the respiration depth of the human subject (step S90).

In the following, with reference to Fig. 27, the respiration depth calculating process executed at step S90 by the CPU 170 will be described in detail. Fig. 27 is a flow chart showing an example of a respiration depth calculating process executed by the CPU 170 (respiration depth calculator). The respiration depth of the human subject calculated at the respiration depth calculating process is used in a respiration depth displaying process, as will be described later.

As shown in Fig. 27, the CPU 170 first decides whether or not the current time reaches a sampling time (step S501), and if the decision at step S501 is affirmative, the process proceeds to step S502. At step S502, the CPU 170 decides whether or not the respiratory action of the human subject is inhalation. Specifically, if the first difference $\Delta Z_a$ is positive, the CPU 170 decides that the respiratory action is inhalation. If the first difference $\Delta Z_a$ is negative, the CPU 170 decides that the respiratory action is exhalation. When the respiratory action is decided as inhalation, the CPU 170 sets the inhalation-or-exhalation flag $F_3$ to plus one. When the respiratory action is decided as exhalation, the CPU 170 sets the inhalation-or-exhalation flag $F_3$ to zero. In the initial status, the inhalation-or-exhalation flag $F_3$ is one as the default value.

If the decision at step S502 is affirmative (inhalation), the CPU 170 executes a peak-hold process (step S503). More specifically, the CPU 170 holds the maximum among the first differences $\Delta Z_a$ at multiple sampling times as a peak value $\Delta Z_a(MAX)$. If the decision at step S502 is negative (exhalation), the CPU 170 executes a bottom-hold process (step S504). More specifically, the CPU 170 holds the minimum among the first differences $\Delta Z_a$ at multiple sampling times as a bottom value $\Delta Z_a(MIN)$.

Next, at step S505, the CPU 170 decides whether or not the n-th sampling time is a zero-cross time. More specifically, the CPU 170 decides whether or not zero-cross-time reference flag $F_2$ at the sampling time is plus one, whereby deciding whether or not the sampling time is a zero-cross time. If the decision at step S505 is negative, the respiration depth calculating process at the sampling time ends. If the decision at step S505 is affirmative, at step S506, the CPU 170 decides whether or not the differential coefficient $dZ_a$ of the first bioelectrical impedance $Z_a$ at the sampling time is positive (greater than zero). In other words, the CPU 170 decides whether or not the respiratory action is changing from exhalation to inhalation at the sampling time.

If the decision at step S506 is affirmative, on the assumption that the respiratory action is changing from exhalation to inhalation at the n-th sampling time, the CPU 170 calculates the sum of the absolute values of the peak value $\Delta Z_a(MAX)$ and the bottom value $\Delta Z_a(MIN)$ held at that time, and decides the resulting sum as the respiration depth $Z_{ap-p}$ at the last respiratory action (step S507). Then, the CPU 170 executes an inhalation flag setting process (step S508). More specifically, the CPU 170 sets the inhalation-or-exhalation flag $F_3$ to plus one. Then, the CPU 170 initializes the peak-hold process (step S509). More specifically, the CPU 170 sets the peak value $\Delta Z_a(MAX)$ set at step S503 to the initial value zero, and ends the respiration depth calculating process at the sampling time.

If the decision at step S506 is negative, on the assumption that the respiratory action is changing from inhalation to exhalation at the sampling time, the CPU 170 executes an exhalation flag setting process (step S510). More specifically, the CPU 170 sets the inhalation-or-exhalation flag $F_3$ to zero. Then, the CPU 170 initializes the bottom-hold process (step S511). More specifically, the CPU 170 sets the bottom value $\Delta Z_a(MIN)$ set at step S504 to the initial value zero, and ends the respiration depth calculating process at the sampling time.

As has been described above, in this embodiment, the CPU 170 executes the respiration analysis process at every sampling time, so as to calculate the ratio $\Delta R_{ib}/\Delta A_b$ corresponding to the first difference $\Delta Z_a$ and the second difference $\Delta Z_b$ at every sampling time. Accordingly, it is possible to accurately decide the type of respiration of the human subject (abdominal respiration or costal respiration) in real time.

1.5 RESPIRATION DEPTH DISPLAYING PROCESS

Next, a respiration depth displaying process executed by the CPU 170 will be described. In this embodiment, the CPU 170 executes the respiration depth displaying process at every respiration of the human subject for displaying (reporting) the magnitude of each of abdominal respiration and costal respiration and a margin level beyond the essential respiration depth with respect to each of abdominal respiration and costal respiration in the single respiration. More specifically, at every respiration of the human subject, on the basis of the respiration depth at the respiration and the results of the respiration analysis process at the respiration, the CPU 170 controls the display device 160 (reporter) to show the magnitude of each of costal respiration and abdominal respiration and the margin level with respect to costal respiration and abdominal respiration at the single respiration. As shown in Fig. 26, in this embodiment, in addition to the magnitude of each of costal respiration and abdominal respiration and the margin level with respect to costal respiration and abdominal respiration at the single respiration, the CPU 170 controls the display device 160 to display the abdominal respiration percentage level that is a ratio of abdominal respiration in the single respiration. A first bar graph BG1 I shown in Fig. 26 indicates the magnitude of each of costal respiration and abdominal respiration and the margin level with respect to costal respiration and abdominal respiration. A second bar graph BG2 shown in Fig. 26 indicates the abdominal respiration percentage level of the human subject. The bar graphs will be described later in more detail.

With reference to Fig. 28, the respiration depth displaying process executed by the CPU 170 will be described in detail. Fig. 28 is a flow chart showing an example of the respiration depth displaying process. As shown in Fig. 28, the CPU 170 (normalizer) normalizes the respiration depth at the last single respiration (step S601). In this specification, "normalize a (the) respiration depth" is meant to adjust the respiration depth at the last single respiration calculated by the respiration depth calculating process in order to exclude individual variation of physical constitutions of human subjects. Specifically, a normalized respiration depth value $\%\Delta Z_a$ is calculated as the respiration depth $\Delta Z_{ap-p}$ at the last respiration divided by the second centering value $Z_{b0}$ at the last respiration (more exactly, the average of the second centering values $Z_{b0}$ at multiple sampling times within the last respiration) multiplied by 100. The calculation is expressed as:

$$\%\Delta Z_a = (\Delta Z_{ap-p}/Z_{b0}) * 100$$

Next, at step S602, the CPU 170 decides the number of degree indicators to be colored in the first bar graph BG1 on the basis of the normalized respiration depth value $\%\Delta Z_a$ calculated at step S601. The number of colored degree indicators will be referred to as a "first colored-degree-indicator number". More specifically, the CPU 170 calculates a normalized value $\%\Delta T_V$ of a one-time ventilation volume corresponding to the normalized respiration depth value $\%\Delta Z_a$ calculated at step S601, and decides the first colored-degree-indicator number on the basis of the normalized one-time ventilation volume $\%\Delta T_V$. The term "one-time ventilation volume" is meant to be a volume of air entering and leaving the lungs of the human subject in a single respiratory action, and will be referred to as $\Delta T_V$. The term "normalize a (the) one-time ventilation volume" is meant to amend a one-time ventilation volume $\Delta T_V$ in order to exclude individual variation of physical constitutions of human subjects. In this embodiment, the normalized one-time ventilation volume $\%\Delta T_V$ is calculated using a coefficient $\%V_C$ that was calculated as the breathing capacity $V_{CA}$ of the human subject measured actually by a spirometer or other suitable device divided by the normal breathing capacity $V_{CN}$. This calculation is expressed as $\%V_C = V_{CA}/V_{CN}$). As is well known, the normal breathing capacity $V_{CN}$ is (27.63 - 0.112 *age) * height (cm) for males and is (21.78 - 0.101 * age) * height (cm) for females.

Fig. 29 is a diagram showing relationships between the normalized one-time ventilation volume $\%\Delta T_V$ and the normalized respiration depth value $\%\Delta Z_a$. Fig. 29 shows results of experiments in which a one-time ventilation volume $\Delta T_V$ is measured three times (small ventilation once, middle ventilation once, and large ventilation once) for each of 20 human subjects. As shown in Fig. 29, there is a close correlative relationship between the normalized one-time ventilation volume $\%\Delta T_V$ and the normalized respiration depth value $\%\Delta Z_a$, in which the coefficient of correlation R = 0.75, and the probability P < 0.01. Regression formula (4) below is derived from the correlative relationship.

$$\%\Delta Z_a = c_0 * \%\Delta T_V \quad ... \quad (4)$$

where $c_0$ is a coefficient of regression. According to the inventor's analysis, $c_0$ is 50.954.

In accordance with the above-described regression formula (4) (second formula), the CPU 170 (reporter) calculates the normalized one-time ventilation volume $\%\Delta T_V$ corresponding to the normalized respiration depth value $\%\Delta Z_a$ calculated at step S601. Then, on the basis of the calculated the normalized one-time ventilation volume $\%\Delta T_V$, the CPU 170 decides the first colored-degree-indicator number. In this embodiment, the maximum of the first colored-degree-indicator number is ten, which includes five degrees for costal respiration and five degrees for abdominal respiration (see Fig. 26).

As shown in Fig. 29, in this embodiment, when the normalized one-time ventilation volume $\%\Delta T_V$ is equal to or greater than a first predetermined value $\alpha_1$, the respiration depth of the human subject is considered to be the maximum. In this case, the CPU 170 decides the first colored-degree-indicator number as ten, which is the maximum. When the normalized one-time ventilation volume $\%\Delta T_V$ is equal to or greater than a second predetermined value $\alpha_2$, which is less than $\alpha_1$, and is less than the first predetermined value $\alpha_1$, the respiration depth of the human subject is considered to be large (see Fig. 29). In this case, the CPU 170 decides the first colored-degree-indicator number as eight. When the normalized one-time ventilation volume $\%\Delta T_V$ is equal to or greater than a third predetermined value $\alpha_3$, which is less than $\alpha_2$, and is less than the second predetermined value $\alpha_2$, the respiration depth of the human subject is considered to be in the middle (see Fig. 29). In this case, the CPU 170 decides the first colored-degree-indicator number as six. When the normalized one-time ventilation volume $\%\Delta T_V$ is equal to or greater than a fourth predetermined value $\alpha_4$, which is less than $\alpha_3$, and is less than the third predetermined value $\alpha_3$, the respiration depth of the human subject is considered to be small (see Fig. 29). In this case, the CPU 170 decides the first colored-degree-indicator number as four. When the normalized one-time ventilation volume $\%\Delta T_V$ is less than the fourth predetermined value $\alpha_4$, the respiration depth of the human subject is considered to be an essential respiration depth at rest (see Fig. 29). In this case, the CPU 170 decides the first colored-degree-indicator number as two.

Returning to Fig. 28, description will be continued. As shown in Fig. 28, after the decision of the number of degree indicators to be colored (step S602), the CPU 170 (abdominal respiration percentage level calculator) decides the abdominal respiration percentage level at the last single respiration (step S603). Specifically, the CPU 170 decides a percentage value of the abdominal respiration percentage level on the basis of the ratio $\Delta R_{ib}/\Delta A_b$ at the last single respiration (more exactly, the average of the ratios $\Delta R_{ib}/\Delta A_b$ at multiple sampling times within the last single respiration). The abdominal respiration percentage level is represented in a number within a range from zero to 100. The greater the abdominal respiration percentage level, the greater the degree of abdominal respiration (the greater the ratio of abdominal respiration in respiration of the human subject).

As shown in Fig. 28, after step S603, the CPU 170 executes a degree-indicator-number distribution process for distributing the first colored-degree-indicator number decided at step S602 to abdominal respiration and costal respiration (step S604). More specifically, the CPU 170 executes the degree-indicator-number distribution process on the basis of the first colored-degree-indicator number decided at step S602 and the abdominal respiration percentage level decided at step S602. As an example, let us assume that the first colored-degree-indicator number indicating the whole respiration depth is six, whereas the abdominal respiration percentage level is 70. The abdominal respiration percentage level "70" indicates that the ratio of abdominal respiration to costal respiration is 7:3. The first colored-degree-indicator number "6" is divided by the ratio indicated by the abdominal respiration percentage level, so that four degree-indicators are allocated to abdominal respiration and two degree-indicators are allocated to costal respiration. Consequently, as shown in Fig. 26, the colored-degree-indicator number in the first bar graph BG1 that indicates the magnitude of costal respiration is set to two, whereas the colored-degree-indicator number in the first bar graph BG 1 that indicates the magnitude of abdominal respiration is set to four.

As shown in Fig. 28, after step S604, in accordance with the respiration speed of the human subject, the CPU 170 (reporter) decides a margin level with respect to each of abdominal respiration and costal respiration in the last single respiration (step S605). The margin level is a respiration depth beyond the essential respiration depth at rest with respect to each of abdominal respiration and costal respiration. The essential respiration depth at rest corresponds to the respiration speed of the human subject. In this embodiment, the relationship between respiration speed and the degree of essential respiration depth at rest is described in the program for executing the respiration depth displaying process. In accordance with the relationship, on the basis of the respiration speed, the CPU 170 controls the display device 160 to indicate the degree of essential respiration depth in the first bar graph BG 1 shown in Fig. 26.

Let us assume that the last single respiration took four seconds or more. The degree of essential respiration depth at rest is two in accordance with the relationship. The CPU 170 distributes the number two equally to abdominal respiration and costal respiration, so that one degree is allocated to abdominal respiration, whereas one degree is allocated to costal respiration, and causes the display device 160 to show the degree of essential respiration depth (one) for each of abdominal respiration and costal respiration in the first bar graph BG1 (as depicted by a faint color in Fig. 26). As described in conjunction with the above-described step S604, the colored-degree-indicator number in the first bar graph BG1 that indicates the magnitude of costal respiration is set to two, whereas the colored-degree-indicator number in the first bar graph BG1 that indicates the magnitude of abdominal respiration is set to four. The margin level is the respiration depth beyond the essential respiration depth. Therefore, the margin level for costal respiration is degree one, whereas that for abdominal respiration is degree three (as depicted by a deep color in Fig. 26). As a result, the display device 160 displays the margin level for each of abdominal respiration and costal respiration in the first bar graph BG 1.

In this case, if the colored-degree-indicator number in the first bar graph BG1 that indicates the magnitude of abdominal respiration is one or more, the magnitude of abdominal respiration of the human subject satisfies the essential level. If the colored-degree-indicator number is two, the magnitude of abdominal respiration of the human subject has a small margin level beyond the essential level. If the colored-degree-indicator number is three, the magnitude of abdominal respiration of the human subject has a middle margin level beyond the essential level. If the colored-degree-indicator number is four, the magnitude of abdominal respiration of the human subject has a large margin level beyond the essential level. If the colored-degree-indicator number is five, the magnitude of abdominal respiration of the human subject has the maximum margin level beyond the essential level. As described above, in the assumed example, since the colored-degree-indicator number in the first bar graph BG1 that indicates the magnitude of abdominal respiration is four, the magnitude of abdominal respiration of the human subject has a large margin level beyond the essential level (faint colored-degree-indicator number one). The same is true for costal respiration. In the assumed example, since the colored-degree-indicator number in the first bar graph BG1 that indicates the magnitude of costal respiration is two, the magnitude of costal respiration of the human subject has a small margin level beyond the essential level (faint colored-degree-indicator number one).

The higher the respiration speed, the greater the essential respiration depth at rest. This means that when the respiration speed or rate is higher, the degree of essential respiration depth for each of abdominal respiration and costal respiration depicted by a faint color in the first bar graph BG1 is greater. For example, if the cycle of respiration is equal to or greater than three seconds and is less than four seconds, the degree of essential respiration depth for each of abdominal respiration and costal respiration depicted by a faint color in the first bar graph BG1 is four. The CPU 170 distributes the number four equally to abdominal respiration and costal respiration, so that two degree-indicators are allocated to abdominal respiration, whereas two degree-indicators are allocated to costal respiration, and causes the display device 160 to show the degree of essential respiration depth (two) in a faint color for each of abdominal respiration and costal respiration in the first bar graph BG1. The margin level for each of costal respiration and abdominal respiration is based on the thus allocated degree of essential respiration depth.

After step S605, the CPU 170 causes the display device 160 to show the magnitude of each of costal respiration and abdominal respiration and the margin level with respect to costal respiration and abdominal respiration in the first bar graph BG1, and to show the abdominal respiration percentage level in the second bar graph BG2 (step S606). As shown in Fig. 26, since the degree of essential respiration depth and the margin level are depicted in different colors, the human subject or another person can easily understand the magnitude of each of abdominal respiration and costal respiration and the margin level beyond the essential respiration depth of the human subject.

As shown in Fig. 26, the abdominal respiration percentage level depicted in the second bar graph BG2 is classified into five degrees corresponding to five sections. According to the abdominal respiration percentage level calculated at the above-described step S603, one of the five sections in the second bar graph BG2 is colored. Specifically, if the abdominal respiration percentage level is from zero to 20, the CPU 170 causes the display device 160 to color the uppermost section. If the abdominal respiration percentage level is from 21 to 40, the CPU 170 causes the display device 160 to color the second top section. If the abdominal respiration percentage level is from 41 to 60, the CPU 170 causes the display device 160 to color the middle section. If the abdominal respiration percentage level is from 61 to 80, the CPU 170 causes the display device 160 to color the second bottom section. If the abdominal respiration percentage level is from 81 to 100, the CPU 170 causes the display device 160 to color the lowermost section. As described above, in the illustrated example, the abdominal respiration percentage level is 70, and the second bottom section in the second bar graph BG2 is colored as shown in Fig. 26.

As has been described above, in this embodiment, the CPU 170 causes the display device 160 to show the magnitude of each of abdominal respiration and costal respiration and a margin level beyond the essential respiration depth with respect to each of abdominal respiration and costal respiration at every respiration of the human subject. Accordingly, the human subject or another person can understand strengths and weaknesses of activity of the costal respiratory muscles and abdominal respiratory muscles of the human subject. Whereas the human subject is made aware of the strength of the human subject, the human subject may be motivated to train the respiratory muscles by, for example, voluntary abdominal respiration in order to overcome a weakness. In accordance with this embodiment, the margin level of the respiration capability of the human subject can be known even if the human subject does not breathe at the maximum respiration depth, in contrast to use of spirometers. Therefore, the use of the body condition determination apparatus is safer for human subjects than the use of spirometers.

## 2. SECOND EMBODIMENT

In addition to costal respiration and abdominal respiration, respiration of human beings includes a draw-in respiration in which inhalation and exhalation are repeated with the abdomen held in a constricted position. By draw-in respiration, inner muscles at the body trunk (e.g., the transverse abdominal muscle and the erector muscle of the spine) that are not frequently used in normal daily activity can be toned up effectively. Strengthening the inner muscles improves the function of respiration, and strengthening muscles at the body trunk supporting the backbone improves the motor function. Accordingly, draw-in respiration has been incorporated into training of athletes in order to improve motion function. In addition, draw-in respiration has been recommended for improvement or prevention of backaches in the fields of physical therapy and rehabilitation. Draw-in respiration is also effective for dieting.

In accordance with a modification of the body condition determination apparatus 1 of the first embodiment, it is possible to determine that the type of respiration of the human subject is costal respiration, abdominal respiration, or draw-in respiration. This modification will be described in a second embodiment. The structure of the body condition determination apparatus in the second embodiment is the same as that in the first embodiment, and therefore the same elements as in the first embodiment will not be described in detail and the same reference symbols for such elements will be used in the following description.

Fig. 30 is a graph showing change over time of each of the first bioelectrical impedance $Z_a$ at the upper body trunk and the second bioelectrical impedance $Z_b$ at the middle body trunk when respiration is draw-in respiration. As shown in Fig. 30, in draw-in respiration, both of the first bioelectrical impedance $Z_a$ and the second bioelectrical impedance $Z_b$ increase at inhalations, whereas both of the first bioelectrical impedance $Z_a$ and the second bioelectrical impedance $Z_b$ decrease at exhalations. The manner of change is the same as that in costal respiration since human beings expand and contract the thoracic cage in both of costal respiration and draw-in respiration. However, in draw-in respiration, the abdomen is held in a constricted position continually so as to be stressed continually. Therefore, the standard level of the second bioelectrical impedance $Z_b$ at the middle body trunk in draw-in respiration is higher than that in costal respiration, as shown in Fig. 30.

Thus, it is possible to determine whether or not respiration of the human subject is draw-in respiration on the basis of the difference in the standard level of the second bioelectrical impedance $Z_b$ between draw-in respiration and costal respiration. In the first embodiment, by the respiration analysis process (Fig. 14) described in conjunction with the first embodiment, the type of respiration of the human subject is determined as costal or abdominal on the basis of the average of the ratio $\Delta R_{ib}/\Delta A_b$. If respiration is determined as costal as the average of the ratio $\Delta R_{ib}/\Delta A_b$ is greater than one, determination is again made as to whether the type of respiration is actually costal respiration or is draw-in respiration. More specifically, if the standard level (second centering value $Z_{b0}$) of the second bioelectrical impedance $Z_b$ is higher than the standard level in costal respiration by a predetermined value or more, respiration of the human subject is draw-in respiration.

Each human subject may have a different standard level (second centering value $Z_{b0}$) of the second bioelectrical impedance $Z_b$ in costal respiration. Accordingly, it is preferable that, in order to distinguish draw-in respiration, the standard level (second centering value $Z_{b0}$) of the second bioelectrical impedance $Z_b$ in costal respiration be determined in advance for the human subject. More preferably the standard level in costal respiration may be stored in the second memory 130. In addition, the above-described predetermined value should be stored in the first memory 120 in advance.

Accordingly, the CPU 170 of the body condition determination apparatus 1 in this embodiment reports a message for instructing the human subject to repeat costal respiration, and calculates the average of multiple second centering values $Z_{b0}$ during the repetition of costal respiration. The CPU 170 stores the average as the standard level of the second bioelectrical impedance $Z_b$ in the second memory 130. Hereinafter, the standard level of the second bioelectrical impedance $Z_b$ stored in the second memory 130 for costal respiration will be referred to as $Z_{b1}$.

On the other hand, the above-described predetermined value to be stored in the first memory 120 can be determined in accordance with experiments in which the standard level of the second bioelectrical impedance $Z_b$ for each of many human subjects in costal respiration is determined, and the standard level of the second bioelectrical impedance $Z_b$ for each of many human subjects in draw-in respiration is also determined. The predetermined value corresponds to the difference between the standard level in costal respiration and the standard level in draw-in respiration. Hereinafter, the predetermined value stored in the first memory 120 will be referred to as $\Delta Z_{b1}$.

Fig. 31 is a flow chart showing an example of a respiration type determination process. This process is executed after completion of the respiration analysis process described in conjunction with the first embodiment. In a manner similar to the first embodiment, in this embodiment, the CPU 170 also executes the respiration analysis process shown in Fig. 14 at each sampling time.

Then, the CPU 170 starts the respiration type determination process. The CPU 170 first decides whether or not the average of $\Delta R_{ib}/\Delta A_b$ ($[\Sigma\Delta R_{ib}/\Delta A_b]/N_i$) is equal to or less than 1.0 (step S701). If the decision at step S701 is affirmative, the CPU 170 determines that respiration of the human subject is abdominal respiration (step S702).

If the decision at step S701 is negative, the CPU 170 retrieves the standard level $Z_{b1}$ of the second bioelectrical impedance $Z_b$ in costal respiration from the second memory 130, and retrieves the predetermined value $\Delta Z_{b1}$ from the first memory 120 (step S703). At this step, the predetermined value $\Delta Z_{b1}$ retrieved from the first memory 120 may be amended on the basis of the personal or individual body information (including the height, age, and sex) entered at step S 1 (Fig. 5) or the weight measured at step S2 (Fig. 5).

Then, the CPU 170 decides whether or not the second centering value $Z_{b0}$ generated at step S70 in the second difference calculating process for calculating the second difference $\Delta Z_b$ is equal to or greater than the sum of the standard level $Z_{b1}$ and the predetermined value $\Delta Z_{b1}$ (step S704). The second centering value $Z_{b0}$ used in the comparison at step S704 may be, for example, the average of the second centering values $Z_{b0}$ within the last single respiration or within the last multiple respirations. If the decision at step S704 is affirmative, the CPU 170 determines that respiration of the human subject is draw-in respiration (step S705). If the decision at step S704 is negative, the CPU 170 determines that respiration of the human subject is costal respiration (step S706).

As has been described above, according to this embodiment, it is possible to determine accurately that the type of respiration of the human subject is abdominal respiration, costal respiration, or draw-in respiration in real time. In an alternative embodiment, the body condition determination apparatus 1 may determine whether or not respiration of the human subject is draw-in respiration (and need not determine that the type of respiration of the human subject is abdominal or costal).

In this embodiment, the standard level $Z_{b1}$ of the second bioelectrical impedance $Z_b$ in costal respiration stored in the second memory 130 is the average of a plurality of second centering value $Z_{b0}$ determined during costal respiration. However, the present invention should not be limited to the disclosure. For example, the standard level $Z_{b1}$ may be a second bioelectrical impedance $Z_b$ determined during costal respiration.

At step S703, the CPU 170 compares the second centering value $Z_{b0}$ with the sum of the standard level $Z_{b1}$ and the predetermined value $\Delta Z_{b1}$. However, the present invention should not be limited to the disclosure. For example, the CPU 170 may multiply the standard level $Z_{b1}$ by a predetermined coefficient (e.g., 1.035) greater than 1.0, and then may determine whether or not the second centering value $Z_{b0}$ is equal to or greater than the standard level $Z_{b1}$ multiplied by the coefficient. The coefficient can also be determined in accordance with experiments in which the standard level of the second bioelectrical impedance $Z_b$ for each of many human subjects in costal respiration is determined, and the standard level of the second bioelectrical impedance $Z_b$ for each of many human subjects in draw-in respiration is also determined. The coefficient corresponds to the ratio between the standard level in costal respiration and the standard level in draw-in respiration. The value obtained as the standard level $Z_{b1}$ multiplied by the coefficient may be stored in the second memory 130 instead of the standard level $Z_{b1}$ per se, and at step S703 the CPU 170 may compare the second centering value $Z_{b0}$ with the value retrieved from the second memory 130.

The respiration depth calculating process and the respiration depth displaying process described in conjunction with the first embodiment are executed irrespective to the type of respiration of the human subject. That is to say, they are executed even if the human subject performs draw-in respiration. As a result, even in draw-in respiration, the first bar graph BG1 and the second bar graph BG2 shown in Fig. 26 are shown on the display device 160. The human subject or another person can know the magnitude of draw-in respiration of the human subject by observing the degree indicators indicative of magnitude of costal respiration in the first bar graph BG1.

The second embodiment may be modified as follows.

2.1. FIRST MODIFICATION OF SECOND EMBODIMENT

As shown in Fig. 30, in draw-in respiration, only the standard level of the second bioelectrical impedance $Z_b$ at the middle body trunk increases in comparison with that in costal respiration and abdominal respiration. Accordingly, the CPU 170 may continually monitor the first centering value $Z_{a0}$ that is the standard level of the first bioelectrical impedance $Z_a$ at the upper body trunk and the second centering value $Z_{b0}$ that is the standard level of the second bioelectrical impedance $Z_b$ at the middle body trunk, and it may determine that the last respiration is draw-in respiration when only the second centering value $Z_{b0}$ increases by a predetermined value (e.g., 0.5 ohms).

## 2.2. SECOND MODIFICATION OF SECOND EMBODIMENT

As shown in Fig. 30, in draw-in respiration, both of the amplitudes (local maximum-local minimum) of the first bioelectrical impedance $Z_a$ and the second bioelectrical impedance $Z_b$ are less than those in costal respiration and abdominal respiration. In order to identify draw-in respiration on the basis of the amplitudes, thresholds for both amplitudes may be determined and stored in the first memory 120. The CPU 170 may continually monitor the amplitudes of the first bioelectrical impedance $Z_a$ and the second bioelectrical impedance $Z_b$, and may determine that the last respiration is draw-in respiration when both amplitudes are less than the thresholds (e.g., 1.8 ohms) and when only the second centering value $Z_{b0}$ increases by a predetermined value (in a manner similar to the first modification). The thresholds may be different from each other, or they may be the same as each other, so that a single common threshold may be stored in the first memory 120.

## 2.3. THIRD MODIFICATION OF SECOND EMBODIMENT

In draw-in respiration, both of the first bioelectrical impedance $Z_a$ and the second bioelectrical impedance $Z_b$ increase at inhalations, whereas both of the first bioelectrical impedance $Z_a$ and the second bioelectrical impedance $Z_b$ decrease at exhalations, in a manner similar to that in costal respiration. Accordingly, the CPU 170 may continually monitor the first bioelectrical impedance $Z_a$ and the second bioelectrical impedance $Z_b$, and may determine that the last respiration is draw-in respiration when both of the first bioelectrical impedance $Z_a$ and the second bioelectrical impedance $Z_b$ increase at inhalations, whereas both of the first bioelectrical impedance $Z_a$ and the second bioelectrical impedance $Z_b$ decrease at exhalations and when only the second centering value $Z_{b0}$ increases by a predetermined value (in a manner similar to that in the first modification).

## 3. THIRD EMBODIMENT

A Lissajous figure showing the status of breathing of the human subject, e.g., change over time in each of the first bioelectrical impedance $Z_a$ and the second bioelectrical impedance $Z_b$ is a convenient expedient for reporting whether respiration of the human is mainly dependent on costal respiration or abdominal respiration. When respiration of the human subject is costal respiration, as shown in Fig. 10, both the first bioelectrical impedance $Z_a$ and the second bioelectrical impedance $Z_b$ increase at inhalations, whereas both the first bioelectrical impedance $Z_a$ and the second bioelectrical impedance $Z_b$ decrease at exhalations. Therefore, the Lissajous figure showing this case within a single respiration is, for example, as shown in Fig. 32. That is to say, when the ratio of costal respiration in a single respiration is extremely high, the Lissajous figure indicates a substantially straight track.

When respiration of the human subject is abdominal respiration, as shown in Fig. 9, both of the first bioelectrical impedance $Z_a$ and the second bioelectrical impedance $Z_b$ increase at inhalations, but the first bioelectrical impedance $Z_a$ decreases, whereas the second bioelectrical impedance $Z_b$ increases at exhalations. Therefore, the Lissajous figure showing this case within a single respiration is, for example, as shown in Fig. 33. That is to say, when a single respiration includes abdominal respiration, the Lissajous figure indicates a track of a bent shape, e.g., a boomerang shape.

Accordingly, by presenting a Lissajous figure showing the status of the last respiration of the human subject, the human subject or another person can easily understand whether respiration of the human is costal respiration or abdominal respiration. In this case, it is not necessary to execute the above-described respiration analysis process, and the type of respiration of the human subject can be assumed in a simple fashion. In addition, the human subject can cause the human subject's respiration to resemble costal respiration by breathing so that the human subject's Lissajous figure indicates a track resembling a straight line. Accordingly, presenting a Lissajous figure is used as biofeedback information for training for appropriate breathing.

The Lissajous figures of Figs. 32 and 33 show only the status of the last single respiration of the human subject. However, it is possible to generate Lissajous figures showing the status of multiple respirations on the basis of data on first bioelectrical impedances $Z_a$ and the second bioelectrical impedances $Z_b$ in the multiple respirations as shown in Figs. 34 and 35. When the ratio of costal respiration in multiple respirations is high (respiration of the human is mainly dependent on costal respiration), the Lissajous figure is, for example, as shown in Fig. 34. When multiple respirations include abdominal respiration, the Lissajous figure is, for example, as shown in Fig. 35. In addition, it is possible to change to display the Lissajous figure at every respiration of the human subject in order to show the instantaneous status cyclically.

A third embodiment for displaying a Lissajous figure will be described. The structure of the body condition determination apparatus in the third embodiment is the same as that in the first embodiment, and therefore the same elements as in the first embodiment will not be described in detail and the same reference symbols for such elements will be used in the following description.

## 3.1. DISPLAYING NORMAL LISSAJOUS FIGURE

Fig. 36 is a flow chart showing an example of a Lissajous figure displaying process. In the flow chart, the processes of steps S801 through S804 are the same as those in the above-described first embodiment (steps S10 through S40 in Fig. 14), and will not be described in detail.

After step S804, the CPU 170 (display data generator) generates display data for displaying a Lissajous figure (step S805). In the Lissajous figure, for example, the X axis is the second bioelectrical impedance $Z_b$, whereas the Y axis is the first bioelectrical impedance $Z_a$. The second memory 130 has a Lissajous figure memory area for temporarily storing the display data for displaying a Lissajous figure in the display device 160.

The display data represents coordinates of dots to be shown in a screen for the Lissajous figure, in which each dot has an x-coordinate indicating a measurement value of the second bioelectrical impedance $Z_b$ at one time and a y-coordinate indicating a measurement value of the first bioelectrical impedance $Z_a$ at that time. Whenever a new dot position is determined, the CPU 170 updates the display data in order to update the track in the Lissajous figure.

The CPU 170 retrieves the display data from the Lissajous figure memory area, and causes the display device 160 to show the Lissajous figure indicated by the display data (step S806). The Lissajous figure displaying process is executed at every sampling time, so that the display data is updated at every sampling time and the Lissajous figure displayed on the display device 160 is also updated at every sampling time.

The display device 160 thus shows the Lissajous figure in real time insofar as the human subject performs respiration. When the ratio of costal respiration in respiration is extremely high, the display device 160 will show a Lissajous figure similar to that in Fig. 32 or 34. When respiration includes abdominal respiration, the display device 160 will show a Lissajous figure similar to that in Fig. 33 or 35. When respiration of the human subject is changing from costal to abdominal, the display device 160 will show a Lissajous figure similar to that in Fig. 37, in which the track of the Lissajous figure is changing from a straight shape rising from bottom left to top right to a bent shape of which the lower portion is downward-sloping.

As shown in Figs. 32 and 34, when the ratio of costal respiration in respiration is extremely high, the track of the Lissajous figure is of a straight shape rising from bottom left to top right. When costal respiration is shallow, the track of the Lissajous figure is small. When costal respiration is deep, the track of the Lissajous figure is large.

As shown in Figs. 33 and 35, when respiration includes abdominal respiration, the track of the Lissajous figure is of a bent shape. The Lissajous figure shown in Fig. 33 indicates a case in which 50% of a single respiration is costal and 50% of the single respiration is abdominal. In this case, the track of the Lissajous figure is of a boomerang shape (an L-shape) that is symmetric with respect to a horizontal line. However, if the percentage of abdominal respiration is less than that of costal respiration, the upper upward-sloping portion of the track of the Lissajous figure corresponding to costal respiration is larger than that in Fig. 33, whereas the lower downward-sloping portion of the track of the Lissajous figure corresponding to abdominal respiration is smaller than that in Fig. 33. If the percentage of abdominal respiration is greater than that of costal respiration, the upper upward-sloping portion of the track of the Lissajous figure corresponding to costal respiration is smaller than that in Fig. 33, whereas the lower downward-sloping portion of the track of the Lissajous figure corresponding to abdominal respiration is larger than that in Fig. 33. Thus, depending on the percentage of abdominal respiration, the track of the Lissajous figure describes various tracks.

Theoretically, when abdominal respiration occupies 100% of respiration, the track of the Lissajous figure is of an inclined straight shape in which the inclination is opposite to that in costal respiration (downward sloping in the coordinate system in Fig. 33). However, respiration of human beings must include costal respiration except for persons for whom the diaphragms do not work at all due to a disorder (e.g., a disease). This can be confirmed by observing that even if a human stops breathing, when the human expands and contracts the abdomen, the diaphragm moves up and down so as to expand and contract the lungs. Accordingly, even if the human subject performs abdominal respiration as much as possible, the track of the Lissajous figure is of a bent shape having a downward-sloping straight portion corresponding to costal respiration.

The bend angle AG formed between the downward-sloping straight portion (approximate straight line LN1) corresponding to costal respiration and the upward-sloping straight portion (approximate straight line LN2) corresponding to abdominal respiration shown in Fig. 33 is small when abdominal respiration is shallow. When abdominal respiration is deep, the bend angle AG is large. In addition, the shallower the abdominal respiration, the smaller the track of the Lissajous figure.

Thus, the track of the Lissajous figure varies depending on whether or not respiration is costal or abdominal. The size and the shape of the track of the Lissajous figure vary depending on the magnitude (depth) of each of the magnitude (depth) of each of costal respiration and abdominal respiration. By observing the Lissajous figure, the human subject or another person can understand whether current respiration of the human subject is costal or abdominal, or can understand whether respiration of the human subject is mainly dependent on costal respiration or abdominal respiration. The human subject or another person can also understand the magnitude of each of costal respiration and abdominal respiration by the Lissajous figure. Accordingly, the body condition determination apparatus 1 can be used as a breathing training apparatus.

When the human subject trains for costal breathing, the human subject may pay attention to the Lissajous figure so that the track of the Lissajous figure becomes a straight shape rising from bottom left to top right and the size of the track becomes large. When the human subject trains for abdominal breathing, the human subject may pay attention to the Lissajous figure so that the track of the Lissajous figure is of a bent shape, and the size and the bend angle AG become large. Thus, by observing the Lissajous figure and confirming the type and the magnitude of breathing at any time, the human subject can train for appropriate costal or abdominal breathing.

When respiration of the human subject is draw-in respiration, as described above with reference to Fig. 30 in conjunction with the second embodiment, both the first bioelectrical impedance $Z_a$ and the second bioelectrical impedance $Z_b$ increase at inhalations, whereas both the first bioelectrical impedance $Z_a$ and the second bioelectrical impedance $Z_b$ decrease at exhalations. The manner of change is the same as that in costal respiration since human beings expand and contract the thoracic cage in both costal respiration and draw-in respiration. However, in draw-in respiration, the abdomen is held in a constricted position continually so as to be stressed continually. Therefore, the standard level of the second bioelectrical impedance $Z_b$ at the middle body trunk in draw-in respiration is higher than that in costal respiration as shown in Fig. 30. For this reason, as in Fig. 45 which shows Lissajous figures in draw-in respiration and costal respiration, both tracks of the Lissajous figures are of a straight shape rising from bottom left to top right, but locations of the two tracks are different in the axis indicating the second bioelectrical impedance $Z_b$ (the X axis in Fig. 45).

Thus, by observing the Lissajous figure, the human subject or another person can understand whether or not respiration of the human subject is draw-in respiration. The shallower the draw-in respiration, the smaller the track of the Lissajous figure, so that the magnitude of draw-in respiration can be understood from the Lissajous figure. By observing the Lissajous figure and confirming the type and the magnitude of respiration at any time, the human subject can train for appropriate draw-in breathing.

As has been described above, by virtue of displaying the Lissajous figure, the human subject or another person can understand the type and magnitude of respiration of the human subject, and can understand whether or not the human subject is performing appropriately the target type of breathing. Accordingly, the human subject can train for breathing effectively.

By an effective training for breathing, respiratory muscles (for example, the transverse abdominal muscle, the diaphragm, the internal and external intercostal muscles, the sternomastoid muscle, the anterior scalene muscle, the middle scalene muscle, the posterior scalene muscle, the abdominal rectus muscle, the internal and external abdominal oblique muscles, etc.) can be toned up effectively. In particular, the transverse abdominal muscle is a body trunk muscle that significantly influences not only respiration, but also motion functions. By draw-in respiration, not only respiratory muscles, but also inner muscles at the body trunk (e.g., the erector muscle of the spine) can be strengthened effectively. For this reason, training for breathing enhances not only respiratory functions, but also motion functions, and is effective for improvement or prevention of backache and for dieting.

In addition, training for breathing improves mental health. For example, deep breathing (deep abdominal respiration) or respiration in which time of exhalation is longer than that of inhalation improves parasympathetic action and promotes relaxation.

The body condition determination apparatus 1 of this embodiment determines the first bioelectrical impedance $Z_a$ and the second bioelectrical impedance $Z_b$, and displays a Lissajous figure indicating change over time of each of the impedances. Accordingly, among the respiration analysis process (Fig. 14) described in conjunction with the first embodiment, it is not necessary to execute the first centering process (step S50), the first difference calculating process (step S60), the second difference calculating process (step S70), and the $\Delta R_{ib}/\Delta A_b$ assumption process (step S80). The entire process in the body condition determination apparatus 1 can thus be simplified. However, it is possible to execute these steps even in this embodiment.

Training for breathing includes, for example, rehabilitation of respiratory function for patients with respiratory disease or for human subjects with exacerbated respiratory function, training of athletes for improving motion function, and training of physically unimpaired persons for strengthening respiratory functions or mental health and for improving respiratory functions that are deteriorated by smoking, lifestyle, lack of activity, or aging.

In the Lissajous figures in Figs. 32 through 35 and 37, the X axis is the second bioelectrical impedance $Z_b$, whereas the Y axis is the first bioelectrical impedance $Z_a$, but the X axis and the Y axis may be replaced with each other. For example, Fig. 38 is a Lissajous figure for abdominal respiration corresponding to Fig. 36, in which the X axis is the first bioelectrical impedance $Z_a$, whereas the Y axis is the second bioelectrical impedance $Z_b$. Even if the X axis and the Y axis are replaced, the track of the Lissajous figure is of a bent shape. In a Lissajous figure in which the X axis and the Y axis are thus replaced (e.g., Fig. 39 corresponding to Fig. 37), when respiration of the human subject is changing from costal to abdominal, the track of the Lissajous figure is changing from a straight shape rising from bottom left to top right to a bent shape of which the left portion is upward-sloping. In addition, the X axis and the Y axis of a Lissajous figure may be inclined at 45 degrees as shown in Fig. 50. In summary, the Lissajous figure may be represented in any type of orthogonal coordinate system having two orthogonal coordinate axes in which one axis is the first bioelectrical impedance $Z_a$ and the other axis is the second bioelectrical impedance $Z_b$.

3.2. DISPLAYING LISSAJOUS FIGURES FOR THE RIGHT LUNG AND THE LEFT LUNG

In order to recognize the difference between the respiration capabilities of the right lung and the left lung, the body condition determination apparatus 1 may display the Lissajous figures for the right lung and the left lung. In order to generate the display data for the Lissajous figure for the right lung, instead of the first bioelectrical impedance $Z_a$, the bioelectrical impedance at the right upper body trunk including the upper lobe of the right lung of the human subject and excluding the abdomen of the human subject may be used. As the bioelectrical impedance at the right upper body trunk, bioelectrical impedance at the right upper extremity and the upper body trunk determined in the manner shown in part (D) of Fig. 4 may be used. In order to generate the display data for the Lissajous figure for the left lung, instead of the first bioelectrical impedance $Z_a$, the bioelectrical impedance at the left upper body trunk including the upper lobe of the left lung of the human subject and excluding the abdomen of the human subject may be used. As the bioelectrical impedance at the left upper body trunk, bioelectrical impedance at the left upper extremity and the upper body trunk determined in the manner shown in part (E) of Fig. 4 may be used. Consequently, in order to generate the display data for two Lissajous figures for both of the right lung and the left lung, it is preferable to determine the bioelectrical impedance at the right upper body trunk, the bioelectrical impedance at the left upper body trunk, and the bioelectrical impedance at the middle body trunk.

In the specification, the bioelectrical impedance at the right upper body trunk will be referred to as the right first bioelectrical impedance $Z_{aR}$, whereas the bioelectrical impedance at the left upper body trunk will be referred to as the left first bioelectrical impedance $Z_{aL}$. The bioelectrical impedance at the middle body trunk will be referred to as the second bioelectrical impedance $Z_b$ as has been described above.

For displaying two Lissajous figures for the right lung and the left lung, the CPU 170 (bioelectrical impedance determiner) controls switching selection of the current electrodes X1 through X4 and the voltage electrodes Y1 through Y4, and determines the right first bioelectrical impedance $Z_{aR}$, the left first bioelectrical impedance $Z_{aL}$, and the second bioelectrical impedance $Z_b$ at steps S802 and S803 of the Lissajous figure displaying process (Fig. 36). For example, for determining the right first bioelectrical impedance $Z_{aR}$, the CPU 170 causes the electrode switching circuit 252 to select the left-hand current electrode X3 and the right-hand current electrode X4, and causes the electrode switching circuit 251 to select the right-foot voltage electrode Y2 and the right-hand voltage electrode Y4. Then, the CPU 170 determines the right first bioelectrical impedance $Z_{aR}$ at the right upper body trunk on the basis of the current data $D_i$ indicating the reference current $I_{ref}$ flowing between the right and left hands and the voltage data $D_v$ indicating the potential difference between the right-foot voltage electrode Y2 and the right-hand voltage electrode Y4. For determining the left first bioelectrical impedance $Z_{aL}$, the CPU 170 causes the electrode switching circuit 252 to select the left-hand current electrode X3 and the right-hand current electrode X4, and causes electrode switching circuit 251 to select the left-foot voltage electrode Y1 and the left-hand voltage electrode Y3. Then, the CPU 170 determines the left first bioelectrical impedance $Z_{aL}$ at the left upper body trunk on the basis of the current data $D_i$ indicating the reference current $I_{ref}$ flowing between the right and left hands and the voltage data $D_v$ indicating the potential difference between the left-foot voltage electrode Y1 and the left-hand voltage electrode Y3.

Next, the CPU 170 executes a smoothing process for measurement values the right first bioelectrical impedance $Z_{aR}$, for measurement values of the left first bioelectrical impedance $Z_{aL}$, and for measurement values of the second bioelectrical impedance $Z_b$ at step S804. At step S805, the CPU 170 generates display data for displaying a Lissajous figure for the right lung, in which, for example, the X axis is the second bioelectrical impedance $Z_b$, whereas the Y axis is the right first bioelectrical impedance $Z_{aR}$, and is for displaying another Lissajous figure for the right lung, in which the X axis is the second bioelectrical impedance $Z_b$, whereas the Y axis is the left first bioelectrical impedance $Z_{aL}$. Then, at step S806, the CPU 170 supplies the display data to the display device 160 for causing the display device 160 to show the two Lissajous figures for the right lung and the left lung.

In this case, since two Lissajous figures for the right lung and the left lung are displayed, the type and the magnitude of respiration with respect to the right lung and the left lung can be understood. By comparing two Lissajous figures, it is possible to easily understand the difference between the respiration capabilities of the right lung and the left lung. In addition, it is possible to train for breathing of the right lung and the left lung, respectively. There is no significant difference between the respiration capabilities of the right lung and the left lung of a physically unimpaired person. However, if one of the right lung and the left lung has been diseased, there is a significant difference between the respiration capabilities of the right lung and the left lung. If one of the right lung and the left lung was previously diseased, there may be a difference between the respiration capabilities of the right lung and the left lung. A method for improving the respiration capability of only the left lung is one in which the human subject repeats respiration while a load is applied to the left lung by positioning the left arm behind the right shoulder and pushing the left elbow backward with the right hand. This method is suitable for, for example, a person whose respiration capability of the left lung is lower than the respiration capability of the right lung.

Two Lissajous figures for the right lung and the left lung may be arranged next to each other on the display device 160. However, in order to facilitate understanding of the differences between the respiration capabilities of the right lung and the left lung, it is preferable to overlay the Lissajous figures one on the other as shown in Fig. 40. The CPU 170 may cause the Lissajous figures for the right lung and the left lung to be overlaid one on the other on a single screen.

When two Lissajous figures for the right lung and the left lung are overlaid, in order to distinguish the Lissajous figures, it is preferable that the manner for displaying the Lissajous figure for the right lung be different from that for the left lung. For example, the CPU 170 may indicate that the color of the Lissajous figure for the right lung is blue and the color of the Lissajous figure for the left lung is red. Alternatively, the CPU 170 may allocate different line thicknesses or line types (e.g., solid line or dotted line) to two Lissajous figures for the right lung and the left lung. Even if two Lissajous figures for the right lung and the left lung may be arranged next to each other, it is possible that the manner for displaying the Lissajous figure for the right lung will be different from that for the left lung.

In addition, as shown in Fig. 41, the difference between two Lissajous figures for the right lung and the left lung may be highlighted. At each sampling time, the CPU 170 compares the coordinates $(X_R, Y_R)$ of the instantaneous dot of the Lissajous figure for the right lung, in which the x-coordinate indicates the instantaneous measurement value of the second bioelectrical impedance $Z_b$ and the y-coordinate indicates the instantaneous measurement value of the right first bioelectrical impedance $Z_{aR}$ with the coordinates $(X_L, Y_L)$ of the instantaneous dot of the Lissajous figure for the left lung, in which the x-coordinate indicates the instantaneous measurement value of the second bioelectrical impedance $Z_b$ and the y-coordinate indicates the instantaneous measurement value of the left first bioelectrical impedance $Z_{aL}$. If the dot positions represented by the coordinates $(X_R, Y_R)$ and the coordinates $(X_L, Y_L)$ are different, the CPU 170 generates additional display data representing a bar (straight line) connecting the positions. The difference between two Lissajous figures for the right lung and the left lung can be highlighted by the bars, so that it is possible to recognize the difference between the respiration capabilities of the right lung and the left lung.

The CPU 170 may allocate different colors to the bars at exhalation and the bars at inhalation. As shown in Fig. 20, the first bioelectrical impedance $Z_a$ is higher than the first centering value $Z_{a0}$ at inhalations, and is lower than that at exhalations. Accordingly, when the average of the first centering value $Z_{a0}$ at the last respiration is as indicated by the horizontal straight line (two-dot chain line) in Fig. 41, it is possible to use different colors for above and below the straight line. However, in order to allocate different colors to the bars at exhalation and the bars at inhalation, the first centering process described in conjunction with the first embodiment should be executed for the right first bioelectrical impedance $Z_{aR}$. Alternatively, instead of the right first bioelectrical impedance $Z_{aR}$, the second centering process described in conjunction with the first embodiment should be executed for the left first bioelectrical impedance $Z_{aL}$.

The CPU 170 may also allocate different colors to the bars on the basis of whether x-coordinate $X_R$ minus x-coordinate $X_L$ is positive or negative at each sampling time. The CPU 170 may also allocate different colors to the bars on the basis of whether y-coordinate $Y_R$ minus y-coordinate $Y_L$ is positive or negative at each sampling time.

The CPU 170 may change the tone of the color of the bars depending on the distance between the position $(X_R, Y_R)$ and the position $(X_L, Y_L)$. For example, if the distance is greater, a deeper color may be used.

Instead of displaying the bars, the area defined between the two Lissajous figures may be painted by a faint color.

The CPU 170 may calculate the differential area between two Lissajous figures for the right lung and the left lung. The differential area indicates the difference between the respiration capabilities of the right lung and the left lung. On the basis of the magnitude of the differential area, the CPU 170 may classify the difference between the respiration capabilities into multiple rankings. The CPU 170 may use the sum of the lengths of the bars indicating the difference instead of the differential area for classifying the difference between the respiration capabilities into multiple rankings.

As shown in Fig. 42, the CPU 170 may calculate the coordinates of the median position between the position $(X_R, Y_R)$ and the position $(X_L, Y_L)$, and may plot the dot of the median position at each sampling time, thereby displaying the median track of two Lissajous figures for the right lung and the left lung.

The CPU 170 may highlight the difference between two Lissajous figures by indicating the different part in the Lissajous figure for the right lung in a thick line and by indicating the different part in the Lissajous figure for the left lung in a thick line.

The X axis and the Y axis may be replaced with each other in the Lissajous figures for the right lung and the left lung. In summary, the Lissajous figure may be represented in any type of orthogonal coordinate system having two orthogonal coordinate axes in which one axis is the right first bioelectrical impedance $Z_{aR}$ or the left first bioelectrical impedance $Z_{aL}$ and the other axis is the second bioelectrical impedance $Z_b$.

Although Fig. 40 through Fig. 42 show Lissajous figures representing abdominal respiration, Lissajous figures may be displayed for representing costal respiration or draw-in respiration.

Although two Lissajous figures are displayed in this embodiment, either one of the two Lissajous figures for the right lung and the left lung may be displayed. For example, if the human subject would like to train for breathing of the right lung, the human subject or another person can manipulate the human interface 150 in order to instruct displaying only the Lissajous figure for the right lung. In this case, the CPU 170 may determine only the right first bioelectrical impedance $Z_{aR}$ and the second bioelectrical impedance $Z_b$ in the Lissajous figure displaying process, and generate the display data for the Lissajous figure for the right lung to cause the display device 160 to show only the Lissajous figure for the right lung.

### 3.3. CENTERING THE LOCATION OF THE LISSAJOUS FIGURE

The displayed location of a Lissajous figure can be centered using the first centering value $Z_{a0}$ and the second centering value $Z_{b0}$ that are described in conjunction with the first embodiment. Description will be made taking as an example a Lissajous figure in which the X axis is the second bioelectrical impedance $Z_b$, whereas the Y axis is the first bioelectrical impedance $Z_a$.

After completion of the smoothing process (step S804) in the Lissajous figure displaying process shown in Fig. 36, the CPU 170 executes steps S50 through S70 in the respiration analysis process (Fig. 14) described in conjunction with the first embodiment, so as to calculate the first centering value $Z_{a0}$ and the second centering value $Z_{b0}$. As is clearly understood from Fig. 20, the first centering value $Z_{a0}$ is the standard level of the standard level of the first bioelectrical impedance $Z_a$, whereas the second centering value $Z_{b0}$ is the standard level of the second bioelectrical impedance $Z_b$.

For generating the display data for displaying a Lissajous figure at step S805 of the Lissajous figure displaying process, the CPU 170 adjusts the displayed location of the Lissajous figure such that the center position C having coordinates $(Z_{b0}, Z_{a0})$ defined by the first centering value $Z_{a0}$ and the second centering value $Z_{b0}$ becomes located at the center of the screen 160A for the Lissajous figure of the display device 160, as shown in Fig. 43. Since the location of the Lissajous figure is centered with respect to the screen 160A, visualization of the Lissajous figure can be facilitated.

Since the first centering value $Z_{a0}$ and the second centering value $Z_{b0}$ is obtained by a moving average process, even if centering of the displayed location of the Lissajous figure is repeated at small intervals (i.e., at each sampling time), the displayed location of the Lissajous figure is not changed remarkably at small intervals. However, repetition of centering of the displayed location of the Lissajous figure results in increase of processing load.

Accordingly, it is possible to reduce the frequency of repetition. For example, the x-coordinate of the center position C of the Lissajous figure may be the average of the first centering values $Z_{a0}$ within the last single respiration and the y-coordinate of the center position C of the Lissajous figure may be the average of the second centering values $Z_{b0}$ within the last single respiration, so that the coordinates of the center position C of Lissajous figure are updated at each respiration (not at each sampling time). Alternatively, the x-coordinate of the center position C of the Lissajous figure may be the average of the first centering values $Z_{a0}$ within a longer window (e.g., 20 seconds) and the y-coordinate of the center position C of Lissajous figure may be the average of the second centering values $Z_{b0}$ within the window, so that the coordinates of the center position C of the Lissajous figure is updated whenever a window passes over. Accordingly, intervals for updating the displayed location of the Lissajous figure may be determined freely.

In addition to centering the displayed location of the Lissajous figure, it is possible to adjust the display range of the Lissajous figure on the screen in each of the X axis and the Y axis. For example, the CPU 170 decides the local maximum (first local maximum) and the local minimum (first local minimum) of measurement values of the first bioelectrical impedance $Z_a$ within every respiration, and decides the local maximum (second local maximum) and the local minimum (second local minimum) of measurement values of the second bioelectrical impedance $Z_b$ within every respiration.

Then, the CPU 170 adjusts the display range of the Lissajous figure on the screen in the X axis on the basis of the second local maximum and the second local minimum, and adjusts the display range of the Lissajous figure on the screen in the Y axis on the basis of the first local maximum and the first local minimum.

More specifically, the CPU 170 adjusts the size and location of the Lissajous figure on the X axis on the screen 160A, so that the displayed range of the Lissajous figure on the X axis corresponding to the difference between the second local maximum and the second local minimum is about 80 to 90% of the width of the screen 160A in the X axis, whereas both of the second local maximum and the second local minimum are displayed within the screen 160A. Similarly, the CPU 170 adjusts the size and location of the Lissajous figure in the Y axis on the screen 160A, so that the displayed range of the Lissajous figure in the Y axis corresponding to the difference between the first local maximum and the first local minimum is about 80 to 90% of the width of the screen 160A in the Y axis, whereas both of the first local maximum and the first local minimum are displayed within the screen 160A.

Thus, for example, as shown in Fig. 44, the Lissajous figure can be displayed and centered at a suitable size with respect to the screen 160A, so that visualization of the Lissajous figure can be facilitated. Intervals for updating the size and location of the Lissajous figure in the X and Y axes may be determined freely. However, since it is preferable that the entire Lissajous figure for at least a single respiration be located within the screen 160A, the CPU 170 preferably decides the first local maximum, the first local minimum, the second local maximum, and the second local minimum from measurement values within a period longer than the period of respiration.

As in Fig. 45 which shows Lissajous figures in draw-in respiration and costal respiration, both tracks of the Lissajous figures are of a straight shape rising from bottom left to top right, but locations of both tracks are different in the axis indicating the second bioelectrical impedance $Z_b$ (the X axis in Fig. 45). If centering of the displayed location of the Lissajous figure is repeated at small intervals, the Lissajous figure will always be displayed at the center of the screen 160A, and it will be difficult to understand whether respiration of the human subject is draw-in respiration or costal respiration from looking at the Lissajous figure. In order to prevent this disadvantageous effect, it is preferable to reduce the frequency of centering of the displayed location of the Lissajous figure on the X axis.

Accordingly, when the displayed location of the Lissajous figure is centered on the basis of the first centering value $Z_{a0}$ and the second centering value $Z_{b0}$, the CPU 170 may execute a second location centering process less frequently than that for a first location centering process, in which the first location centering process is for centering the Lissajous figure in the Y axis on the basis of the first centering value $Z_{a0}$, whereas the second centering process is for centering the Lissajous figure on the X axis on the basis of the second centering value $Z_{b0}$. In addition, when the displayed location of the Lissajous figure is centered on the basis of the first local maximum, the first local minimum, the second local maximum, and the second local minimum, the CPU 170 may execute a second range adjustment process less frequently than that for a first range adjustment process, in which the first range adjustment process is for adjusting the displayed range of the Lissajous figure in the Y axis on the basis of the first local maximum and the first local minimum, whereas the second range adjustment process is for adjusting the displayed range of the Lissajous figure on the X axis on the basis of the second local maximum and the second local minimum.

For example, the first location centering process or the first range adjustment process may be executed at every respiration, whereas the second location centering process or the second range adjustment process may be executed only once (for example, at an initial stage of the process). Alternatively, the first location centering process or the first range adjustment process may be executed at every respiration, whereas the second location centering process or the second range adjustment process may be executed at every 30 respirations. Alternatively, the first location centering process or the first range adjustment process may be executed at every eight seconds, whereas the second location centering process or the second range adjustment process may be executed at every five minutes.

If the second location centering process or the second range adjustment process for centering the Lissajous figure on the X axis corresponding to the second centering value $Z_{b0}$ is executed less frequently, it will be easy to understand whether respiration of the human subject is draw-in respiration or costal respiration from looking at the Lissajous figure. This is because the locations of tracks of the Lissajous figures for draw-in respiration and costal respiration will become different in the X axis for a certain period, even though the shapes of the tracks are similar. In addition, it is possible to reduce power consumption at the body condition determination apparatus 1 by reducing the frequency of the second location centering process or the second range adjustment process. Although the frequency is less, by executing the second location centering process or the second range adjustment process, the Lissajous figure can be displayed on the center of the screen 160A and at a suitable size with respect to the screen 160A.

The second bioelectrical impedance $Z_b$ at the middle body trunk is less likely to be affected by the disturbance resulting from motion of extremities, such as arms, in comparison with the first bioelectrical impedance $Z_a$ at the upper body trunk, and therefore measurement values of the second bioelectrical impedance $Z_b$ are stable. Accordingly, even if centering the Lissajous figure on the X axis corresponding to the second centering value $Z_{b0}$ is executed less frequently, there will be few problems in that the Lissajous figure is not located within the screen 160A.

When the Lissajous figure is displayed in which the X axis is the first bioelectrical impedance $Z_a$, whereas the Y axis is the second bioelectrical impedance $Z_b$, centering the Lissajous figure in the Y axis may be executed less frequently. In summary, it is preferable that centering the Lissajous figure on the axis corresponding to the second bioelectrical impedance $Z_b$ among the two orthogonal coordinate axes, be executed less frequently than that for the other axis corresponding to the first bioelectrical impedance $Z_a$.

Although Figs. 43 and 44 show Lissajous figures representing abdominal respiration, Lissajous figures may be displayed for representing costal respiration or draw-in respiration.

The first bioelectrical impedance $Z_a$ at the upper body trunk has a larger fluctuation range than that of the second bioelectrical impedance $Z_b$ at the middle body trunk. This is because the first bioelectrical impedance $Z_a$ is more likely to be affected by the disturbance resulting from motion of extremities, such as arms. If the frequency of centering the location of the Lissajous figure is reduced and such fluctuation is omitted, there will not be a significant problem. Accordingly, it is possible to execute centering the displayed location of the Lissajous figure on the axis corresponding to the first bioelectrical impedance $Z_a$ at the same frequency (e.g., at every 30 respirations or every five minutes) as that for centering the displayed location of the Lissajous figure on the axis corresponding to the second bioelectrical impedance $Z_b$. That is to say, the cycle for centering in the axis corresponding to the first bioelectrical impedance $Z_a$ may be the same as that for centering in the axis corresponding to the second bioelectrical impedance $Z_b$, and it is possible to prolong the cycle for centering to 30 respirations or five minutes.

### 3.4. TRACK DISPLAYING PROCESS

In a Lissajous figure that continually shows the status of multiple respirations as shown in Fig. 34 or 35, if the manner for displaying the track within a single respiration is the same as that for the track within another single respiration, it is difficult to identify the track for the latest single respiration. Accordingly, it is preferable to use different displaying manners for the track of the latest single respiration and the tracks for past respirations in the Lissajous figure.

For example, the CPU 170 may generate the display data for displaying a Lissajous figure in such a manner that a deep color is allocated to the track for the latest single respiration and a faint color is allocated to the tracks for past respirations, as shown in Fig. 46. Alternatively, the CPU 170 may allocate a solid line to the track for the latest single respiration and may allocate a dotted line to the tracks for past respirations. Alternatively, the CPU 170 may allocate different tones of colors to the tracks for the latest single respiration and for past respirations. For example, the CPU 170 may decide whether or not a new respiration starts on the basis of change in each of the first bioelectrical impedance $Z_a$ and the second bioelectrical impedance $Z_b$, and may allocate red to the track for the new single respiration and may change the color for track for previous respiration from red to blue.

The CPU 170 may change the displaying manner for the tracks of the Lissajous figure depending on the elapsed time. For example, the CPU 170 may lighten the color as the elapsed time increases. In this case, the newer the track, the fainter the color of the track. It is easy to identify the tracks for newer respirations (e.g., the track for the latest respiration).

The Lissajous figure may be represented in any type of orthogonal coordinate system having two orthogonal coordinate axes in which one axis is the first bioelectrical impedance $Z_a$ and the other axis is the second bioelectrical impedance $Z_b$. The X axis and the Y axis may be replaced with each other in the Lissajous figures for the right lung and the left lung. In addition, the X axis and the Y axis of a Lissajous figure may be inclined at any angle, whereas the angle formed between the X axis and the Y axis is also orthogonal.

Although Fig. 46 shows a Lissajous figure representing abdominal respiration; Lissajous figures may be displayed for representing costal respiration or draw-in respiration.

### 3.5. ASSISTANCE DISPLAY

As shown in Fig. 47, it is preferable to display a target Lissajous figure TL showing a target model of breathing to be performed by the human subject in such a manner that the target Lissajous figure TL is overlaid on an actually measured Lissajous figure ML showing the status of breathing of the human subject.

For example, the target Lissajous figure TL may be generated by processing an actually measured Lissajous figure ML of the human subject measured in past times (e.g., an actually measured Lissajous figure ML showing the status of the last respiration of the human subject). When the human subject trains for costal breathing or draw-in breathing, the CPU 170 may generate display data indicating the target Lissajous figure TL that is the actually measured Lissajous figure ML showing the status of the last respiration of the human subject enlarged at a predetermined magnification (e.g., 1.1-fold magnification). The track of actually measured Lissajous figure ML may be of a straight shape rising from bottom left to top right. When the human subject trains for abdominal breathing, the CPU 170 may generate display data indicating the target Lissajous figure TL that is the actually measured Lissajous figure ML showing the status of the last respiration of the human subject which is enlarged at a predetermined magnification or of which the bend angle AG is adjusted. The track of actually measured Lissajous figure ML may be of a bent shape.

The CPU 170 may cause the display device 160 to show the target Lissajous figure TL on the screen 160A, whereas the CPU 170 generates the data for displaying the actually measured current Lissajous figure ML on the basis of measurement values of the first bioelectrical impedance $Z_a$ and measurement values of the second bioelectrical impedance $Z_b$, and may cause the display device 160 to show the actually measured current Lissajous figure ML in such a manner that the actually measured current Lissajous figure ML is overlaid on the target Lissajous figure TL on the screen 160A. Thus, the human subject can train for breathing comparing the actually measured Lissajous figure ML showing the current status of breathing with the target Lissajous figure TL. The human subject may focus on making the track of the actually measured Lissajous figure ML coincide with the track of the target Lissajous figure TL, so as to learn the target breathing.

Instead of generating a target Lissajous figure TL by processing the actually measured Lissajous figure ML measured in past times, the CPU 170 may generate a target Lissajous figure TL that indicates a respiration having a type (costal, abdominal, or draw-in) and a magnitude (depth) to be performed by the human subject. For example, when a training menu for guiding the human subject to train for breathing indicates a step for performing a single costal respiration of which the magnitude is small and a next step for performing a single abdominal respiration of which the magnitude is in the middle, the CPU 170 may generate and display a target Lissajous figure TL corresponding to the single costal respiration of which the magnitude is small, and then may generate and display another target Lissajous figure TL corresponding to the single abdominal respiration of which the magnitude is in the middle in the interval between the last and current respirations. In addition, the CPU 170 may control the cycle for displaying the target Lissajous figure TL in order to guide the rhythm of respiration of the human subject. Thus, by the assistance display for guiding the human subject to perform breathing in which the target Lissajous figure TL is used, an effective guidance is given to the human subject as to the type, magnitude, or rhythm of breathing.

The CPU 170 may allocate different displaying manners (e.g., different colors and the different line types) to the actually measured Lissajous figure ML and the target Lissajous figure TL in order to easily distinguish the actually measured Lissajous figure ML from the target Lissajous figure TL. The CPU 170 may highlight the difference between the two Lissajous figures by showing bars between the tracks of the two Lissajous figures, as shown in Fig. 48.

The CPU 170 may calculate the differential area between the actually measured Lissajous figure ML and the target Lissajous figure TL, and on the basis of the magnitude of the differential area, the CPU 170 may classify the difference into multiple rankings. The CPU 170 may use the sum of the lengths of the bars indicating the difference instead of the differential area for classifying the difference into multiple rankings.

Although the actually measured Lissajous figure ML and the target Lissajous figure TL are overlaid in Figs. 47 and 48, it is not necessary to overlay them. The two Lissajous figures may be arranged next to each other.

The Lissajous figure may be represented in any type of orthogonal coordinate system having two orthogonal coordinate axes in which one axis is the first bioelectrical impedance $Z_a$ and the other axis is the second bioelectrical impedance $Z_b$. The X axis and the Y axis may be replaced with each other in the Lissajous figures for the right lung and the left lung. In addition, the X axis and the Y axis of a Lissajous figure may be inclined at any angle, whereas the angle formed between the X axis and the Y axis is also orthogonal.

## 3.6. DETERMINATION AS TO WHETHER LUNG VENTILATION CAPABILITY IS GOOD OR BAD

It is possible to determine whether the lung ventilation capability (respiration capability) is good or bad on the basis of the inclination angle of the track of the Lissajous figure. For example, when the human subject performs costal respiration, the CPU 170 may obtain an approximately straight line LN of the track of a Lissajous figure for a single respiration as shown in Fig. 49, in accordance with a suitable algorithm, for example, the least-square method, on the basis the x-y coordinates of the track corresponding to the measurement values of the first bioelectrical impedance $Z_a$ and the second bioelectrical impedance $Z_b$ obtained at each sampling time within the single respiration. Next, the CPU 170 (inclination angle calculator) may calculate an angle $\alpha$ formed between the approximate straight line LN and the X axis, and decides the angle $\alpha$ as an inclination angle of the track of the Lissajous figure.

As shown in Fig. 49, when the X axis is the second bioelectrical impedance $Z_b$, whereas the Y axis is the first bioelectrical impedance $Z_a$, the higher the lung ventilation capability, the greater the inclination angle $\alpha$ of the approximate straight line LN (of the track for a single respiration). In other words, when the lung ventilation capability is greater, the inclination angle $\alpha$ is nearer to 90 degrees. Therefore, the CPU 170 (ventilation capability determiner) may compare the inclination angle $\alpha$ with a predetermined reference inclination angle $\beta$, and may determine that the lung ventilation capability is good when the inclination angle $\alpha$ is equal to or greater than the reference inclination angle $\beta$. When the inclination angle $\alpha$ is less than the reference inclination angle $\beta$, the CPU 170 may determine that the lung ventilation capability is bad. The reference inclination angle $\beta$ is a threshold for facilitating the determination as to whether the lung ventilation capability is good or bad, and may be defined on the basis of experiments from which the inclination angle of the track of a Lissajous figure for a single respiration is determined for a number of human subjects. The reference inclination angle $\beta$ is stored in the first memory 120.

As has been described above, it is possible to easily determine whether the lung ventilation capability is good or bad on the basis of the inclination angle of the track of the Lissajous figure.

Depending on the posture of a human subject (standing, sitting, or supine), the inclination angle may be varied. Accordingly, multiple reference inclination angles $\beta$ may be defined depending on the posture and be stored in the first memory 120. In this case, for example, the human interface 150 may be used to input the posture of the human subject, and the reference inclination angle $\beta$ corresponding to the posture may be retrieved from the first memory 120. The reference inclination angle $\beta$ retrieved from the first memory 120 may be amended on the basis of the personal or individual body information (including the height, age, and sex) entered at step S1 (Fig. 5) or the weight measured at step S2 (Fig. 5).

In an alternative embodiment, as shown in Fig. 50, the Lissajous figure may be rotated by coordinate conversion so that the straight line inclined at the reference inclination angle $\beta$ from the X axis is oriented vertically. If the approximate straight line LN of the track of the resulting Lissajous figure is vertical or sloping from top left to bottom right, the lung ventilation capability may be determined as good. If the approximate straight line LN is sloping from bottom left to top right, the lung ventilation capability may be determined as bad.

When the human subject performs abdominal respiration, an approximate straight line LN may be obtained for a part of the track encompassed by the oval shown in Fig. 51, and then it is possible to determine whether the lung ventilation capability is good or bad in a manner similar to that in costal respiration. When the human subject performs draw-in respiration, it is possible to determine whether the lung ventilation capability is good or bad in a manner similar to that in costal respiration.

The track of a Lissajous figure showing status within two or more respirations or a half of a respiration may be used for determining whether respiration capability is good or bad, instead of the track of a Lissajous figure showing status within a single respiration.

In determination as to whether the lung ventilation capability is good or bad, the Lissajous figure may be represented in any type of orthogonal coordinate system having two orthogonal coordinate axes in which one axis is the first bioelectrical impedance $Z_a$ and the other axis is the second bioelectrical impedance $Z_b$. The X axis and the Y axis may be replaced with each other in the Lissajous figures for the right lung and the left lung. In addition, the X axis and the Y axis of a Lissajous figure may be inclined at any angle, whereas the angle formed between the X axis and the Y axis is also orthogonal.

In an alternative embodiment, a reference inclination angle $\beta$ stored in the first memory 120 may be defined freely, and it is possible to determine whether or not the lung ventilation capability of the human subject is higher than a predetermined reference capability.

## 3.7. TIME COMPRESSION DISPLAYING OF GRAPH SHOWING RESPIRATION DEPTH

The CPU 170 may calculate the respiration depth at every respiration by executing the respiration depth calculating process (Fig. 27) described in conjunction with the first embodiment. That is to say, on the basis of measurement values of the first bioelectrical impedance $Z_a$ and the second bioelectrical impedance $Z_b$, the CPU 170 may calculate the respiration depth at every respiration. The CPU 170 (graph generator) may execute the normalization process (step S601 in Fig. 28 described in conjunction with the first embodiment) for the calculated respiration depths, and may generate display data for indicating a graph (respiration depth graph) showing change over time of respiration depth for displaying it on the display device 160. For example, the respiration depth graph is as shown in Fig. 52A.

The human subject or another person may refer to the respiration depth graph in order to understand how the magnitude (depth) of respiration is changed due to training for breathing. For this purpose, it is important to know the magnitude of latest respirations. The time for breathing training may be frequently long, e.g., ten minutes or more. In order to display the entire graph from the start of measurement to the current time on the display device 160, it is preferable that the graph be compressed in the direction of the time axis. If the entire graph is uniformly compressed, the time resolution will be reduced uniformly in the graph. This results in it being difficult to recognize details of the magnitude of the latest respirations.

Accordingly, the CPU 170 generates the display data for the graph in such a manner that the graph is nonlinearly compressed in the direction of the time axis and earlier time intervals (nearer to the start of measurement) are more compressed than later time intervals, so that the time resolution for later time intervals is higher than that for earlier time intervals. For example, the CPU 170 may generate the graph as a single logarithmic chart in which the time axis is represented on a logarithmic scale, as shown in Fig. 52B, so that the time resolution for later time intervals is higher than that for earlier time intervals. Alternatively, as shown in Fig. 52C, the CPU 170 may divide the entire elapsed time (from the start of measurement to the present) into three time intervals, and the earliest interval is most compressed, whereas the latest interval is least compressed, so that time resolution for the latest time interval is higher than that for earlier time intervals. In either of the cases in Figs 52B and 52C, although the graph is compressed in the direction of the time axis, it is easy to recognize details of the magnitude of the latest respirations.

It is possible to use the teachings in the above-described third embodiment when Lissajous figures for the right lung and the left lung are displayed. More specifically, it is possible to center the displayed location of each of the Lissajous figures for the right lung and the left lung in a screen. It is possible to use the above-described track displaying process for each of the Lissajous figures for the right lung and the left lung. It is possible to use the above-described assistance display for each of the Lissajous figures for the right lung and the left lung. It is possible to determine whether the lung ventilation capability is good or bad for each of the right lung and the left lung.

In the third embodiment, the Lissajous figure is represented in such a manner that one axis is the first bioelectrical impedance $Z_a$ and the other axis is the second bioelectrical impedance $Z_b$. However, the CPU 170 may execute steps S10 through S70 in the respiration analysis process (Fig. 14) described in conjunction with the first embodiment, so as to calculate the first differences $\Delta Z_a$ and the second differences $\Delta Z_b$. Then, the CPU 170 may generate display data for displaying a Lissajous figure in an orthogonal coordinate system having two orthogonal coordinate axes in which one axis is the first difference $\Delta Z_a$ and the other axis is the second difference $\Delta Z_b$.

Teachings in this embodiment can be combined with teachings in the first or second embodiment. For example, the body condition determination apparatus 1 may display at least one Lissajous figure and may determine and report whether respiration of the human subject is costal respiration or abdominal respiration. The body condition determination apparatus 1 may display at least one Lissajous figure and may display the bar graphs BG1 and BG2 shown in Fig. 26. The body condition determination apparatus 1 may display at least one Lissajous figure and may determine and report that respiration of the human subject is costal respiration, abdominal respiration, or draw-in respiration.

### 4. FOURTH EMBODIMENT

Fig. 60 is a flow chart showing an operation of a body condition determination apparatus of a fourth embodiment according to the present invention. In the fourth embodiment, the body condition determination apparatus executes steps S1 through S4 as similar to Fig. 5 described in conjunction with the first embodiment. The CPU 170 executes a respiration-characteristic-determination-and-displaying process at every respiration of the human subject for determining a respiration characteristic (characteristic of lung ventilation function) of the human subject at the respiration and for displaying (reporting) the determination result (step S5). The body condition determination apparatus of the fourth embodiment can be used as a respiration characteristic determination apparatus, i.e., a diagnostic device than is adapted for determining respiration characteristics of the human subject.

The respiration-characteristic-determination-and-displaying process executed by the CPU 170 will be described. In the following, with reference to Fig. 61, the respiration-characteristic-determination-and-displaying process executed by the CPU 170 will be described in detail. Fig. 61 is a flow chart showing an example of the process for determining and displaying respiration characteristics.

As shown in Fig. 61, the CPU 170 first decides whether or not the respiration depth of the human subject at the last single respiration is equal to or greater than an essential respiration depth at rest (step S 1000). More specifically, in a manner similar to that in steps S601 and S602 in Fig. 28 of the above-described respiration depth displaying process in conjunction with the first embodiment, the CPU 170 calculates the normalized one-time ventilation volume $\%\Delta T_V$ corresponding to the normalized respiration depth value $\%\Delta Z_a$ at the last single respiration. Then, on the basis of the normalized one-time ventilation volume $\%\Delta T_V$, CPU 170 decides whether or not the respiration depth of the human subject is equal to or greater than the essential respiration depth at rest. If the normalized one-time ventilation volume $\%\Delta T_v$ is equal to or greater than the fourth predetermined value $\alpha_4$ (see Fig. 29), CPU 170 decides that the respiration depth of the human subject is equal to or greater than the essential respiration depth at rest.

If the decision at step S1000 is negative, the CPU 170 reports guidance information for guiding the human subject to breathe at a respiration depth that is equal to or greater than the essential respiration depth at rest (step S1001). The guidance information, i.e., guidance message, may be shown on the display device 160 or announced by speech. Alternatively, the guidance information may be shown on the display device 160 and announced by speech.

If the decision at step S1000 is affirmative, the CPU 170 decides a respiration characteristic of the human subject at the last single respiration (step S1002). The CPU 170 also decides the respiration characteristic of the human subject at the last single respiration (step S1002) after S1001. More specifically, the CPU 170 decides the respiration characteristic of the human subject at the last single respiration on the basis of measurement values of the first bioelectrical impedance $Z_a$ and measurement values of the second bioelectrical impedance $Z_b$ within the last single respiration.

For example, if the human subject has a history of chest disease and the function at the chest part that contributes to respiration (e.g., internal and external intercostal muscles) is deteriorated, the motion at the chest skeletal muscle in respiration is very small and is less than that of a physically unimpaired person. In order to ensure a sufficient ventilation volume, such a human subject will move the diaphragm remarkably, so that the displacement of the diaphragm is large. The same is true for a human subject having deteriorated function in the chest due to aging.

Change in the first bioelectrical impedance $Z_a$ at the upper body trunk including the upper lobes of the lungs of the human subject and excluding the abdomen of the human subject corresponding to a volume of air entering and leaving the lungs of the human subject. Change in the second bioelectrical impedance $Z_b$ at the middle body trunk including the median and lower lobes of the lungs of the human subject and the abdomen of the human subject corresponds to movement of the diaphragm. The greater the movement of the diaphragm, the greater the change in the second bioelectrical impedance $Z_b$.

Even if the ventilation volume of air entering and leaving the lungs of the human subject with deteriorated function at the chest part that contributes to respiration in a single respiration were the same as that of a physically unimpaired person, change in the second bioelectrical impedance $Z_b$ in a single respiration of the human subject is greater than that of the physically unimpaired person because of the greater movement of the diaphragm.

Fig. 62 is a graph showing change over time of each of the first difference $\Delta Z_a$ and the second difference $\Delta Z_b$ in respiration of a human subject. The human subject is a male who does not have a history of disease in the chest (physically unimpaired person). Fig. 63 is a graph showing change over time of each of the first difference $\Delta Z_a$ and the second difference $\Delta Z_b$ in respiration of a human subject. The human subject is a male who has a history of disease in the chest. As will be understood from Figs. 62 and 63, for the human subject with deteriorated function in the chest that contributes to respiration, the amplitude of the second difference $\Delta Z_b$ is larger.

Accordingly, in this embodiment, it is decided whether or not a respiration characteristic (the function at the chest part that contributes to respiration) of the human subject is normal on the basis of measurement values of the second bioelectrical impedance $Z_b$ and the first bioelectrical impedance $Z_a$.

As described above, change in the second bioelectrical impedance $Z_b$ during exhalations of abdominal respiration is completely different from that of the first bioelectrical impedance $Z_a$. Irrespective of whether respiration of the human subject is costal respiration or abdominal respiration, change in the second bioelectrical impedance $Z_b$ during inhalations is similar to change in the first bioelectrical impedance $Z_a$. Accordingly, the waveform of change in second bioelectrical impedance $Z_b$ includes distortion resulting from exhalations in abdominal respiration. Thus, it is preferable to determine whether or not the function at the chest part that contributes to respiration of the human subject is normal on the basis of change in each of the first bioelectrical impedance $Z_a$ and the second bioelectrical impedance $Z_b$ at inhalations.

Accordingly, in this embodiment, it is determined whether or not the function at the chest part that contributes to respiration of the human subject is normal on the basis of change in each of the first bioelectrical impedance $Z_a$ and the second bioelectrical impedance $Z_b$ at inhalations. At step S1003, the CPU 170 selects a peak value $\Delta Z_a(MAX)$ among the first differences $\Delta Z_a$ at the inhalation of the last single respiration of the human subject, and selects a peak value $\Delta Z_b(MAX)$ among the second differences $\Delta Z_b$ at the inhalation of the last single respiration. Next, the CPU 170 calculates the ratio of the peak value $\Delta Z_b(MAX)$ to the peak value $\Delta Z_a(MAX)$, and decides the ratio $\Delta Z_b(MAX)/\Delta Z_a(MAX)$ as a costal-abdominal ventilation balance value BP. If the costal-abdominal ventilation balance value BP is equal to or greater than a predetermined value, the CPU 170 decides that the function at the chest part that contributes to respiration of the human subject is abnormal. If the costal-abdominal ventilation balance value BP is less than the predetermined value, the CPU 170 decides that the function at the chest part that contributes to respiration of the human subject is normal.

Usually, females perform respiration mainly dependent on costal respiration, whereas males perform respiration mainly dependent on abdominal respiration. Even if the ventilation volume of a male were the same as that of a female, the costal-abdominal ventilation balance value BP of the male would be greater than that of the female because of the larger movement of the diaphragm. In addition, for human beings with large visceral fat, the range of movement of the abdominal skeletal muscle is limited. Although females have a compensatory ability to ensure respiration capability when the range of movement of the abdominal skeletal muscle is limited due to pregnancy or obesity, males do not have such a compensatory ability. Therefore, males with large visceral fat have a low closing volume, and have a high costal-abdominal ventilation balance value BP. Thus, from the above-described costal-abdominal ventilation balance value BP, a respiration characteristic of the human subject can be decided.

As shown in Fig. 61, after step S1002, the CPU 170 causes the display device 160 to display the decision result (step 51003). In this embodiment, the decision result of step S1002 is displayed as a third bar graph BG4 shown in Fig. 64. As shown in Fig. 64, the costal-abdominal ventilation balance value BP depicted by the third bar graph BG4 is classified into five degrees corresponding to five sections. According to the costal-abdominal ventilation balance value BP calculated at step S1002, one of the five sections in the third bar graph BG4 is colored. Specifically, if costal-abdominal ventilation balance value BP is from zero to 0.4, the CPU 170 causes the display device 160 to color the uppermost section. If costal-abdominal ventilation balance value BP is from 0.4 to 0.8, the CPU 170 causes the display device 160 to color the second top section. If costal-abdominal ventilation balance value BP is from 0.8 to 1.2, the CPU 170 causes the display device 160 to color the middle section. If costal-abdominal ventilation balance value BP is from 1.2 to 1.6, the CPU 170 causes the display device 160 to color the second bottom section. If costal-abdominal ventilation balance value BP is greater than 1.6, the CPU 170 causes the display device 160 to color the lowermost section. For example, if the costal-abdominal ventilation balance value BP is 0.5, and the second bottom section in the third bar graph BG4 is colored as shown in Fig. 64.

For example, if respiration of the human subject is mainly dependent on costal respiration (normal female respiration), the uppermost section in the third bar graph BG4 will be colored. If respiration of the human subject is mainly dependent on abdominal respiration (normal male respiration), the second top section in the third bar graph BG4 will be colored.

If the respiration function of the human subject is deteriorated (function at the chest part that contributes to respiration is abnormal or the visceral fat is large), one of the middle to the lowermost sections of the third bar graph BG4 will be colored. By observing the third bar graph BG4, the human subject or another person can accurately and easily understand the respiration characteristic (e.g., deterioration of function at the chest part that contributes to respiration) of the human subject. As has been described above, according to this embodiment, a respiration characteristic of the human subject can be identified accurately and easily.

5. VARIATIONS

The present invention is not limited to the above-described embodiments. For example, variations described below may be made without departing from the scope of the present invention. Any combination of the variations below may also be made without departing from the scope of the present invention.

## 5.1. FIRST VARIATION

The CPU 170 may execute an assistance report for guiding the human subject to perform appropriate breathing. For example, the CPU 1 70 may cause the display device 160 to show and change a third bar graph BG3 for instructing appropriate rhythm and pattern of inhalations and exhalations and appropriate respiration depth. As shown in Fig. 53, the total number of degrees capable of being depicted by the third bar graph BG3 is six, in which three degrees are allocated to the inhalation side, whereas three degrees are allocated to the exhalation side. The greater the number of degree indicators colored, the greater the respiration depth to be performed. The number of colored degree indicators will be referred to as a "colored-degree-indicator number".

If the colored-degree-indicator number at the inhalation side is one (only one degree indicator at the inhalation side is colored), the third bar graph BG3 instructs the human subject to perform a small-depth inhalation. If the colored-degree-indicator number at the inhalation side is two (two degree indicators at the inhalation side are colored), the third bar graph BG3 instructs the human subject to perform a middle-depth inhalation. If the colored-degree-indicator number at the inhalation side is three (all degree indicators at the inhalation side are colored), the third bar graph BG3 instructs the human subject to perform a large-depth inhalation.

If the colored-degree-indicator number at the exhalation side is one (only one degree indicator at the exhalation side is colored), the third bar graph BG3 instructs the human subject to perform a small-depth exhalation. If the colored-degree-indicator number at the exhalation side is two (two degree indicators at the exhalation side are colored), the third bar graph BG3 instructs the human subject to perform a middle-depth exhalation. If the colored-degree-indicator number at the exhalation side is three (all degree indicators at the exhalation side are colored), the third bar graph BG3 instructs the human subject to perform a large-depth exhalation.

Let us assume that the body condition determination apparatus instructs the human subject to perform middle-depth inhalations twice and then to perform middle-depth exhalations twice. In this case, the indication on the third bar graph BG3 is changed as shown in Fig. 54. When a single breathing takes two seconds, the change in the indication is meant to guide two middle-depth exhalations and two middle-depth inhalation in four seconds. This is only an example, and various patterns of change may be used for instructing appropriate rhythm and pattern of inhalations and exhalations and appropriate respiration depth. For example, it is possible to report assistance information for guiding the human subject to perform abdominal breathing. Instead of, or in addition to, displaying the third bar graph BG3, assistance information may be announced by speech.

By the above-described assistance report, the human subject is guided to perform appropriate breathing paying attention to the rhythm, pattern, and depth. In addition, the human subject can confirm the current status of breathing of the human subject by observing the aforementioned first bar graph BG1 and the second bar graph BG2. In other words, the indication on the first bar graph BG1 and the second bar graph BG2 may be used for biofeedback information for training for breathing.

## 5.2. SECOND VARIATION

As shown in Fig. 55, the CPU 170 may cause the display device 160 to display a schematic view for showing the magnitude of respiration. Fig. 55 includes four schematic views. In the schematic views, the greater the magnitude of respiration, the greater the area colored in the illustration of the lungs. Such a schematic view can be displayed as a measurement result or as guidance information for guiding the human subject to perform breathing.

Alternatively, the type and magnitude of respiration can be reported by an animation that shows a human model or an abstract animal model. When respiration of the human subject is small-depth costal respiration, an animation is displayed in which the model's chest repeats contraction and expansion in a small range. When respiration of the human subject is large-depth costal respiration, another animation is displayed in which the model's chest repeats contraction and expansion in a large range. When respiration of the human subject is small-depth abdominal respiration, an animation is displayed in which the model's abdomen repeats contraction and expansion in a small range. When respiration of the human subject is large-depth abdominal respiration, another animation is displayed in which the model's abdomen repeats contraction and expansion in a large range. When respiration of the human subject is small-depth draw-in respiration, an animation is displayed in which the model's chest repeats contraction and expansion in a small range, whereas the model's abdomen is held in a constricted position. When respiration of the human subject is large-depth draw-in respiration, another animation is displayed in which the model's chest repeats contraction and expansion in a large range whereas the model's abdomen is held in a constricted position. Such an animation can be displayed as a measurement result or as guidance information for guiding the human subject to perform breathing.

Thus, measurement results or guidance information can be reported in an easily understandable manner.

## 5.3. THIRD VARIATION

In the above-described embodiments, four current electrodes and four voltage electrodes are arranged at both hands and both feet in accordance with the limb-lead eight-electrode method. The present invention is not limited to the embodiment. For example, the bioelectrical impedance at the upper body trunk can be measured with the use of a combination of the limb-lead method and ear electrodes. By using ear electrodes, the bioelectrical impedance at the upper body trunk is influenced by only one upper extremity rather than both upper extremities. If ear electrodes are incorporated in earphones or headphones, further advantages are obtained since sound information and relaxing effects are given to the human subject.

In the above-described embodiments, impedances are determined when the human subject is standing. However, impedances may be determined when the human subject is at a sitting or a relaxed position on a sofa, a seat, e.g., a lavatory seat, or a chair, e.g., a massage chair, with electrodes being arranged at parts of the seat, armrests, footrests, or a combination thereof

Furthermore, impedances are determined when the human subject is bathing with electrodes being arranged at armrests and the bottom surface of the bathtub at which the buttocks and soles of the human subject are in contact. The body trunk includes physiological saline and is electrically more conductive than the hot water in a bathtub. Accordingly, when the human subject is relaxed while bathing, impedances are determined and breathing training may be performed.

The body condition determination apparatus 1 of the above-described embodiments may include a blood-pressure meter with a cuff. Change of the status of breathing or the tension on the arms is determined on the basis of the determined impedances, and obtained information may be used for correcting or compensating the measured blood pressure.

It is preferable to relax the human subject when the analysis of respiration or training for breathing is executed. Accordingly, it is desirable to display a picture of peaceful scene, to turn on relaxing music or sounds of birds or a waterfall, and to adjust the temperature and humidity when the analysis of respiration or training for breathing is executed.

It is also preferable to display a video about training for appropriate breathing in order to enhance the efficiency of training.

## 5.4 FOURTH VARIATION

In the above-described embodiments, the CPU 170 calculates the bioelectrical impedance at the right upper extremity and the upper body trunk on the basis of the current data $D_i$ indicating the reference current $I_{ref}$ flowing between the right and left hands and the voltage data $D_v$ indicating the potential difference between the right-foot voltage electrode Y2 and the right-hand voltage electrode Y4. However, other schemes may be used to determine the first bioelectrical impedance $Z_a$ at the upper body trunk including the upper lobes of the lungs of the human subject and excluding the abdomen of the human subject. For example, on the basis of the current data $D_i$ indicating the reference current $I_{ref}$ flowing between the right and left hands and the voltage data $D_v$ indicating the potential difference between the left-foot voltage electrode Y1 and the left-hand voltage electrode Y3, the bioelectrical impedance at the left upper extremity and the upper body trunk may be calculated, and the resulting impedance may be used as the first bioelectrical impedance $Z_a$.

In the above-described embodiments, the CPU 170 calculates the second bioelectrical impedance $Z_b$ at the middle body trunk on the basis of the current data $D_i$ indicating the reference current $I_{ref}$ flowing between the left foot and the right hand and the voltage data $D_v$ indicating the potential difference between left-hand voltage electrode Y3 and the right-foot voltage electrode Y2. However, other schemes may be used to determine the second bioelectrical impedance $Z_b$ at the middle body trunk including the median and lower lobes of the lungs of the human subject and the abdomen of the human subject. For example, on the basis of the current data $D_i$ indicating the reference current $I_{ref}$ flowing between the right foot and the left hand and the voltage data $D_v$ indicating the potential difference between the right-hand voltage electrode Y4 and the left-foot voltage electrode Y1 the bioelectrical impedance at the middle body trunk may be calculated, and the resulting impedance may be used as the second bioelectrical impedance $Z_b$.

Without use of the limb-lead eight-electrode method, current electrodes and voltage electrodes may be adhered directly to the body trunk of the human subject so as to determine the bioelectrical impedance at the upper body trunk of the human subject including the lungs and excluding the abdomen, the bioelectrical impedance at the right upper body trunk of the human subject including the right lung and excluding the abdomen, the bioelectrical impedance at the left upper body trunk of the human subject including the left lung and excluding the abdomen, or the bioelectrical impedance at the middle body trunk of the human subject including the abdomen.

When bioelectrical impedance $Z_{aL}$, and $Z_b$ are used in order to display two Lissajous figures for the right lung and the left lung, the CPU 170 may use the right first bioelectrical impedance $Z_{aR}$ or the left first bioelectrical impedance $Z_{aL}$ as the first bioelectrical impedance $Z_a$ at the upper body trunk, and may decide whether the type of respiration of the right lung or the left lung of the human subject. Similarly, the CPU 170 may use the right first bioelectrical impedance

$Z_{aR}$ or the left first bioelectrical impedance $Z_{aL}$ as the first bioelectrical impedance $Z_a$ at the upper body trunk, and may execute the respiration depth calculating process and the respiration depth displaying process (first embodiment), so as to display the first bar graph BG1 and the second bar graph BG2 for the right lung or the left lung.

## 5.5 FIFTH VARIATION

In the above-described respiration depth displaying process, the CPU 170 calculates the normalized respiration depth value $\%\Delta Z_a$ as the respiration depth $\Delta Z_{ap-p}$ at the last respiration divided by the second centering value $Z_{b0}$ at the last respiration multiplied by 100. The calculation is expressed as:

$$\%\Delta Z_a = (\Delta Z_{ap-p}/Z_{b0}) * 100$$

However, other schemes may be used for normalizing the respiration depth $\Delta Z_{ap-p}$. In summary, any type of normalization scheme may be used for adjusting the respiration depth $\Delta Z_{ap-p}$ in order to exclude individual variation of physical constitutions of human subjects. For example, when an index indicative of the skeletal muscle mass (degree of development of skeletal muscle) is MV, an index indicative of the height of the human subject and the length of part in which the bioelectrical impedance is measured is H, bioelectrical impedance at a part which is important for development of the skeletal muscle is $Z_x$, MV is proportional to $H^2/Z_x$. Instead of the second centering value $Z_{b0}$, using $H^2/Z_x$, the respiration depth $\Delta Z_{ap-p}$ may be normalized. In order to assume the index MV, a multiple linear regression analysis may be used, and sex, age, and weight may be used as variables in the multiple linear regression analysis in order to enhance accuracy.

## 5.6. SIXTH VARIATION

The present invention may be applied in a system including a game machine. Fig. 56 is an overall view showing a body condition determination system 5 including a household game machine 300. As shown in Fig. 56, the body condition determination system 5 includes a biological information input apparatus 200', a game machine 300 (respiration characteristic analysis apparatus), a controller 350, and a monitor 400. The game machine 300 includes a disk slot into which an optical disk 500 is inserted.

The biological information input apparatus 200' includes a platform 20' on which the human subject stands, a left-hand left electrode handle 30L, and a right-hand right electrode handle 30R. The biological information input apparatus 200' basically has the same structure as that of the bioelectrical impedance determination part 200 shown in Fig. 1, and additionally includes a weighing scale. The biological information input apparatus 200' is connected with the controller 350 via a communication cable, and biological information measured at the biological information input apparatus 200', e.g., bioelectrical impedances at various parts of the human body and the human body weight, is supplied from the biological information input apparatus 200' via the controller 350 and the communication cable to the game machine 300.

The controller 350 is a human interface. The human subject or another person may manipulate the controller 350 in order to input various instructions and personal information on the human subject, for example, the height, age, and sex into the controller 350. The controller 350 is communicable with the game machine 300 by radiowaves, e.g., Bluetooth (registered trademark), and transmits to the game machine 300 instructions input to the controller 350 and the biological information supplied from the biological information input apparatus 200'.

The monitor 400 may be, for example, a television receiver connected with the game machine 300 via a communication cable.

The optical disk 500 stores a program and data for executing various processes that have been described in conjunction with the above-described first through third embodiments and the first through fifth variations.

In this embodiment, biological information measured at the biological information input apparatus 200' is supplied via the controller 350 to the game machine 300. However, the biological information input apparatus 200' may supply the biological information to the game machine 300 by radiowaves. In this case, the biological information input apparatus 200' may include a radio module for radio communication with the game machine 300. The biological information input apparatus 200' may supply the biological information to the game machine 300 by cable. In this case, the biological information input apparatus 200' may be connected with the game machine 300 via a communication cable. Instead of the left-hand left electrode handle 30L, the current electrode X3 and the voltage electrodes Y3 may be provided in a left-hand controller having at least part of the function of the controller 350. Instead of the right-hand right electrode handle 30R, the current electrode X4 and the voltage electrodes Y4 may be provided in a right-hand controller having at least part of the function of the controller 350.

Fig. 57 is a block diagram showing a structure of the game machine 300. As shown in Fig. 57, the game machine 300 includes a ROM 301, a RAM 302, a hard disk 303, a disk drive 310, a radio communication module 320, an image processor 330, a sound processor 340, and a CPU 360. The ROM 301 stores a program or data for controlling the whole game machine 300. The RAM 302 is used as a work area for the CPU 360.

The hard disk 303 stores a program or data read from the optical disk 500. Such data include data describing a training menu management table TBL that will be described with reference to Fig. 58. The disk drive 310 reads the program or data from the optical disk 500. The program or data may be supplied via another information storage medium instead of the optical disk 500, or may be downloaded from another apparatus, such as a server apparatus via a communication network. For downloading, the game machine 300 may include a network communication module.

The radio communication module 320 controls radiowave communication with the controller 350. The radio communication module 320 serves as an input part for inputting biological information measured at the biological information input apparatus 200' to the game machine 300 (respiration characteristic analysis apparatus).

The image processor 330 generates image data and supplies the image data to the monitor 400. The sound processor 340 generates audio data indicating sound effects and speech and supplies the audio data to the monitor 400 in order that speakers of the monitor 400 can emit sounds.

The CPU 360 serves as a main controller for controlling the entire game machine 300 by executing various programs stored in the ROM 301 and the hard disk 303. For example, the CPU 360 controls the radio communication module 320, thereby communicating with the biological information input apparatus 200' via the controller 350. The CPU 360 instructs the biological information input apparatus 200' to select appropriate electrodes among the current electrodes X1 through X4 and the voltage electrodes Y1 through Y4, to determine bioelectrical impedances, and to measure body weight.

The CPU 360 of the game machine 300 executes the respiration analysis process described in conjunction with the first embodiment, so as to determine whether respiration of the human subject is costal respiration or abdominal respiration. The CPU 360 also executes the respiration depth calculating process and the respiration depth displaying process described in conjunction with the first embodiment, so as to cause the monitor 400 to display the first bar graph BG1 indicative of the magnitude of each of costal respiration and abdominal respiration, and to display the second bar graph BG2 indicative of the abdominal respiration percentage level.

The CPU 360 may execute the respiration type determination process described in conjunction with the second embodiment, so as to decide that respiration of the human subject is costal respiration, abdominal respiration, or draw-in respiration. The CPU 360 may execute the Lissajous figure displaying process described in conjunction with the third embodiment, so as to display at least one Lissajous figure on the monitor 400. The CPU 360 may execute the process for training the human subject for appropriate breathing using the bar graph BG1 through BG3, the Lissajous figure, the schematic view of lungs, or the like. Thus, the CPU 360 may execute various processes described in conjunction with the first through third embodiments and the first through fifth variations.

Fig. 58 is a diagram showing the data format of a training menu management table TBL. The training menu management table TBL is used for training the human subject for breathing in accordance with training menus that match respiration capability of the human subject. The training menu management table TBL records multiple groups, each consisting of multiple menus of training for breathing, each group corresponding to a ranking of respiration capability, and requirements for advancing through the rankings and for advancing to the next ranking. In the illustrated example, there are five rankings (rankings 1 to 5). For each group at a ranking, 20 training menus are prepared.

Examples of the training menus include a training for learning costal breathing and abdominal breathing, a training for learning a complete breathing in which costal breathing and abdominal breathing is combined, a training for learning draw-in breathing, a training for improving lung ventilation capability by guiding into abdominal breathing and complete breathing, a training for improving respiratory functions and motion functions by guiding into draw-in breathing, and a training for strengthening respiratory functions and motion functions by guiding into various types of breathing with load on respiratory muscles by holding various poses used in *yoga* or *Qigong.*

In the specification, a complete breathing means a type of respiration in which lung respiration capability is used to the maximum with movement of the abdomen, chest, and scapular region. At inhalation in complete breathing, the human expands the abdomen at the start of aspiration, expands the thoracic cage with moving the chest forward while holding aspiration, and then moves the shoulders upward and aspires a little more, whereby the lung volume is broadened to the maximum. Exhalation in complete breathing is opposite to inhalation. At exhalation in complete breathing, the human moves the shoulders downward at the start of expiration, constricts the thoracic cage while holding expiration, and constricts the abdomen and expires more.

Examples of the training menus also include a training menu in which a Lissajous figure, the bar graphs BG1 and BG2, or a schematic view of lungs showing the status of breathing of the human subject is displayed, so that the human subject can confirm the status of breathing during training. Examples of the training menus also include a training menu in which a target Lissajous figure, the bar graph BG3, or schematic view of lungs showing a target model of breathing to be performed by the human subject is displayed, so that the human subject can confirm the target model of breathing during training. Examples of the training menus also include a training menu in which both of a Lissajous figure, the bar graphs BG1 and BG2, or a schematic view of lungs showing the status of breathing of the human subject and a target Lissajous figure, the bar graph BG3, or schematic view of lungs showing a target model of breathing to be performed by the human subject are displayed, so that the human subject can compare the status of breathing and the target model of breathing during training.

The requirement for advancing through each ranking may be, for example, that the magnitude of costal respiration or abdominal respiration is equal to or greater than a predetermined standard level; that the lung ventilation capability is equal to or greater than a predetermined standard level; that the human subject can perform draw-in respiration; that the abdominal respiration percentage level is equal to or greater than a predetermined standard level; or all of 20 training menus for the ranking have been completed. The number of rankings described in the training menu management table TBL is not limited as long as it is at least two. The number of the training menus in each group at a ranking is also not limited as long as it is at least one.

More specific examples of training menus in each ranking will be described next.

5.6.1. RANKING 1 (TRAINING OF PATIENTS WITH SEVERE RESPIRATORY DISEASE)

The training menus include training plans for strengthening the costal respiratory muscles that contribute to fundamental respiratory motion by guiding into costal breathing, thereby healing respiratory functions deteriorated by respiratory disease.

5.6.2. RANKING 2 (TRAINING OF PATIENTS WITH MILD RESPIRATORY DISEASE)

The training menus include training plans for strengthening the abdominal respiratory muscles including the diaphragm by guiding into abdominal breathing, thereby healing respiratory functions deteriorated by respiratory disease.

5.6.3. RANKING 3 (STANDARD TRAINING OF PHYSICALLY UNIMPAIRED PERSONS)

The training menus include training plans for enhancing costal respiratory muscles and abdominal respiratory muscles by guiding into the complete breathing into which costal breathing and abdominal breathing are combined, thereby improving healthy respiratory functions or healthy respiratory functions deteriorated by smoking, lifestyle, lack of activity, or aging.

5.6.4. RANKING 4 (LIGHTLY-LOADED TRAINING)

The training menus include training plans for strengthening inner muscles at the body trunk (e.g., the transverse abdominal muscle and the erector muscle of the spine) by guiding into draw-in breathing, thereby improving respiratory functions, preventing backache, or enhancing motion functions.

5.6.5. RANKING 5 (HIGHLY-LOADED TRAINING OF ATHLETES)

The training menus include training plans for strengthening motion functions by guiding into draw-in breathing with load on respiratory muscles by holding various poses used in *yoga* or *Qigong.*

Training for patients with respiratory disease (rankings 1 and 2) will be conducted under medical guidance by therapy specialists. Training for patients with respiratory disease will be conducted by human subjects whose diaphragm can function to some extent and whose respiratory functions are expected to be improved by training of breathing.

For all of rankings 1 to 5, a load may be applied in such a manner that the human subject breathes through pursed lips. It is possible to freely determine combination of type of respiration and poses, and allocation of time in training.

Fig. 59 is a flow chart showing an example of a breathing training management process. The breathing training management process is started to be executed when the human subject starts training for breathing using the system 5. In the breathing training management process, the CPU 360 first executes a process for determining the respiration capability of the human subject (step S901). For example, the CPU 360 reports a message for instructing the human subject to perform a type of respiration, and then executes the respiration analysis process and the respiration depth calculating process described in conjunction with the first embodiment, or the Lissajous figure displaying process and the process for deciding whether the lung ventilation capability is good or bad described in conjunction with the third embodiment. Therefore, the CPU 360 determines the respiration capability of the human subject that may include, for example, the magnitude of each of costal respiration and abdominal respiration, the lung ventilation capability, and the abdominal respiration percentage level.

In order to determine the normal respiration capability of the human subject at normal status, step S901 may be executed without informing that the respiration parameters are being determined. At step S901, the respiration capability of the human subject may be determined on the basis of the first bioelectrical impedance $Z_a$ and the second bioelectrical impedance $Z_b$, or on the basis of the bioelectrical impendances $Z_{aL}$, and $Z_b$.

Next, at step S902, the CPU 360 refers to the training menu management table TBL, and it identifies the ranking in the table corresponding to the respiration capability of the human subject determined at step S901. Next, the CPU 360 selects the group of training menus corresponding to the ranking from among the training menu management table TBL (step S903). For example, if the ranking of the human subject is ranking 3, the CPU 360 selects the group consisting of menus 41 through 60 from among the training menu management table TBL shown in Fig. 58.

Then, at step S904, the CPU 360 executes a breathing training process for training the human subject for breathing using the training menus selected at step S903. For example, if the ranking of the human subject is ranking 3, the CPU 360 executes a process for prompting the human subject to train for breathing using menus 41 through 60. If the ranking of the human subject is ranking 3 for standard training of physically unimpaired persons, in accordance with the training menus, the CPU 360 guides the human subject into the complete breathing into which costal breathing and abdominal breathing are combined, so that the human subject learns the complete breathing or improves lung ventilation capability. The CPU 360 may cause the monitor 400 to display the Lissajous figure, the bar graphs BG1 and BG2, or a schematic view of lungs showing the status of breathing of the human subject, so that the human subject can confirm the status of breathing during training. The CPU 360 may cause the monitor 400 to display a target Lissajous figure, the bar graph BG3, or schematic view of lungs showing a target model of breathing to be performed by the human subject, so that the human subject can confirm the target model of breathing during training. The CPU 360 may cause the monitor 400 to display a Lissajous figure, the bar graphs BG1 and BG2, or a schematic view of lungs showing the status ofbreathing of the human subject and a target Lissajous figure, the bar graph BG3 , or schematic view of lungs showing a target model of breathing to be performed by the human subject, so that the human subject can compare the status of breathing and the target model of breathing during training.

Then, the CPU 360 retrieves the requirement for advancing through the ranking in which the human subject is being placed from the training menu management table TBL, and decides whether or not the requirement for advancing through the ranking is satisfied (step S905). For example, if the ranking on which the human subject is being placed is ranking 3, the CPU 360 reads requirement C from the training menu management table TBL shown in Fig. 58, and decides whether or not requirement C is satisfied.

For example, if requirement C is that the magnitude of abdominal respiration is equal to or greater than a predetermined standard value, the CPU 360 decides whether or not the magnitude of abdominal respiration of the human subject is equal to or greater than the predetermined standard value, on the basis of the result of the respiration analysis process and the respiration depth calculating process described in conjunction with the first embodiment. Alternatively, if requirement C is that the lung ventilation capability is equal to or greater than a predetermined standard value, the CPU 360 decides whether or not the lung ventilation capability is equal to or greater than the predetermined standard value, on the basis of the result of the process for deciding whether the lung ventilation capability is good or bad described in conjunction with the third embodiment. If requirement C is that the human subject can perform draw-in respiration, the CPU 360 decides whether or not the human subject can perform draw-in respiration, on the basis of the respiration type determination process described in conjunction with the second embodiment. If requirement C is that all of 20 training menus for the ranking have been completed, the CPU 360 decides whether or not the menus 41 to 60 have been completed.

If the decision at step S905 is negative, the process returns to step S904 for continuing the breathing training at the current ranking. If the decision at step S905 is affirmative, the CPU 360 advances the ranking of the human subject to the next ranking (step S906), and returns to step S903. For example, if the current ranking of the human subject is ranking 3, the CPU 360 changes the ranking of the human subject to ranking 4 at step S906, and returns to step S903, and starts a new training in accordance with menus 61 to 80 corresponding to ranking 4 from the training menu management table TBL.

If the human subject or another person manipulates the controller 350 to instruct the end of training, the breathing training management process is thus ended for the human subject. At the ending thereof, the CPU 360 stores information in the ranking of the human subject is written on the hard disk 303. When the human subject next starts training for breathing, the CPU 360 reads the information on the ranking from the hard disk 303, and then starts with step S903 in the next breathing training management process.

As has been described above, according to this variation, respiration of the human subject can be determined easily at home in a manner similar to the measurements of the body weight and the body fat. In addition, training for breathing can be conducted easily at home by the game machine 300. In this variation, the human subject can effectively train for breathing in accordance with the training menus that match the respiration capability of the human subject. The training menus are prepared at each ranking of respiration capability, and if the requirement defined at each ranking is satisfied, the human subject can advance to the next ranking. Accordingly, the training process has a game element by which the human subject is amused, and the human subject is motivated to train for breathing.

Instead of the game machine 300, the body condition determination apparatus 1 in any one of the first through third embodiments may execute the breathing training management process (Fig. 59). In this case, the computer program and data, such as the training menu management table TBL (Fig. 58) to execute the breathing training management process may be stored in the first memory 120 of the body condition determination apparatus 1. Instead of the game machine 300, a personal computer or a portable electrical device (e.g., a cell phone or a tablet computer) may be used for executing the breathing training management process, and a head-mounted display may be used as a display device, especially in the use of a portable electrical device.

5.7. SEVENTH VARIATION

The body condition determination apparatus 1 need not include the display device 160, and may instead cause an external display device to display Lissajous figures, bar graphs BG1 through BG3, etc. The body condition determination apparatus 1 need not include the bioelectrical impedance determination part 200, and may include an input part for inputting to the body condition determination apparatus 1 information on the first bioelectrical impedance $Z_a$ and the second bioelectrical impedance $Z_b$ (or the bioelectrical impedances $Z_{aL}$, and $Z_b$) that are determined at an external bioelectrical impedance determination apparatus. The external bioelectrical impedance determination apparatus may send the information to the input device by radiowaves or by cable. The input part may be a communication interface, for example, a radiowave communication module, a network communication module, or a USB (Universal Serial Bus) interface.

5.8. EIGHTH VARIATION

The Lissajous figure may be displayed not only at training for breathing, but also at determination of the type and magnitude of respiration. In addition, the present invention is not limited to an apparatus or system that is adapted for only determining the type and magnitude of respiration and prompting training for breathing. For example, the teaching of the present invention can be incorporated into various strength-training machines or systems, or in fitness training machines or systems

5.9. NINTH VARIATION

In the above-described fourth embodiment, the costal-abdominal ventilation balance value BP indicating respiration characteristics of both lungs of the human subject are calculated and displayed. However it is possible to calculate and display an index indicative of respiration characteristics of the right lung (right costal-abdominal ventilation balance value BPR) and an index indicative of respiration characteristics of the left lung (left costal-abdominal ventilation balance value BPL).

The right costal-abdominal ventilation balance value BPR is calculates on the basis of measurement values of the right first bioelectrical impedance $Z_{aR}$ at the right upper body trunk including the upper lobe of the right lung and excluding the abdomen and measurement values of the right second bioelectrical impedance $Z_{bR}$ at the right middle body trunk including the median and lower lobes of the right lung and the abdomen.

The right first bioelectrical impedance $Z_{aR}$ is calculated on the basis of the current data $D_i$ indicating the reference current $I_{ref}$ flowing between the right and left hands and the voltage data $D_v$ indicating the potential difference between right-foot voltage electrode Y2 and the right-hand voltage electrode Y4. The manner of change in the right first bioelectrical impedance $Z_{aR}$ in respiration is the same as that in the first bioelectrical impedance $Z_a$.

The right second bioelectrical impedance $Z_{bR}$ is calculated on the basis of the current data $D_i$ indicating the reference current $I_{ref}$ flowing between the right hand and the right foot and the voltage data $D_v$ indicating the potential difference between the left hand and the left foot. The manner of change in the right second bioelectrical impedance $Z_{bR}$ in respiration is the same as that in the second bioelectrical impedance $Z_b$.

A standard level of change over time in the right first bioelectrical impedance $Z_{aR}$ used for extracting information on respiration of the human subject will be referred to as the third centering value $Z_{aR0}$. The manner for generation or calculation of the third centering value $Z_{aR0}$ is the same as that of the above-described first centering value $Z_{a0}$. A standard level of change over time in the right second bioelectrical impedance $Z_{bR}$ used for extracting information on respiration of the human subject will be referred to as the fourth centering value $Z_{bR0}$. The manner for generation or calculation of the fourth centering value $Z_{bR0}$ is the same as that of the above-described second centering value $Z_{b0}$.

The difference between the measurement value of the right first bioelectrical impedance $Z_{aR}$ and the third centering value $Z_{aR0}$ will be referred to as the third difference $\Delta Z_{aR}$. The difference between the measurement value of the right second bioelectrical impedance $Z_{bR}$ and the fourth centering value $Z_{bR0}$ will be referred to as the fourth difference $\Delta Z_{bR}$. The CPU 170 selects a peak value $\Delta Z_{aR}(MAX)$ among the third differences at the inhalation of the last single respiration of the human subject, and selects a peak value $\Delta Z_{bR}(MAX)$ among the fourth differences at the inhalation of the last single respiration. Next, the CPU 170 calculates the ratio of the peak value $\Delta Z_{bR}(MAX)$ to the peak value $\Delta Z_{aR}$

(MAX), and decides the ratio $\Delta Z_{bR}(MAX)/\Delta Z_{aR}(MAX)$ as the right costal-abdominal ventilation balance value BPR, and causes the display device 160 to show the right costal-abdominal ventilation balance value BPR.

The left costal-abdominal ventilation balance value BPL is calculated on the basis of measurement values of the left first bioelectrical impedance $Z_{aL}$ at the left upper body trunk including the upper lobe of the left lung and excluding the abdomen and measurement values of the left second bioelectrical impedance $Z_{bL}$ at the left middle body trunk including the median and lower lobes of the left lung.

The left first bioelectrical impedance $Z_{aL}$ is calculated on the basis of the current data $D_i$ indicating the reference current $I_{ref}$ flowing between the right and left hands and the voltage data $D_v$ indicating the potential difference between the left-foot voltage electrode Y1 and the left-hand voltage electrode Y3. The manner of change in the left first bioelectrical impedance $Z_{aL}$ in respiration is the same as that in the first bioelectrical impedance.

The left second bioelectrical impedance $Z_{bL}$ is calculated on the basis of the current data $D_i$ indicating the reference current $I_{ref}$ flowing between the left hand and the left foot and the voltage data $D_v$ indicating the potential difference between the right hand and the right foot. The manner of change in the left second bioelectrical impedance $Z_{bL}$ in respiration is the same as that in the second bioelectrical impedance $Z_b$.

A standard level of change over time in the left first bioelectrical impedance $Z_{aL}$ used for extracting information on respiration of the human subject will be referred to as the fifth centering value $Z_{aL0}$. The manner for generation or calculation of the fifth centering value $Z_{aL0}$ is the same as that of the above-described first centering value $Z_{a0}$. A standard level of change over time in the left second bioelectrical impedance $Z_{bL}$ used for extracting information on respiration of the human subject will be referred to as the sixth centering value $Z_{bL0}$. The manner for generation or calculation of the sixth centering value $Z_{bL0}$ is the same as that of the above-described second centering value $Z_{b0}$.

The difference between the measurement value of the left first bioelectrical impedance $Z_{aL}$ and the fifth centering value $Z_{aL0}$ will be referred to as the fifth difference $\Delta Z_{aL}$. The difference between the measurement value of the left second bioelectrical impedance $Z_{bL}$ and the sixth centering value $Z_{bL0}$ will be referred to as the sixth difference $\Delta Z_{bL}$. The CPU 170 selects a peak value $\Delta Z_{aL}(MAX)$ among the fifth differences at the inhalation of the last single respiration of the human subject, and selects a peak value $\Delta Z_{bL}(MAX)$ among the sixth differences at the inhalation of the last single respiration. Next, the CPU 170 calculates the ratio of the peak value $\Delta Z_{bL}(MAX)$ to the peak value $\Delta Z_{aL}(MAX)$, and decides the ratio $\Delta Z_{bL}(MAX)/\Delta Z_{aL}(MAX)$ as the left costal-abdominal ventilation balance value BPL, and causes the display device 160 to show the left costal-abdominal ventilation balance value BPL. By displaying the right and left costal-abdominal ventilation balance values BPR and BPL, it is possible to determine the ventilation characteristics of the right lung and the ventilation characteristics of the left lung.

## 5.10. TENTH VARIATION

In the above-described fourth embodiment, the costal-abdominal ventilation balance value BP indicative of respiration characteristics of the human subject of a single respiration is the ratio $\Delta Z_b(MAX)/\Delta Z_a(MAX)$ in which $\Delta Z_a(MAX)$ is the peak value of the first differences $\Delta Z_a$ at inhalations in the single respiration and $\Delta Z_b(MAX)$ is the peak value of the second differences $\Delta Z_b$ at inhalations in the single respiration. However, the costal-abdominal ventilation balance value BP may be, for example, $\Delta Z_a(MAX)/\Delta Z_b(MAX))$. Alternatively, the costal-abdominal ventilation balance value BP may be, for example, the ratio of an integral value of the second differences $\Delta Z_b$ to an integral value of the first difference $\Delta Z_a$ at inhalations of a single respiration, or the ratio of an integral value of the first difference $\Delta Z_a$ to an integral value of the second differences $\Delta Z_b$ at inhalations of a single respiration. In summary, the costal-abdominal ventilation balance value BP may be a ratio between the first differences $\Delta Z_a$ and the second differences $\Delta Z_b$.

## 5.11. ELEVENTH VARIATION

In the above-described fourth embodiment, the costal-abdominal ventilation balance value BP is the ratio of $\Delta Z_b(MAX)$ (that is, the index indicative of respiration functions of the median and lower lobes of the lungs of the human subject) to $\Delta Z_a(MAX)$ (that is, the index indicative of respiration functions of the upper lobes of the lungs of the human subject). However, the costal-abdominal ventilation balance value BP may be, for example, the ratio between $\Delta Z_a(MAX)$ and the sum of $\Delta Z_a(MAX)$ of $\Delta Z_b(MAX)$, i.e., the ratio between the index indicative of respiration functions of the upper lobes of the lungs of the human subject and the index indicative of respiration functions of the entire lobes of the lungs of the human subject. Alternatively, the costal-abdominal ventilation balance value BP may be, for example, the ratio between $\Delta Z_b(MAX)$ and the sum of $\Delta Z_a(MAX)$ of $\Delta Z_b(MAX)$, i.e., the ratio between the index indicative of respiration functions of the median and lower lobes of the lungs of the human subject and the index indicative of respiration functions of the entire lobes of the lungs of the human subject.

5.12. TWELFTH VARIATION

Instead of the body condition determination apparatus 1 or the biological information input apparatus 200', an apparatus 620 shown in Figs. 65 and 66 may be used. The apparatus 620 includes a platform 610 on which the human subject stands and a handle unit 620. The platform 610 is provided with a left-foot current electrode X1 and a left-foot voltage electrode Y1 on which the left foot of the human subject will be placed, and a right-foot current electrode X2 and a right-foot voltage electrode Y2 on which the right foot of the human subject will be placed.

The handle unit 620 is provided with a human interface 622 and a display device 626. The human interface 622 includes touch buttons 624, and the human subject or another person may manipulate the touch buttons 624 in order to input personal information on the human subject, for example, the height, age, and sex into the apparatus 620. The display device 626 shows the measurement results, such as the weight or the type of respiration. The display device 626 also shows instructions (of rhythm and pattern of exhalation and inhalation) to exhale and inhale in order to lead the human subject to perform abdominal breathing. The display device 626 shows messages for leading the human subject to input various information into the human interface 622. The handle unit 620 further includes a right electrode handle 630R and a left electrode handle 630L. The right electrode handle 630R includes a right-hand current electrode X4 and a right-hand voltage electrode Y4, whereas the left electrode handle 30L includes a left-hand current electrode X3 and a left-hand voltage electrode Y3,

The handle unit 620 is mechanically connected with a housing (not shown) beneath the platform 610 via a cable 640, and is electrically connected with electric circuits beneath the platform 610. The cable 640 is extendable, i.e., capable of being pull out from the housing (not shown) beneath the platform 610, so that the handle unit 620 can be brought into a position shown in Fig. 66 in which the apparatus 620 is away from the platform 610, from another position shown in Fig. 65 in which the apparatus 620 is close to the platform 610. The cable 640 is retractable, i.e., wound by a take-up unit (not shown) beneath the platform 610, so that handle unit 620 can be brought into the position shown in Fig. 65 in which the apparatus 620 is close to the platform 610, from the position shown in Fig. 66 in which the apparatus 620 is away from the platform 610.

In the use of the apparatus 620, the human subject stands on the platform 610, grips the electrode handles 630R and 630L, extends the cable 640, and holds the arms at a predetermined able, e.g., horizontally. Then, the bioelectrical impedances are measured.

**Claims**

1. A respiration characteristic analysis apparatus (1, 620) comprising:

a bioelectrical impedance determiner (170, 200) adapted for determining a first bioelectrical impedance ($Z_a$, $Z_{aR}$, $Z_{aL}$) at the upper body trunk of a human subject including the upper lobes of the lungs of the human subject and excluding the abdomen of the human subject, and a second bioelectrical impedance ($Z_b$, $Z_{bR}$, $Z_{bL}$) at the middle body trunk of the human subject including the median and lower lobes of the lungs of the human subject and the abdomen of the human subject; and

an analyzer (170) adapted for analyzing a respiration characteristic of the human subject on the basis of change over time in each of the first bioelectrical impedance ($Z_a$, $Z_{aR}$, $Z_{aL}$) and the second bioelectrical impedance ($Z_b$, $Z_{bR}$, $Z_{bL}$) determined by the bioelectrical impedance determiner (170, 200).

2. The respiration characteristic analysis apparatus (1, 620) according to claim 1, further comprising:

a centering value generator (170) adapted for generating a first centering value ($Z_{a0}$, $Z_{aR0}$, $Z_{aL0}$) that is an average of the first bioelectrical impedances ($Z_a$, $Z_{aR}$, $Z_{aL}$) within a past unit time on the basis of change over time in the first bioelectrical impedance ($Z_a$, $Z_{aR}$, $Z_{aL}$), and for generating a second centering value ($Z_{b0}$, $Z_{bR0}$, $Z_{bL0}$) that is an average of the second bioelectrical impedances ($Z_b$, $Z_{bR}$, $Z_{bL}$) within a past unit time on the basis of change over time in the second bioelectrical impedance ($Z_b$, $Z_{bR}$, $Z_{bL}$), the first centering value ($Z_{a0}$, $Z_{aR0}$, $Z_{aL0}$) being a standard level of change over time in the first bioelectrical impedance ($Z_a$, $Z_{aR}$, $Z_{aL}$), the second centering value ($Z_{b0}$, $Z_{bR0}$, $Z_{bL0}$) being a standard level of change over time in the second bioelectrical impedance ($Z_b$, $Z_{bR}$, $Z_{bL}$);

a first difference calculator (170) adapted for calculating a first difference ($\Delta Z_a$, $\Delta Z_{aR}$, $\Delta Z_{aL}$) between the first bioelectrical impedance ($Z_a$, $Z_{aR}$, $Z_{aL}$) and the first centering value ($Z_{a0}$, $Z_{aR0}$, $Z_{aL0}$); and

a second difference calculator (170) adapted for calculating a second difference ($\Delta Z_b$, $\Delta Z_{bR}$, $\Delta Z_{bL}$) between the second bioelectrical impedance ($Z_b$, $Z_{bR}$, $Z_{bL}$) and the second centering value ($Z_{b0}$, $Z_{bR0}$, $Z_{bL0}$),

wherein the analyzer (170) is adapted for analyzing the respiration characteristic of a part of the human subject that contributes to respiration of the human subject on the basis of the first difference ($\Delta Z_a$, $\Delta Z_{aR}$, $\Delta Z_{aL}$) and the second difference ($\Delta Z_b$, $\Delta Z_{bR}$, $\Delta Z_{bL}$).

3. The respiration characteristic analysis apparatus (1, 620) according to claim 2, further comprising a zero-cross time decider (170) for deciding zero-cross times in which the first bioelectrical impedance ($Z_a$, $Z_{aR}$, $Z_{aL}$) is equal to the first centering value ($Z_{a0}$, $Z_{aR0}$, $Z_{aL0}$),

wherein the bioelectrical impedance determiner (170, 200) is adapted for determining the first bioelectrical impedance ($Z_a$, $Z_{aR}$, $Z_{aL}$) and the second bioelectrical impedance ($Z_b$, $Z_{bR}$, $Z_{bL}$) at each sampling time occurring at a predetermined cycle,

wherein the centering value generator (170) is adapted for generating the first centering value ($Z_{a0}$, $Z_{aR0}$, $Z_{aL0}$) on the basis of the first bioelectrical impedance ($Z_a$, $Z_{aR}$, $Z_{aL}$) at each of sampling times, a number of the sampling times being predetermined, and

wherein the centering value generator (170) is adapted for generating the second centering value ($Z_{b0}$, $Z_{bR0}$, $Z_{bL0}$) on the basis of the second bioelectrical impedance ($Z_b$, $Z_{bR}$, $Z_{bL}$) at each of zero-cross times decided by the zero-cross time decider (170), a number of the zero-cross times being predetermined.

4. The respiration characteristic analysis apparatus (1, 620) according to claim 3, wherein the centering value generator (170) is adapted for calculating a moving average (MA10) at each sampling time, the moving average being a moving average of the first bioelectrical impedances ($Z_a$, $Z_{aR}$, $Z_{aL}$) at multiple sampling times within a centering period starting from a time point that is a predetermined time length before a current sampling time and ending at the current sampling time, and wherein the centering value generator (170) is adapted for generating the first centering value ($Z_{a0}$, $Z_{aR0}$, $Z_{aL0}$) at the current sampling time on the basis of the moving averages (MA10) at multiple sampling times.

5. The respiration characteristic analysis apparatus (1, 620) according to claim 4, wherein a time length of the centering period is variable and is set depending on the respiration speed of the human subject at the current sampling time.

6. The respiration characteristic analysis apparatus (1, 620) according to any one of claims 3 through 5, wherein the centering value generator (170) is adapted for deciding whether or not each sampling time is a zero-cross time, and for generating the second centering value ($Z_{b0}$, $Z_{bR0}$, $Z_{bL0}$) at the current sampling time on the basis of the second bioelectrical impedances ($Z_b$, $Z_{bR}$, $Z_{bL}$) including the second bioelectrical impedance ($Z_b$, $Z_{bR}$, $Z_{bL}$) at the current sampling time if the current sampling time is a zero-cross time, and wherein the centering value generator (170) is adapted for deciding the second centering value ($Z_{b0}$, $Z_{bR0}$, $Z_{bL0}$) generated at a last sampling time as the second centering value ($Z_{b0}$, $Z_{bR0}$, $Z_{bL0}$) at the current sampling time if the current sampling time is not a zero-cross time.

7. The respiration characteristic analysis apparatus (1, 620) according to any one of claims 1 through 6, wherein the analyzer (170) is adapted for analyzing whether or not a function of the part of the human subject that contributes to respiration of the human subject is normal, on the basis of change over time in each of the first bioelectrical impedance ($Z_a$, $Z_{aR}$, $Z_{aL}$) and the second bioelectrical impedance ($Z_b$, $Z_{bR}$, $Z_{bL}$).

8. The respiration characteristic analysis apparatus (1, 620) according to any one of claims 2 through 7, wherein the analyzer (170) is adapted for deciding that a function of the part of the human subject that contributes to respiration of the human subject is abnormal if a ratio ($\Delta Z_b$(MAX)/$\Delta Z_a$(MAX), $\Delta Z_{bR}$(MAX)/$\Delta Z_{aR}$(MAX), $\Delta Z_{bL}$(MAX)/$\Delta Z_{aL}$(MAX)) of the peak value ($\Delta Z_b$(MAX), $\Delta Z_{bR}$(MAX), $\Delta Z_{bL}$(MAX)) of change in the second difference ($\Delta Z_b$, $\Delta Z_{bR}$, $\Delta Z_{bL}$) to the peak value ($\Delta Z_a$(MAX), $\Delta Z_{aR}$(MAX), $\Delta Z_{aL}$(MAX)) of change in the first difference ($\Delta Z_a$, $\Delta Z_{aR}$, $\Delta Z_{AL}$) is equal to or greater than a predetermined threshold, and wherein the analyzer (170) is adapted for deciding that a function of the part of the human subject that contributes to respiration of the human subject is normal if the ratio of the peak value ($\Delta Z_b$(MAX), $\Delta Z_{bR}$(MAX), $\Delta Z_{bL}$(MAX)) of change in the second difference ($\Delta \mathbf{Z}_b$, $\Delta Z_{bR}$, $\Delta Z_{bL}$) to the peak value ($\Delta Z_a$(MAX), $\Delta Z_{aR}$(MAX), $\Delta Z_{aL}$(MAX)) of change in the first difference ($\Delta Z_a$, $\Delta Z_{aR}$, $\Delta Z_{aL}$) is less than the predetermined threshold.

9. The respiration characteristic analysis apparatus (1, 620) according to any one of claims 1 through 6, wherein the analyzer (170) is adapted for calculating indicative information ($\Delta R_{ib}/\Delta A_b$) that is used for identifying whether respiration of the human subject is abdominal or costal, on the basis of change over time in each of the first bioelectrical impedance ($Z_a$, $Z_{aR}$, $Z_{aL}$) and the second bioelectrical impedance ($Z_b$, $Z_{bR}$, $Z_{bL}$).

10. The respiration characteristic analysis apparatus (1, 620) according to claim 9, wherein the indicative information ($\Delta R_{ib}/\Delta A_b$) indicates a ratio between variation in the costal circumference ($\Delta Ri_b$) and variation in the abdominal

circumference in respiration ($\Delta A_b$), and
wherein the analyzer (170) is adapted for executing an arithmetic process in accordance with a formula expressing a relationship among indicative information ($\Delta R_{ib}/\Delta A_b$), first differences ($\Delta Z_a$, $\Delta Z_{aR}$, $\Delta Z_{aL}$), and second differences ($\Delta Z_b$, $\Delta Z_{bR}$, $\Delta Z_{bL}$), thereby calculating the indicative information ($\Delta R_{ib}/\Delta A_b$) corresponding to the first difference ($\Delta Z_a$, $\Delta Z_{aR}$, $\Delta Z_{aL}$) calculated by the first difference calculator (170) and the second difference ($\Delta Z_b$, $\Delta Z_{bR}$, $\Delta Z_{bL}$) calculated by the second difference calculator (170).

11. The respiration characteristic analysis apparatus (1, 620) according to claim 10, wherein the formula is expressed as

$$\Delta R_{ib}/\Delta A_b = (a * \Delta Z_b - \Delta Z_a)/\Delta Z_a + b,$$

wherein $\Delta R_{ib}$ is the variation in the costal circumference of the human subject, $\Delta A_b$ is the variation in the abdominal circumference of the human subject, $\Delta R_{ib}/\Delta A_b$ is the indicative information, $\Delta Z_a$ is the first difference, $\Delta Z_b$ is the second difference, and $a$ and b are constants.

12. The respiration characteristic analysis apparatus (1, 620) according to claim 11, wherein the ratio $\Delta R_{ib}/\Delta A_b$ indicates that respiration of the human subject is costal respiration if $\Delta R_{ib}/\Delta Ab$ is greater than a predetermined threshold, and wherein the ratio $\Delta R_{ib}/\Delta A_b$ indicates that respiration of the human subject is abdominal respiration if $\Delta R_{ib}/\Delta A_b$ is equal to or less than the predetermined threshold.

13. The respiration characteristic analysis apparatus (1, 620) according to any one of claims 9 through 11, wherein the analyzer (170) is adapted for calculating indicative information ($\Delta R_{ib}/\Delta A_b$) that is used for identifying whether respiration of the human subject is abdominal respiration, costal respiration, or a respiration in which inhalation and exhalation are repeated with the abdomen held in a constricted position, on the basis of change over time in each of the first bioelectrical impedance ($Z_a$, $Z_{aR}$, $Z_{aL}$) and the second bioelectrical impedance ($Z_b$, $Z_{bR}$, $Z_{bL}$).

14. The respiration characteristic analysis apparatus (1, 620) according to claim 11, wherein the analyzer (170) is adapted for calculating the ratio $\Delta R_{ib}/\Delta A_b$ as the indicative information that is used for identifying whether respiration of the human subject is abdominal respiration, costal respiration, or a respiration in which inhalation and exhalation are repeated with the abdomen held in a constricted position, on the basis of change over time in each of the first bioelectrical impedance ($Z_a$, $Z_{aR}$, $Z_{aL}$) and the second bioelectrical impedance ($Z_b$, $Z_{bR}$, $Z_{bL}$),
wherein the ratio $\Delta R_{ib}/\Delta A_b$ indicates that respiration of the human subject is abdominal respiration if $\Delta R_{ib}/\Delta A_b$ is equal to or less than a predetermined threshold,
wherein the ratio $\Delta R_{ib}/\Delta A_b$ indicates that respiration of the human subject is respiration in which inhalation and exhalation are repeated with the abdomen held in a constricted position if $\Delta R_{ib}/\Delta A_b$ is greater than a predetermined threshold and if the current second centering value ($Z_{b0}$, $Z_{bR0}$, $Z_{bL0}$) generated by the centering value generator (170) is equal to or greater than a sum of a standard second centering value ($Z_{b0}$, $Z_{bR0}$, $Z_{bL0}$) in costal respiration of the human subject and a predetermined value, and
wherein the ratio $\Delta R_{ib}/\Delta A_b$ indicates that respiration of the human subject is costal respiration if $\Delta R_{ib}/\Delta A_b$ is greater than a predetermined threshold and if the current second centering value ($Z_{b0}$, $Z_{bR0}$, $Z_{bL0}$) generated by the centering value generator (170) is less than a sum of a standard second centering value ($Z_{b0}$, $Z_{bR0}$, $Z_{bL0}$) in costal respiration of the human subject and a predetermined value.

15. The respiration characteristic analysis apparatus (1, 620) according to any one of claims 9 through 14, further comprising:

a respiration depth calculator (170) adapted for calculating a respiration depth of the human subject at every respiration of the human subject;
an abdominal respiration percentage level calculator (170) adapted for calculating, at every respiration of the human subject, an abdominal respiration percentage level that is a ratio of the abdominal respiration in the single respiration on the basis of the indicative information ($AR_{ib}/\Delta A_b$) calculated by the analyzer (170); and
a reporter (160, 170) adapted for reporting, at every respiration of the human subject, a magnitude of each of abdominal respiration and costal respiration and a margin level beyond an essential respiration depth with respect to each of abdominal respiration and costal respiration in a single respiration, on the basis of the respiration depth and the abdominal respiration percentage level at a current single respiration.

**16.** The respiration characteristic analysis apparatus (1, 620) according to claim 15, further comprising a normalizer (170) adapted for normalizing the respiration depth calculated by the respiration depth calculator (170), wherein the reporter (160, 170) is adapted for executing an arithmetic process in accordance with a second formula expressing a relationship between respiration depths and one-time ventilation volumes, each of which is a volume of air entering and leaving the lungs of human beings in a single respiratory action, thereby calculating a one-time ventilation volume corresponding to the respiration depth normalized by the normalizer (170), and wherein the reporter (160, 170) is adapted for deciding the magnitude of each of abdominal respiration and costal respiration and the margin level beyond the essential respiration depth with respect to each of abdominal respiration and costal respiration, on the basis of the one-time ventilation volume and the abdominal respiration percentage level, and for reporting the magnitude of each of abdominal respiration and costal respiration and the margin level beyond the essential respiration depth with respect to each of abdominal respiration and costal respiration.

**17.** The respiration characteristic analysis apparatus (1, 620) according to any one of claims 1 through 16, further comprising a display data generator (170) adapted for generating display data for displaying a Lissajous figure showing change over time in the first bioelectrical impedance ($Z_a$, $Z_{aR}$, $Z_{aL}$) and change over time in the second bioelectrical impedance ($Z_b$, $Z_{bR}$, $Z_{bL}$) in an orthogonal coordinate system having two orthogonal coordinate axes in which a first axis is the first bioelectrical impedance ($Z_a$, $Z_{aR}$, $Z_{aL}$) and a second axis is the second bioelectrical impedance ($Z_b$, $Z_{bR}$, $Z_{bL}$).

**18.** The respiration characteristic analysis apparatus (1, 620) according to claim 9, further comprising:

a display data generator (170) adapted for generating display data for displaying a Lissajous figure showing change over time in the first bioelectrical impedance ($Z_a$, $Z_{aR}$, $Z_{aL}$) and change over time in the second bioelectrical impedance ($Z_b$, $Z_{bR}$, $Z_{bL}$) in an orthogonal coordinate system having two orthogonal coordinate axes in which a first axis is the first bioelectrical impedance ($Z_a$, $Z_{aR}$, $Z_{aL}$) and a second axis is the second bioelectrical impedance ($Z_b$, $Z_{bR}$, $Z_{bL}$.); and
a centering value generator (170) adapted for generating a first centering value ($Z_{a0}$, $Z_{aR0}$, $Z_{aL0}$) that is an average of the first bioelectrical impedances ($Z_a$, $Z_{aR}$, $Z_{aL}$) within a past unit time on the basis of change over time in the first bioelectrical impedance ($Z_a$, $Z_{aR}$, $Z_{aL}$, and for generating a second centering value ($Z_{b0}$, $Z_{bR0}$, $Z_{bL0}$) that is an average of the second bioelectrical impedances ($Z_b$, $Z_{bR}$, $Z_{bL}$) within a past unit time on the basis of change over time in the second bioelectrical impedance ($Z_b$, $Z_{bR}$, $Z_{bL}$), the first centering value ($Z_{a0}$, $Z_{aR0}$, $Z_{aL0}$) being a standard level of change over time in the first bioelectrical impedance ($Z_a$, $Z_{aR}$, $Z_{aL}$), the second centering value ($Z_{b0}$, $Z_{bR0}$, $Z_{bL0}$) being a standard level of change over time in the second bioelectrical impedance ($Z_b$, $Z_{bR}$, $Z_{bL}$),
wherein the display data generator (170) is adapted for generating the display data for displaying the Lissajous figure so that a position (C) on the Lissajous figure defined by the first centering value ($Z_{a0}$, $Z_{aR0}$, $Z_{aL0}$) and the second centering value ($Z_{b0}$, $Z_{bR0}$, $Z_{bL0}$) is located at a center of a screen (160A) in which the Lissajous figure is displayed.

**19.** The respiration characteristic analysis apparatus (1, 620) according to claim 9, further comprising:

a display data generator (170) adapted for generating display data for displaying a Lissajous figure showing change over time in the first bioelectrical impedance ($Z_a$, $Z_{aR}$, $Z_{aL}$) and change over time in the second bioelectrical impedance ($Z_b$, $Z_{bR}$, $Z_{bL}$) in an orthogonal coordinate system having two orthogonal coordinate axes in which a first axis is the first bioelectrical impedance ($Z_a$, $Z_{aR}$, $Z_{aL}$ and a second axis is the second bioelectrical impedance ($Z_b$, $Z_{bR}$, $Z_{bL}$); and
a centering value generator (170) adapted for generating a first centering value ($Z_{a0}$, $Z_{aR0}$, $Z_{aL0}$) that is an average of the first bioelectrical impedances ($Z_a$, $Z_{aR}$, $Z_{aL}$) within a past unit time on the basis of change over time in the first bioelectrical impedance ($Z_a$, $Z_{aR}$, $Z_{aL}$), and for generating a second centering value ($Z_{b0}$, $Z_{bR0}$, $Z_{bL0}$) that is an average of the second bioelectrical impedances ($Z_b$, $Z_{bR}$, $Z_{bL}$) within a past unit time on the basis of change over time in the second bioelectrical impedance ($Z_b$, $Z_{bR}$, $Z_{bL}$), the first centering value ($Z_{a0}$, $Z_{aR0}$, $Z_{aL0}$) being a standard level of change over time in the first bioelectrical impedance ($Z_a$, $Z_{aR}$, $Z_{aL}$), the second centering value ($Z_{b0}$, $Z_{bR0}$, $Z_{bL0}$) being a standard level of change over time in the second bioelectrical impedance ($Z_b$, $Z_{bR}$, $Z_{bL}$),
wherein when the display data generator (170) generates the display data for displaying the Lissajous figure, the display data generator (170) is adapted for executing a first location centering process in which the Lissajous figure is centered in the first axis with respect to a screen (160A) in which the Lissajous figure is displayed on the basis of the first centering value ($Z_{a0}$, $Z_{aR0}$, $Z_{aL0}$), and is adapted for executing a second location centering process in which the Lissajous figure is centered in the second axis with respect to the screen (160A) on the basis of the second centering value ($Z_{bo}$, $Z_{bR0}$, $Z_{bL0}$), and wherein the display data generator (170) is adapted for executing the second location centering process less frequently than that for the first location centering process.

20. The respiration characteristic analysis apparatus (1, 620) according to any one of claims 17 through 19, further comprising a local-maximum-and-minimum decider (170) adapted for deciding a first local maximum that is a local maximum of change in the first bioelectrical impedance ($Z_a$, $Z_{aR}$, $Z_{aL}$), for deciding a first local minimum that is a local minimum of change in the first bioelectrical impedance ($Z_a$, $Z_{aR}$, $Z_{aL}$), for deciding a second local maximum that is a local maximum of change in the second bioelectrical impedance ($Z_b$, $Z_{bR}$, $Z_{bL}$), and for deciding a second local minimum that is a local minimum of change in the second bioelectrical impedance ($Z_b$, $Z_{bR}$, $Z_{bL}$), wherein the display data generator (170) is adapted for generating the display data for displaying the Lissajous figure so that a range of the Lissajous figure on a screen (160A) in which the Lissajous figure is displayed in the first and second axes is adjusted on the basis of the first local maximum, the first local minimum, the second local maximum, and the second local minimum.

21. The respiration characteristic analysis apparatus (1, 620) according to any one of claims 17 through 19, further comprising a local-maximum-and-minimum decider (170) adapted for deciding a first local maximum that is a local maximum of change in the first bioelectrical impedance ($Z_a$, $Z_{aR}$, $Z_{aL}$), for deciding a first local minimum that is a local minimum of change in the first bioelectrical impedance ($Z_a$, $Z_{aR}$, $Z_{aL}$), for deciding a second local maximum that is a local maximum of change in the second bioelectrical impedance ($Z_b$, $Z_{bR}$, $Z_{bL}$), and for deciding a second local minimum that is a local minimum of change in the second bioelectrical impedance ($Z_b$, $Z_{bR}$, $Z_{bL}$), wherein when the display data generator (170) generates the display data for displaying the Lissajous figure, the display data generator (170) is adapted for executing a first range adjustment process in which a range of the Lissajous figure on a screen (160A) in which the Lissajous figure is displayed in the first axis is adjusted on the basis of the first local maximum and the first local minimum, and is adapted for executing a second range adjustment process in which a range of the Lissajous figure on the screen (160A) in the second axis is adjusted on the basis of the second local maximum and the second local minimum, and wherein the display data generator (170) is adapted for executing the second range adjustment process less frequently than that for the first range adjustment process.

22. The respiration characteristic analysis apparatus (1, 620) according to any one of claims 17 through 21, wherein the display data generator (170) is adapted for generating the display data for displaying the Lissajous figure so that a displaying manner for a track of the Lissajous figure for a latest single respiration is different from a displaying manner for a track of the Lissajous figure for past respirations.

23. The respiration characteristic analysis apparatus (1, 620) according to any one of claims 17 through 21, wherein the display data generator (170) is adapted for generating the display data for displaying the Lissajous figure so that a displaying manner for tracks of the Lissajous figure is changed depending on an elapsed time.

24. The respiration characteristic analysis apparatus (1, 620) according to any one of claims 17 through 23, wherein the display data generator (170) is adapted for further generating target display data for displaying a target Lissajous figure showing a target model of breathing having a type and a magnitude of respiration to be performed by the human subject for guiding the human subject to perform breathing.

25. The respiration characteristic analysis apparatus (1, 620) according to any one of claims 17 through 24, further comprising:

   an inclination angle calculator (170) adapted for calculating an inclination angle ($\alpha$) of a track of the Lissajous figure; and
   a ventilation capability determiner (170) adapted for comparing the inclination angle ($\alpha$) calculated by the inclination angle calculator (170) with a predetermined reference inclination angle ($\beta$), so as to decide whether or not a lung ventilation capability of the human subject is good or bad.

26. The respiration characteristic analysis apparatus (1, 620) according to any one of claims 9 through 25, further comprising:

   a respiration depth calculator (170) adapted for calculating a respiration depth of the human subject at every respiration of the human subject; and

a graph generator (170) adapted for generating display data for indicating a graph showing change over time of respiration depth calculated by the respiration depth calculator (170), in such a manner that the graph is nonlinearly compressed in a direction of the time axis and earlier time intervals are more compressed than later time intervals, so that a time resolution for later time intervals is higher than that for earlier time intervals.

27. The respiration characteristic analysis apparatus (1, 620) according to any one of claims 9 through 25, further comprising:

a memory (120) adapted for storing training menus that are used for training the human subject for breathing, the training menus being classified into rankings of respiration capability, the memory (120) storing requirements for advancing through the rankings;
a respiration capability determiner (170) adapted for determining a respiration capability of the human subject on the basis of change over time in each of the first bioelectrical impedance ($Z_a$, $Z_{aR}$, $Z_{aL}$) and the second bioelectrical impedance ($Z_b$, $Z_{bR}$, $Z_{bL}$); and
a training manager (170) adapted for referring to the memory (120) for identifying a ranking corresponding to the respiration capability determined by the respiration capability determiner (170), and for executing a process for training the human subject for breathing using the training menus corresponding to the ranking, wherein the training manager (170) is adapted for advancing the ranking to a next ranking if the requirement for advancing through the ranking is satisfied.

28. The respiration characteristic analysis apparatus (1, 620) according to claim 9,
wherein the bioelectrical impedance determiner (170, 200) is adapted for determining a right first bioelectrical impedance ($Z_{aR}$) at the right upper body trunk of the human subject including the upper lobe of the right lung of the human subject and excluding the abdomen of the human subject, for determining a left first bioelectrical impedance ($Z_{aL}$) at the left upper body trunk of the human subject including the upper lobe of the left lung of the human subject and excluding the abdomen of the human subject, and for determining the second bioelectrical impedance ($Z_b$, $Z_{bR}$, $Z_{bL}$) at the middle body trunk, and
wherein the analyzer (170) is adapted for calculating indicative information ($\Delta R_{ib}/\Delta A_b$) that is used for identifying whether respiration of the human subject is abdominal or costal, on the basis of change over time in each of the right first bioelectrical impedance ($Z_{aR}$), the left first bioelectrical impedance ($Z_{aL}$), and the second bioelectrical impedance ($Z_b$, $Z_{bR}$, $Z_{bL}$).

29. The respiration characteristic analysis apparatus (1, 620) according to claim 28, further comprising a display data generator (170) adapted for generating first display data for displaying a first Lissajous figure showing change over time in the right first bioelectrical impedance ($Z_{aR}$) and change over time in the second bioelectrical impedance ($Z_b$, $Z_{bR}$, $Z_{bL}$) in an orthogonal coordinate system having two orthogonal coordinate axes in which a first axis is the right first bioelectrical impedance ($Z_{aR}$) and a second axis is the second bioelectrical impedance ($Z_b$, $Z_{bR}$, $Z_{bL}$), and for generating second display data for displaying a second Lissajous figure showing change over time in the left first bioelectrical impedance ($Z_{aL}$) and change over time in the second bioelectrical impedance ($Z_b$, $Z_{bR}$, $Z_{bL}$) in an orthogonal coordinate system having two orthogonal coordinate axes in which a first axis is the left first bioelectrical impedance ($Z_{aL}$) and a second axis is the second bioelectrical impedance ($Z_b$, $Z_{bR}$, $Z_{bL}$).

30. The respiration characteristic analysis apparatus (1, 620) according to claim 29, wherein the display data generator (170) is adapted for generating the first display data for displaying the first Lissajous figure and the second display data for displaying the second Lissajous figure so that the first Lissajous figure and the second Lissajous figure are overlaid on a screen (160A).

31. The respiration characteristic analysis apparatus (1, 620) according to claim 29 or 30, wherein the display data generator (170) is adapted for generating the first display data for displaying the first Lissajous figure and the second display data for displaying the second Lissajous figure so that a displaying manner for the first Lissajous figure is different from a displaying manner for the second Lissajous figure.

32. The respiration characteristic analysis apparatus (1, 620) according to claim 29 or 31, further comprising a track analyzer (170) adapted for detecting differences between a track of the first Lissajous figure and a track of the second Lissajous figure,
wherein the display data generator (170) is adapted for generating the first display data for displaying the first Lissajous figure and the second display data for displaying the second Lissajous figure so that the differences are highlighted on a screen (160A).

**33.** A respiration characteristic analysis apparatus (300) comprising:

an input part (320) for inputting to the respiration characteristic analysis apparatus (300) a first bioelectrical impedance ($Z_a$, $Z_{aR}$, $Z_{aL}$) at the upper body trunk of a human subject including the upper lobes of the lungs of the human subject and excluding the abdomen of the human subject and a second bioelectrical impedance ($Z_b$, $Z_{bR}$, $Z_{bL}$) at the middle body trunk of the human subject including the median and lower lobes of the lungs of the human subject and the abdomen of the human subject, the first bioelectrical impedance ($Z_a$, $Z_{aR}$, $Z_{aL}$) and the second bioelectrical impedance ($Z_b$, $Z_{bR}$, $Z_{bL}$) being determined at a bioelectrical impedance determination apparatus; and

an analyzer (360) adapted for analyzing a respiration characteristic of the human subject on the basis of change over time in each of the first bioelectrical impedance ($Z_a$, $Z_{aR}$, $Z_{aL}$) and the second bioelectrical impedance ($Z_b$, $Z_{bR}$, $Z_{bL}$).

**34.** A respiration characteristic analysis system (5) comprising:

a bioelectrical impedance determiner (360) adapted for determining a first bioelectrical impedance ($Z_a$, $Z_{aR}$, $Z_{aL}$) at the upper body trunk of a human subject including the upper lobes of the lungs of the human subject and excluding the abdomen of the human subject and a second bioelectrical impedance ($Z_b$, $Z_{bR}$, $Z_{bL}$) at the middle body trunk of the human subject including the median and lower lobes of the lungs of the human subject and the abdomen of the human subject; and

an analyzer (360) adapted for analyzing a respiration characteristic of the human subject on the basis of change over time in each of the first bioelectrical impedance ($Z_a$, $Z_{aR}$, $Z_{aL}$) and the second bioelectrical impedance ($Z_b$, $Z_{bR}$, $Z_{bL}$) determined by the bioelectrical impedance determiner (360).

**35.** The respiration characteristic analysis apparatus (1, 620) according to claim 1 or 2, wherein the bioelectrical impedance determiner (170, 200) is adapted for determining a right first bioelectrical impedance ($Z_{aR}$) at the right upper body trunk of the human subject including the upper lobe of the right lung of the human subject and excluding the abdomen of the human subject, a left first bioelectrical impedance ($Z_{aL}$) at the left upper body trunk of the human subject including the upper lobe of the left lung of the human subject and excluding the abdomen of the human subject, and the second bioelectrical impedance ($Z_b$, $Z_{bR}$, $Z_{bL}$) at the middle body trunk of the human subject including the median and lower lobes of the lungs of the human subject and the abdomen of the human subject,

the respiration characteristic analysis apparatus (1, 620) further comprising a display data generator (170) adapted for generating first display data for displaying a first Lissajous figure showing change over time in the right first bioelectrical impedance ($Z_{aR}$) and change over time in the second bioelectrical impedance ($Z_b$, $Z_{bR}$, $Z_{bL}$) in an orthogonal coordinate system having two orthogonal coordinate axes in which a first axis is the right first bioelectrical impedance ($Z_{aR}$) and a second axis is the second bioelectrical impedance ($Z_b$, $Z_{bR}$, $Z_{bL}$), and for generating second display data for displaying a second Lissajous figure showing change over time in the left first bioelectrical impedance ($Z_{aL}$) and change over time in the second bioelectrical impedance ($Z_b$, $Z_{bR}$, $Z_{bL}$) in an orthogonal coordinate system having two orthogonal coordinate axes in which a first axis is the left first bioelectrical impedance ($Z_{aL}$) and a second axis is the second bioelectrical impedance ($Z_b$, $Z_{bR}$, $Z_{bL}$).

**36.** The respiration characteristic analysis apparatus (1, 620) according to claim 35, wherein the display data generator (170) is adapted for generating the first display data for displaying the first Lissajous figure and the second display data for displaying the second Lissajous figure so that the first Lissajous figure and the second Lissajous figure are overlaid on a screen (160A).

**37.** The respiration characteristic analysis apparatus (1, 620) according to claim 35 or 36, wherein the display data generator (170) is adapted for generating the first display data for displaying the first Lissajous figure and the second display data for displaying the second Lissajous figure so that a displaying manner for the first Lissajous figure is different from a displaying manner for the second Lissajous figure.

**38.** The respiration characteristic analysis apparatus (1, 620) according to any one of claims 35 through 37, further comprising a track analyzer (170) adapted for detecting differences between a track of the first Lissajous figure and a track of the second Lissajous figure,

wherein the display data generator (170) is adapted for generating the first display data for displaying the first Lissajous figure and the second display data for displaying the second Lissajous figure so that the differences are highlighted on a screen (160A).

**39.** The respiration characteristic analysis apparatus (1, 620) according to claim 17, wherein the display data generator (170) is adapted for generating the display data for displaying the Lissajous figure so that a position (C) on the Lissajous figure defined by the first centering value ($Z_{a0}$, $Z_{aR0}$, $Z_{aL0}$) and the second centering value ($Z_{b0}$, $Z_{bR0}$, $Z_{bL0}$) is located at a center of a screen (160A) in which the Lissajous figure is displayed.

**40.** The respiration characteristic analysis apparatus (1, 620) according to claim 17, wherein when the display data generator (170) generates the display data for displaying the Lissajous figure, the display data generator (170) is adapted for executing a first location centering process in which the Lissajous figure is centered in the first axis with respect to a screen (160A) in which the Lissajous figure is displayed on the basis of the first centering value ($Z_{a0}$, $Z_{aR0}$, $Z_{aL0}$), and is adapted for executing a second location centering process in which the Lissajous figure is centered in the second axis with respect to the screen (160A) on the basis of the second centering value ($Z_{b0}$, $Z_{bR0}$, $Z_{bL0}$), and wherein the display data generator (170) is adapted for executing the second location centering process less frequently than that for the first location centering process.

**41.** A respiration characteristic analysis apparatus (300) comprising:

an input part (320) for inputting to the respiration characteristic analysis apparatus (300) a first bioelectrical impedance ($Z_a$, $Z_{aR}$, $Z_{aL}$) at the upper body trunk of a human subject including the upper lobes of the lungs of the human subject and excluding the abdomen of the human subject and a second bioelectrical impedance ($Z_b$, $Z_{bR}$, $Z_{bL}$) at the middle body trunk of the human subject including the median and lower lobes of the lungs of the human subject and the abdomen of the human subject, the first bioelectrical impedance ($Z_a$, $Z_{aR}$, $Z_{aL}$) and the second bioelectrical impedance ($Z_b$, $Z_{bR}$, $Z_{bL}$) being determined at a bioelectrical impedance determination apparatus (200', 620); and
a display data generator (360) adapted for generating display data for displaying a Lissajous figure showing change over time in the first bioelectrical impedance ($Z_a$, $Z_{aR}$, $Z_{aL}$) and change over time in the second bioelectrical impedance ($Z_b$, $Z_{bR}$, $Z_{bL}$) in an orthogonal coordinate system having two orthogonal coordinate axes in which a first axis is the first bioelectrical impedance ($Z_a$, $Z_{aR}$, $Z_{aL}$) and a second axis is the second bioelectrical impedance ($Z_b$, $Z_{bR}$, $Z_{bL}$).

**42.** A respiration characteristic analysis system (5) comprising:

an input part (320) for inputting to a respiration characteristic analysis apparatus (300) a first bioelectrical impedance ($Z_a$, $Z_{aR}$, $Z_{aL}$) at the upper body trunk of a human subject including the upper lobes of the lungs of the human subject and excluding the abdomen of the human subject and a second bioelectrical impedance ($Z_b$, $Z_{bR}$, $Z_{bL}$) at the middle body trunk of the human subject including the median and lower lobes of the lungs of the human subject and the abdomen of the human subject to the respiration characteristic analysis apparatus (300), the first bioelectrical impedance ($Z_a$, $Z_{aR}$, $Z_{aL}$) and the second bioelectrical impedance ($Z_b$, $Z_{bR}$, $Z_{bL}$) being determined at a bioelectrical impedance determination apparatus (200', 620);
a display data generator (360) adapted for generating display data for displaying a Lissajous figure showing change over time in the first bioelectrical impedance ($Z_a$, $Z_{aR}$, $Z_{aL}$) and change over time in the second bioelectrical impedance ($Z_b$, $Z_{bR}$, $Z_{bL}$) in an orthogonal coordinate system having two orthogonal coordinate axes in which a first axis is the first bioelectrical impedance ($Z_a$, $Z_{aR}$, $Z_{aL}$) and a second axis is the second bioelectrical impedance ($Z_b$, $Z_{bR}$, $Z_{bL}$); and
a display device (400) adapted for displaying the Lissajous figure on the basis of the display data generated by the display data generator (360).

# Fig. 1

Fig. 2

1    160 (150)

30R

30L

30

X2

Y2

20

X1

Y1

Fig. 3

160 (150)

X4    Y4

X3

Y3

Fig. 4

(A) SYSTEMIC BODY
(B) RIGHT LOWER EXTREMITY
(C) LEFT LOWER EXTREMITY
(D) RIGHT UPPER EXTREMITY AND UPPER BODY TRUNK
(E) LEFT UPPER EXTREMITY AND UPPER BODY TRUNK

CURRENT
VOLTAGE

(F) UPPER BODY TRUNK (BOTH HANDS)
(G) MIDDLE BODY TRUNK
(H) MIDDLE BODY TRUNK
(I) MIDDLE BODY TRUNK
(J) MIDDLE BODY TRUNK
(K) SYSTEMIC BODY (BOTH HANDS–BOTH FEET)

Fig. 5

```
              ┌──────────────┐
              │    START     │
              └──────────────┘
                     │
                     ▼
        ┌────────────────────────┐
  S1 ───│   ENTER PERSONAL       │
        │     INFORMATION        │
        └────────────────────────┘
                     │
                     ▼
        ┌────────────────────────┐
  S2 ───│       MEASURE          │
        │        WEIGHT          │
        └────────────────────────┘
                     │
                     ▼
       ╔════════════════════════╗
  S3 ──║     RESPIRATION        ║◄─────┐
       ║   ANALYSIS PROCESS     ║      │
       ╚════════════════════════╝      │
                     │                 │
                     ▼                 │
        ┌────────────────────────┐     │
  S4 ───│   RESPIRATION DEPTH    │     │
        │   DISPLAYING PROCESS   │─────┘
        └────────────────────────┘
```

Fig. 6

CHEST SKELETAL MUSCLE

ABDOMINAL SKELETAL MUSCLE

LUNG

DIAPHRAGM

VISCERAL TISSUE

Fig. 7A

$Z_b$: BIOELECTRICAL IMPEDANCE
AT MIDDLE BODY TRUNK

| $Z_4$ CHEST SKELETAL MUSCLE | $Z_5$ LUNGS |
| $Z_6$ ABDOMINAL SKELETAL MUSCLE | $Z_7$ VISCERAL TISSUE |

Fig. 7B

$Z_a$: BIOELECTRICAL IMPEDANCE
AT UPPER BODY TRUNK

$Z_3$ UPPER EXTREMITY SKELETAL MUSCLE

| $Z_1$ CHEST SKELETAL MUSCLE | $Z_2$ LUNGS |

Fig. 8

Fig. 9

ABDOMINAL RESPIRATION

INHALATION

EXHALATION

$Z_a$

$Z_b$

IMPEDANCE

TIME

Fig. 10

COSTAL RESPIRATION

INHALATION

EXHALATION

$Z_a$

$Z_b$

IMPEDANCE

TIME

Fig. 11

ABDOMINAL RESPIRATION

$A_b$

$R_{ib}$

MEASUREMENTS BY RESPITRACE

TIME

Fig. 12

COSTAL RESPIRATION

Fig. 13

# Fig. 14

```
        ┌─────────────────────────┐
        │  RESPIRATION ANALYSIS   │
        │        PROCESS          │
        └─────────────────────────┘
                    │
                    ▼◄──────────────┐
        ┌─────────────────────┐     │
S10 ──⟨   SAMPLING TIME?     ⟩─────┘
        └─────────────────────┘  NO
                    │ YES
                    ▼
        ┌─────────────────────────┐
S20 ──│    DETERMINE FIRST       │
        │  BIOELECTRICAL IMPEDANCE │
        └─────────────────────────┘
                    │
                    ▼
        ┌─────────────────────────┐
S30 ──│    DETERMINE SECOND      │
        │  BIOELECTRICAL IMPEDANCE │
        └─────────────────────────┘
                    │
                    ▼
        ┌─────────────────────────┐
S40 ──│    SMOOTHING PROCESS     │
        └─────────────────────────┘
                    │
                    ▼
        ┌─────────────────────────┐
S50 ──│  FIRST CENTERING PROCESS │
        └─────────────────────────┘
                    │
                    ▼
        ┌─────────────────────────┐
S60 ──│     FIRST DIFFERENCE     │
        │   CALCULATION PROCESS    │
        └─────────────────────────┘
                    │
                    ▼
        ┌─────────────────────────┐
S70 ──│     SECOND DIFFERENCE    │
        │   CALCULATION PROCESS    │
        └─────────────────────────┘
                    │
                    ▼
        ┌─────────────────────────┐
S80 ──│  ΔR_ib / ΔA_b ASSUMPTION │
        │        PROCESS           │
        └─────────────────────────┘
                    │
                    ▼
        ┌─────────────────────────┐
S90 ──│   RESPIRATION DEPTH      │
        │   CALCULATION PROCESS    │
        └─────────────────────────┘
                    │
                    ▼
            ┌──────────────┐
            │    RETURN    │
            └──────────────┘
```

S80 block label: $\Delta R_{ib} / \Delta A_b$ ASSUMPTION PROCESS

# Fig. 15

```
        ┌──────────────────────────────┐
        │   FIRST CENTERING PROCESS    │
        └──────────────────────────────┘
                      │
                      ▼
   S51 ┌──────────────────────────────────┐
       │     MA10 CALCULATION PROCESS     │
       └──────────────────────────────────┘
                      │
                      ▼
   S52 ┌──────────────────────────────────┐
       │     MA20 CALCULATION PROCESS     │
       └──────────────────────────────────┘
                      │
                      ▼
   S53 ┌──────────────────────────────────┐
       │    MAX10 EXTRACTION PROCESS      │
       └──────────────────────────────────┘
                      │
                      ▼
   S54 ┌──────────────────────────────────┐
       │    MIN10 EXTRACTION PROCESS      │
       └──────────────────────────────────┘
                      │
                      ▼
   S55 ┌──────────────────────────────────┐
       │     MEDIAN VALUE CALCULATION     │
       │             PROCESS              │
       └──────────────────────────────────┘
                      │
                      ▼
   S56 ┌──────────────────────────────────┐
       │      RESPIRATION TIMING          │
       │     EXTRACTION PROCESS           │
       └──────────────────────────────────┘
                      │
                      ▼
   S57 ┌──────────────────────────────────┐
       │  RESPIRATION SPEED INDICATION    │
       │     FLAG SETTING PROCESS         │
       └──────────────────────────────────┘
                      │
                      ▼
   S58 ┌──────────────────────────────────┐
       │      FIRST CENTERING VALUE       │
       │      CALCULATION PROCESS         │
       └──────────────────────────────────┘
                      │
                      ▼
                 ┌─────────┐
                 │   END   │
                 └─────────┘
```

70

# Fig. 16

```
        ┌─────────────────────────┐
        │  RESPIRATION TIMING     │
        │  EXTRACTION PROCESS     │
        └─────────────────────────┘
                    │
                    ▼
S201 ┌─────────────────────────────┐
     │  DIFFERENTIAL COEFFICIENT    │
     │  CALCULATION PROCESS         │
     └─────────────────────────────┘
                    │
                    ▼
S202      ╱────────────────╲    YES
        ◁   |dZ_a(n)| < 0.1?  ▷────────────────┐
          ╲────────────────╱                   │
                    │ NO                        ▼
                    ▼                   ┌───────────────┐
S203      ╱────────────────╲   NO       │  F_0(n) = 0   │
        ◁   dZ_a(n) > 0?     ▷───┐      └───────────────┘
          ╲────────────────╱     │
                    │ YES        ▼
                    ▼       ┌───────────────┐
           ┌───────────────┐│  F_0(n) = -1  │
           │  F_0(n) = +1  │└───────────────┘
           └───────────────┘
                    │
                    ▼
S204 ╱──────────────────────────────────────────╲   NO
   ◁  |F_0(n)| = |F_0(n-1)|? AND F_0(n-1) ≠ F_0(n)?  ▷───┐
     ╲──────────────────────────────────────────╱        │
                    │ YES                                 │
                    ▼                                     │
S205       ┌───────────────┐                              │
           │  F_0(n) = 0   │                              │
           └───────────────┘                              │
                    │                                     │
                    ▼◄────────────────────────────────────┘
                   ( A )
```

$$S202 \quad |dZ_a(n)| < 0.1?$$

$$S203 \quad dZ_a(n) > 0?$$

$$F_0(n) = 0$$

$$F_0(n) = +1$$

$$F_0(n) = -1$$

$$S204 \quad |F_0(n)| = |F_0(n-1)|? \text{ AND } F_0(n-1) \neq F_0(n)?$$

$$S205 \quad F_0(n) = 0$$

Fig. 17

A

NO — $F_0(n) = 0?$ — S206

YES

S207 — $\sum_{i=n-2}^{n} F_0(i) > +1?$ — YES

NO

NO — $\sum_{i=n-2}^{n} F_0(i) < -1?$ — S208

YES

$F_1(n) = 0$ $F_1(n) = -1$ $F_1(n) = +1$

S209 — $F_1(n) = +1?$ — YES

NO

S211

S210 — ADD ONE TO SAMPLING COUNTER VALUE

INITIALIZE SAMPLING COUNTER VALUE

END

Fig. 18

RESPIRATION SPEED INDICATION
FLAG SETTING PROCESS

$F_0(n) = 0$?　—S301

NO

YES

S302　$F_1(n) = +1$?　YES

NO

NO　$F_1(n) = -1$?　—S304

YES　$N(n-1) > 10$?　—S303　(B)

NO

YES

S305　$N(n-1) > 5$?　YES

NO

$F_{ma}(n) = F_{ma}(n-1)$

(B)

$F_{ma}(n) = 20$

$F_{ma}(n) = 10$

END

73

Fig. 19

FIRST CENTERING VALUE
CALCULATION PROCESS

S401 — $F_{ma}(n) = 10?$ — NO

YES

S402 — $[Z_{a0}(n-2) + Z_{a0}(n-1) + MA10(n)]/3 = Z_{a0}(n)$

$[Z_{a0}(n-2) + Z_{a0}(n-1) + MA20(n)]/3 = Z_{a0}(n)$ — S403

END

74

Fig. 20

## Fig. 21

```
        ┌─────────────────────────┐
        │   SECOND DIFFERENCE     │
        │  CALCULATION PROCESS    │
        └─────────────────────────┘
                    │
                    ▼
S71  ┌─────────────────────────────┐
     │  △MIN5(n) EXTRACTION        │
     │       PROCESS               │
     └─────────────────────────────┘
                    │
                    ▼
S72  ⟨ △MIN5(n) = △MIN5(n−1)?  ⟩────YES──┐
     ⟨  AND F₂(n−1) = +1?        ⟩         │
                    │ NO                    │
                    ▼                        │
S73  ⟨ |△Zₐ(n)| ≤ 0.3? ⟩──NO──────────────┤
                    │ YES                    │
                    ▼                        ▼
           ┌──────────────┐          ┌──────────────┐
           │  F₂(n) = +1  │          │  F₂(n) = 0   │
           └──────────────┘          └──────────────┘
                    │                        │
                    ▼◄───────────────────────┘
S74  ⟨ F₂(n) = +1? ⟩──NO──────────────────┐
                    │ YES                    │
                    ▼                        ▼
S75  ┌─────────────────────┐      ┌─────────────────────┐
     │ CALCULATE SECOND    │      │ Z_b0(n−1) = Z_b0(n) │
     │ CENTERING VALUE     │      └─────────────────────┘
     │      Z_b0(n)        │                 │
     └─────────────────────┘                 │
                    │◄────────────────────────┘
                    ▼
S76  ┌─────────────────────┐
     │ CALCULATE SECOND    │
     │ DIFFERENCE  △Z_b(n) │
     └─────────────────────┘
                    │
                    ▼
               ┌─────────┐
               │   END   │
               └─────────┘
```

Within the flowchart:

- S71: $\triangle MIN5(n)$ EXTRACTION PROCESS
- S72: $\triangle MIN5(n) = \triangle MIN5(n-1)$? AND $F_2(n-1) = +1$?
- S73: $|\triangle Z_a(n)| \leq 0.3$?
- $F_2(n) = +1$
- $F_2(n) = 0$
- S74: $F_2(n) = +1$?
- S75: CALCULATE SECOND CENTERING VALUE $Z_{b0}(n)$
- $Z_{b0}(n-1) = Z_{b0}(n)$
- S76: CALCULATE SECOND DIFFERENCE $\triangle Z_b(n)$

## Fig. 22

## Fig. 23

Fig. 24

C

S85 — $\Delta Z_a(n) < 0?$ — NO

YES

ADD ONE TO LAST COUNT VALUE $N_i(n-1)$ OF THE NUMBER OF INTEGRATIONS

INITIALIZE COUNT VALUE $N_i$ OF THE NUMBER OF INTEGRATIONS

S86 — $\Delta Z_a(n) < 0?$ — NO

YES

$\sum \Delta R_{ib}/\Delta A_b(n-1) + \Delta R_{ib}/\Delta A_b(n) = \sum \Delta R_{ib}/\Delta A_b(n)$

$F_1(n) = +1$

S87 — $N_i(n) = 0?$ — NO

YES

$[\sum \Delta R_{ib}/\Delta A_b(n)]/N_i(n) = \sum \Delta R_{ib}/\Delta A_b/N_i(n)$

$[\sum \Delta R_{ib}/\Delta A_b(n-1)]/N_i(n-1) = \sum \Delta R_{ib}/\Delta A_b(n)/N_i(n)$

END

Fig. 25

Fig. 26

## Fig. 27

RESPIRATION DEPTH
CALCULATION PROCESS

S501 — SAMPLING TIME?

S502 — INHALATION? — NO

YES

S503 — PEAK-HOLD PROCESS

S504 — BOTTOM-HOLD PROCESS

NO — ZERO-CROSS TIME? — S505

YES

S506 — DIFFERENTIAL COEFFICIENT > 0? — NO

YES

S507 — CALCULATE RESPIRATION DEPTH AT LAST RESPIRATORY ACTION

S508 — INHALATION FLAG SETTING PROCESS

S510 — EXHALATION FLAG SETTING PROCESS

S509 — INITIALIZE PEAK-HOLD PROCESS

S511 — INITIALIZE BOTTOM-HOLD PROCESS

END

Fig. 28

RESPIRATION DEPTH
DISPLAYING PROCESS

S601 — NORMALIZE RESPIRATION DEPTH AT
LAST SINGLE RESPIRATION

S602 — DECIDE THE NUMBER OF DEGREE
INDICATORS TO BE COLORED

S603 — DECIDE ABDOMINAL RESPIRATION
PERCENTAGE LEVEL

S604 — DEGREE-NUMBER DISTRIBUTION
PROCESS

S605 — DECIDE MARGIN LEVEL
CORRESPONDING TO RESPIRATION
SPEED

S606 — DISPLAY BAR GRAPHS

Fig. 29

Fig. 30

## Fig. 31

RESPIRATION TYPE
DETERMINATION PROCESS

S701

$[\Sigma \triangle R_{ib} / \triangle A_b]/N_i \leq 1.0?$ — NO

YES

S703

S702 — ABDOMINAL RESPIRATION

RETRIEVE $Z_{b1}$ AND $\triangle Z_{b1}$

S704

$Z_{b0} \geq Z_{b1} + \triangle Z_{b1}?$ — NO

YES

S706

S705 — DRAW-IN RESPIRATION

COSTAL RESPIRATION

END

Fig. 32

Fig. 33

Fig. 34

Fig. 35

Fig. 36

```
              ┌─────────────────────────────┐
              │  LISSAJOUS FIGURE DISPLAYING │
              │          PROCESS             │
              └─────────────────────────────┘
                           │
                           ▼ ◄──────────────┐
                      ╱─────────────╲        │
  S801 ──────┤ SAMPLING TIME? ├─ NO ────────┘
                      ╲─────────────╱
                           │ YES
                           ▼
              ┌─────────────────────────────┐
  S802 ───────┤     DETERMINE FIRST         │
              │  BIOELECTRICAL IMPEDANCE    │
              └─────────────────────────────┘
                           │
                           ▼
              ┌─────────────────────────────┐
  S803 ───────┤    DETERMINE SECOND         │
              │  BIOELECTRICAL IMPEDANCE    │
              └─────────────────────────────┘
                           │
                           ▼
              ┌─────────────────────────────┐
  S804 ───────┤     SMOOTHING PROCESS       │
              └─────────────────────────────┘
                           │
                           ▼
              ┌─────────────────────────────┐
              │    GENERATE DISPLAY DATA     │
  S805 ───────┤ FOR DISPLAYING LISSAJOUS    │
              │          FIGURE              │
              └─────────────────────────────┘
                           │
                           ▼
              ┌─────────────────────────────┐
  S806 ───────┤   DISPLAY LISSAJOUS FIGURE  │
              └─────────────────────────────┘
                           │
                           ▼
                    ┌─────────────┐
                    │   RETURN    │
                    └─────────────┘
```

EP 2 407 100 A1

**Fig. 37**

**Fig. 38**

**Fig. 39**

Fig. 40

RIGHT LUNG

LEFT LUNG

$Z_{aR}, Z_{aL}$ [Ω]

$Z_b$ [Ω]

Fig. 41

RIGHT LUNG

LEFT LUNG

$Z_{aR}, Z_{aL}$ [Ω]

$Z_b$ [Ω]

Fig. 42

RIGHT LUNG

LEFT LUNG

$Z_{aR}, Z_{aL}$ [Ω]

⊢●⊣ AVERAGE

$Z_b$ [Ω]

Fig. 43

$C(Z_{b0}, Z_{a0})$

160A

Fig. 44

160A

Fig. 45

Fig. 46

Fig. 47

Fig. 48

Fig. 49

Fig. 50

Fig. 51

LN

Z$_a$ [Ω]

α

β

Z$_b$ [Ω]

Fig. 52A

◆ PEAK
■ BOTTOM

RESPIRATION DEPTH

0.8

0.4

0.0

-0.4

-0.8

0   4   8   12   16   20   24   28   32   36   40   44   48   52   56   60   64

PRESENT

MEASUREMENT
START

TIME ELAPSED [sec]

Fig. 52B

◆ PEAK
■ BOTTOM

RESPIRATION DEPTH

1.0

0.5

0.0

-0.5

-1.0

-1.5

0      1.6      2.3      4.0      6.3      9.9      16      25      40      63      100

PRESENT

MEASUREMENT
START

LOG OF TIME ELAPSED [sec]

## Fig. 52C

| ◆PEAK |
| ■BOTTOM |

INTERVAL 1 ┆ INTERVAL 2 ┆ INTERVAL 3

RESPIRATION DEPTH

0.8

0.4

0.0

-0.4

-0.8

0  2  4  6  8  10  20  30  40  50  100  150

PRESENT

MEASUREMENT START

TIME ELAPSED [sec]

## Fig. 53

BG3

INHALATION

THREE DEGREES

EXHALATION

THREE DEGREES

Fig. 54

MIDDLE DEPTH
INHALATION

INHA-
LATION

EXHA-
LATION

MIDDLE DEPTH
INHALATION

INHA-
LATION

EXHA-
LATION

MIDDLE DEPTH
EXHALATION

INHA-
LATION

EXHA-
LATION

MIDDLE DEPTH
EXHALATION

INHA-
LATION

EXHA-
LATION

Fig. 55

SMALL ← → LARGE

## Fig. 56

Fig. 57

Fig. 58

TBL: TRAINING MENU
MANAGEMENT TABLE

| | RANKING | BREATHING TRAINING MENUS | REQUIREMENT FOR ADVANCING THROUGH RANKING |
|---|---|---|---|
| HIGH | 5 | MENU 81 → MENU 82 → MENU 83 → ⋯⋯ → MENU 100 | REQUIREMENT E |
| | 4 | MENU 61 → MENU 62 → MENU 63 → ⋯⋯ → MENU 80 | REQUIREMENT D |
| RESPIRATION CAPABILITY | 3 | MENU 41 → MENU 42 → MENU 43 → ⋯⋯ → MENU 60 | REQUIREMENT C |
| | 2 | MENU 21 → MENU 22 → MENU 23 → ⋯⋯ → MENU 40 | REQUIREMENT B |
| LOW | 1 | MENU 01 → MENU 02 → MENU 03 → ⋯⋯ → MENU 20 | REQUIREMENT A |

Fig. 59

```
           ┌──────────────────────────────┐
           │    BREATHING TRAINING        │
           │    MANAGEMENT PROCESS        │
           └──────────────────────────────┘
                          │
                          ▼
           ┌──────────────────────────────┐
   S901 ───┤  DETERMINE RESPIRATION       │
           │       CAPABILITY             │
           └──────────────────────────────┘
                          │
                          ▼
           ┌──────────────────────────────┐
   S902 ───┤      IDENTIFY RANKING        │
           └──────────────────────────────┘
                          │
                          ▼
           ┌──────────────────────────────┐
   S903 ───┤   SELECT TRAINING MENUS      │
           └──────────────────────────────┘
                          │
                          ▼
           ┌──────────────────────────────┐
   S904 ───┤ BREATHING TRAINING PROCESS   │
           └──────────────────────────────┘
                          │
                          ▼
           ╱──────────────────────────────╲
           │   REQUIREMENT FOR            │    NO
   S905 ───┤   ADVANCING THROUGH          │─────►
           │   RANKING IS SATISFIED?      │
           ╲──────────────────────────────╱
                          │ YES
                          ▼
           ┌──────────────────────────────┐
   S906 ───┤   ADVANCE TO NEXT RANKING    │
           └──────────────────────────────┘
```

Fig. 60

```
              ( START )
                  |
    S1 ── ENTER PERSONAL
          INFORMATION
                  |
    S2 ──   MEASURE
             WEIGHT
                  |
    S3 ── RESPIRATION
          ANALYSIS PROCESS
                  |
    S4 ── RESPIRATION DEPTH
          DISPLAYING PROCESS
                  |
    S5 ── RESPIRATION-CHARACTERISTIC-
          DETERMINATION-AND-DISPLAYING
          PROCESS
```

Fig. 61

```
      ( RESPIRATION-CHARACTERISTIC-
        DETERMINATION-AND-DISPLAYING
        PROCESS )
                  |
        RESPIRATION DEPTH ≥ ESSENTIAL      NO
        RESPIRATION DEPTH AT REST? ──────────────┐
   S1000                  |                       |  S1001
                         YES            REPORT GUIDANCE
                          |             INFORMATION
                          |                       |
    S1002 ── DECIDE RESPIRATION ◄─────────────────┘
             CHARACTERISTIC
                  |
    S1003 ── DISPLAY DECISION RESULT
                  |
             ( RETURN )
```

Fig. 63

NEGATIVE $\leftarrow$ 0.0 POSITIVE $\rightarrow$

$\Delta Z_a$

$\Delta Z_b$

Fig. 62

NEGATIVE $\leftarrow$ 0.0 POSITIVE $\rightarrow$

$\Delta Z_b$

$\Delta Z_a$

Fig. 64

COSTAL-ABDOMINAL
BALANCE OF
VENTILATION

0 - 0.4

0.4 - 0.8

0.8 - 1.2

1.2 - 1.6

1.6 -

BG4

## Fig. 65

## Fig. 66

Europäisches
Patentamt

European
Patent Office

Office européen
des brevets

# EUROPEAN SEARCH REPORT

Application Number

EP 11 17 3341

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/118634 A1 (WEILER) 7 May 2009 (2009-05-07) * paragraphs [0023] - [0024] * ----- | 1-42 | INV. A61B5/053 A61B5/08 |
| X | US 2003/216664 A1 (SUAREZ) 20 November 2003 (2003-11-20) * paragraphs [0076], [0087], [0090], [0135], [0149] * ----- | 1-42 | |
| X | US 2004/143194 A1 (KIHARA ET AL) 22 July 2004 (2004-07-22) * paragraph [0184]; figure 25 * ----- | 1-42 | |
| X,P | EP 2 215 966 A2 (TANITA) 11 August 2010 (2010-08-11) * paragraphs [0070] - [0074], [0101] * ----- | 1-42 | |
| X,P | US 2011/060215 A1 (TUPIN ET AL) 10 March 2011 (2011-03-10) * paragraphs [0133], [0134], [0290] * ----- | 1-42 | |
| A | EP 1 536 364 A2 (SONY) 1 June 2005 (2005-06-01) * paragraphs [0016], [0041] * ----- | 1,33,34, 41,42 | TECHNICAL FIELDS SEARCHED (IPC) A61B |
| A | WO 86/00793 A1 (PELTZER) 13 February 1986 (1986-02-13) * page 7, lines 9-14 * ----- | 1,33,34, 41,42 | |

The present search report has been drawn up for all claims

| Place of search | Date of completion of the search | Examiner |
|---|---|---|
| Munich | 19 October 2011 | Martelli, Luca |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 2 407 100 A1**

## ANNEX TO THE EUROPEAN SEARCH REPORT
## ON EUROPEAN PATENT APPLICATION NO.

EP 11 17 3341

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2011

| Patent document cited in search report | | Publication date | Patent family member(s) | | Publication date |
|---|---|---|---|---|---|
| US 2009118634 | A1 | 07-05-2009 | AU | 2008299098 A1 | 19-03-2009 |
| | | | CA | 2699281 A1 | 19-03-2009 |
| | | | CN | 101801265 A | 11-08-2010 |
| | | | EP | 2194863 A1 | 16-06-2010 |
| | | | JP | 2010538748 A | 16-12-2010 |
| | | | US | 2009118634 A1 | 07-05-2009 |
| | | | WO | 2009035965 A1 | 19-03-2009 |
| US 2003216664 | A1 | 20-11-2003 | AT | 345086 T | 15-12-2006 |
| | | | AU | 8384901 A | 17-12-2001 |
| | | | AU | 2001283849 B2 | 17-03-2005 |
| | | | CA | 2411589 A1 | 13-12-2001 |
| | | | DE | 60124541 T2 | 06-09-2007 |
| | | | EP | 1292224 A1 | 19-03-2003 |
| | | | ES | 2276814 T3 | 01-07-2007 |
| | | | JP | 4755801 B2 | 24-08-2011 |
| | | | JP | 2003534867 A | 25-11-2003 |
| | | | US | 2003216664 A1 | 20-11-2003 |
| | | | WO | 0193760 A1 | 13-12-2001 |
| US 2004143194 | A1 | 22-07-2004 | AU | 2002234945 A1 | 19-09-2002 |
| | | | EP | 1374767 A2 | 02-01-2004 |
| | | | US | 2004143194 A1 | 22-07-2004 |
| | | | WO | 02069878 A2 | 12-09-2002 |
| EP 2215966 | A2 | 11-08-2010 | EP | 2215966 A2 | 11-08-2010 |
| | | | JP | 4732527 B2 | 27-07-2011 |
| | | | JP | 2010183980 A | 26-08-2010 |
| | | | US | 2010204601 A1 | 12-08-2010 |
| US 2011060215 | A1 | 10-03-2011 | NONE | | |
| EP 1536364 | A2 | 01-06-2005 | CN | 1620987 A | 01-06-2005 |
| | | | EP | 1536364 A2 | 01-06-2005 |
| | | | JP | 3885794 B2 | 28-02-2007 |
| | | | JP | 2005152462 A | 16-06-2005 |
| | | | KR | 20050051553 A | 01-06-2005 |
| | | | US | 2006189879 A1 | 24-08-2006 |
| WO 8600793 | A1 | 13-02-1986 | EP | 0188508 A1 | 30-07-1986 |
| | | | NL | 8500314 A | 17-02-1986 |
| | | | WO | 8600793 A1 | 13-02-1986 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007043302 A1